# EUROPEAN PATENT APPLICATION

(11) **EP 4 789 670 A1**
(43) Date of publication of application: **12.08.2026**
(21) Application number: 24832160.6
(22) Date of filing: 04.10.2024
(51) Int. Cl.: A61K 31/513, A61K 9/14, A61K 9/16, A61K 9/20, A61K 9/48, A61K 47/02, A61K 47/04, A61K 47/10, A61K 47/12, A61K 47/14, A61K 47/26, A61K 47/32, A61K 47/36, A61K 47/38, A61K 47/44, A61P 31/14, A61P 43/00, C07D 401/14, C12N 9/99

(54) **PREPARATION CONTAINING URACIL DERIVATIVE**

(30) Priority: 05.10.2023 JP 2023173297; 30.05.2024 JP 2024088082
(71) Applicant: Shionogi & Co., Ltd, Osaka-shi, Osaka 541-0045 (JP)
(72) Inventor: OHASHI, Ryo, Osaka-shi, Osaka 541-0045 (JP); OTAKE, Shuichi, Osaka-shi, Osaka 541-0045 (JP); MURATA, Tatsuhiko, Osaka-shi, Osaka 541-0045 (JP); KONDO, Daisuke, Osaka-shi, Osaka 541-0045 (JP); YOKOO, Katsuki, Osaka-shi, Osaka 541-0045 (JP)
(74) Representative: Vossius & Partner Patentanwälte Rechtsanwälte mbB
(86) International application number: PCT/JP2024/035558
(87) International publication number: WO 2025/005305

(57) **Abstract**

The present invention provides a preparation (pharmaceutical composition) comprising, as an active ingredient, a uracil derivative exhibiting coronavirus 3CL protease inhibitory activity, a pharmaceutically acceptable salt thereof, or a solvate thereof.

The present invention further provides a crystal of a uracil derivative exhibiting coronavirus 3CL protease inhibitory activity, a pharmaceutically acceptable salt thereof, or a solvate thereof.

## Description

### Technical Field

The present invention relates to a preparation containing a uracil derivative. The present invention specifically relates to a preparation containing a uracil derivative exhibiting coronavirus 3CL protease inhibitory activity, a pharmaceutically acceptable salt thereof, or a solvate thereof.

The present invention further relates to a solid state (crystal or amorphous form) of a uracil derivative exhibiting coronavirus 3CL protease inhibitory activity, a pharmaceutically acceptable salt thereof, or a solvate thereof.

### Background Art

Nidovirales order, Coronaviridae family, and Orthocoronavirinae subfamily coronaviruses have a genome size of about 30 kb and are the largest single-stranded positive-sense RNA viruses among known RNA viruses. Coronaviruses are classified into four genera: Alphacoronavirus, Betacoronavirus, Gammacoronavirus, and Deltacoronavirus, and seven coronaviruses are known to infect humans, including two alphacoronaviruses (HCoV-229E and HCoV-NL63) and five betacoronaviruses (HCoV-HKU1, HCoV-OC43, SARS-CoV, MERS-CoV, and SARS-CoV-2). Of these, four coronaviruses (HCoV-229E, HCoV-NL63, HCoV-HKU1, and HCoV-OC43) are causative agents of the common cold, whereas the remaining three are severe acute respiratory syndrome (SARS) coronavirus (SARS-CoV), Middle East respiratory syndrome (MERS) coronavirus (MERS-CoV), and novel coronavirus (SARS-CoV-2), which are known to cause severe pneumonia.

Novel coronavirus infections (COVID-19), which emerged in December 2019, rapidly spread throughout the international community, and the World Health Organization (WHO) declared it a pandemic on March 11, 2020. The main routes of transmission of SARS-CoV-2 have been reported to include droplet transmission, contact transmission, and aerosol transmission, and it has been confirmed that SARS-CoV-2 can remain suspended in the air together with aerosols for approximately three hours while retaining infectivity (Non Patent Document 1). The incubation period is approximately 2 to 14 days, and typical symptoms include fever (87.9%), dry cough (67.7%), fatigue (38.1%), and sputum production (33.4%), which are common cold-like symptoms (Non Patent Document 2). In severe cases, respiratory failure due to acute respiratory distress syndrome, acute lung injury, interstitial pneumonia, and the like occurs. Furthermore, multiple organ failure such as renal failure and hepatic failure has also been reported.

When coronaviruses infect cells, they synthesize two polyproteins. These two polyproteins contain a replication complex generating a viral genome, as well as two proteases. The proteases cleave the virally synthesized polyproteins and play an essential role in enabling each protein to function. Among the two proteases, the 3CL protease (main protease) is responsible for most of the polyprotein cleavage (Non Patent Document 3).

Compounds having different chemical structures are known as active ingredients of COVID-19 therapeutic agents targeting the 3CL protease; however, these compounds are chemically distinct from the compound used in the present invention.

Compounds having 3CL protease inhibitory activity are disclosed in Patent Documents 1 to 4 and Non Patent Documents 4 to 14; however, the compounds used in the present invention are neither described nor suggested in any of the documents.

### Prior Art Documents

### Patent Documents

Patent Document 1: WO 2021/205298
Patent Document 2: WO 2021/250648
Patent Document 3: WO 2022/138987
Patent Document 4: WO 2022/138988

### Non Patent Documents

Non Patent Document 1: The NEW ENGLAND JOURNAL of MEDICINE (2020), Vol. 382, pp. 1564-1567
Non Patent Document 2: "Report of the WHO-China Joint Mission on Coronavirus Disease 2019 (COVID-19)", [online], February 28, 2020, WHO, [retrieved on March 16, 2023], Internet <URL: https://www.who.int/docs/default-source/coronaviruse/who-china-joint-mission-on-covid-19-final-report.pdf>
Non Patent Document 3: Science (2003), Vol. 300, pp. 1763-1767
Non Patent Document 4: "A comparative analysis of SARS-CoV-2 antivirals characterizes 3CLpro inhibitor PF-00835231 as a potential new treatment for COVID-19", Journal of Virology, 2021 Mar 10; 95(7), e01819-20
Non Patent Document 5: Cell Research (2020), Vol. 30, pp. 678-692
Non Patent Document 6: Science (2020), Vol. 368, pp. 409-412
Non Patent Document 7: ACS Central Science (2021), Vol. 7, No. 3, pp. 467-475
Non Patent Document 8: 261st Am Chem Soc (ACS) Natl Meet · 2021-04-05 / 2021-04-16 · Virtual, N/A · Abst 243
Non Patent Document 9: Science (2021), Vol. 374, pp. 1586-1593
Non Patent Document 10: "Discovery and Development of PBI-0451", [online], March 24, 2022, 35th International Conference on Antiviral Research (ICAR), [retrieved on March 16, 2023], Internet <URL: https://ir.pardesbio.com/static-files/fc7c4f8c-e0bd-4b97-8c9c-eff09bafd4db>
Non Patent Document 11: Molecules (2020), vol. 25, p. 3193
Non Patent Document 12: Molecules (2020), vol.25, p. 3920
Non Patent Document 13: European Journal of Medicinal Chemistry (2020), Vol. 206, p. 112711
Non Patent Document 14: Journal of the American Chemical Society (2022), vol. 144, pp. 2905-2920

### SUMMARY OF THE INVENTION

### TECHNICAL PROBLEMS TO BE SOLVED BY THE INVENTION

An object of the present invention is to provide a preparation comprising, as an active ingredient, a uracil derivative exhibiting coronavirus 3CL protease inhibitory activity, a pharmaceutically acceptable salt thereof, or a solvate thereof. An object of the present invention is preferably to provide an oral preparation comprising a uracil derivative exhibiting antiviral activity, particularly coronavirus replication-inhibiting activity, a pharmaceutically acceptable salt thereof, or a solvate thereof.

Further, an object of the present invention is to provide a solid state (crystal or amorphous form) of a uracil derivative exhibiting coronavirus 3CL protease inhibitory activity, a pharmaceutically acceptable salt thereof, or a solvate thereof.

### MEANS FOR SOLVING THE PROBLEM

The present invention relates to the following.
(1) A preparation comprising, as an active ingredient, a compound represented by Formula (I): 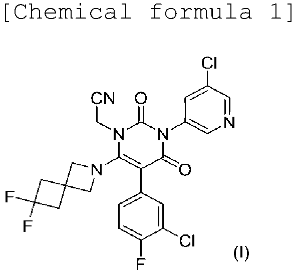 , a pharmaceutically acceptable salt thereof, or a solvate thereof.
(2) The preparation according to the above (1), wherein the active ingredient is an amorphous form of the compound represented by Formula (I), a pharmaceutically acceptable salt thereof, or a solvate thereof.
(3) The preparation according to the above (2), wherein the active ingredient is an amorphous form of the compound represented by Formula (I).
(4) The preparation according to the above (3), wherein the amorphous form of the compound represented by Formula (I) is comprised in a solid dispersion.
(5) The preparation according to the above (4), wherein the solid dispersion further comprises a polymer.
(6) The preparation according to the above (5), wherein the polymer is one or more selected from a vinyl-based polymer, a cellulose-based polymer, or an acrylic acid-based polymer.
(7) The preparation according to the above (6), wherein the polymer is a vinyl-based polymer, and the vinyl-based polymer is one or more selected from the group consisting of copovidone, polyvinyl pyrrolidone, polyvinyl polypyrrolidone, polyvinyl alcohol, copolymer of polyvinyl alcohol, acrylic acid, and methyl methacrylate, poly(vinyl alcohol)-graft-poly(ethylene glycol) copolymer, polyvinyl acetal diethylaminoacetate, a fumaric acid-stearic acid-polyvinyl acetal diethylaminoacetate-hydroxypropyl methylcellulose mixture, and hydroxypropyl methylcellulose, and polyvinyl acetal diethylaminoacetate.
(8) The preparation according to the above (7), wherein the vinyl-based polymer is copovidone.
(9) The preparation according to the above (6), wherein the polymer is a cellulose-based polymer, and the cellulose-based polymer is one or more selected from the group consisting of hypromellose acetate succinate, hypromellose phthalate, hydroxypropyl cellulose, low substituted hydroxypropylcellulose, hypromellose, hydroxyethyl cellulose, hydroxyethyl methylcellulose, hydroxypropyl methylcellulose phthalate, methylcellulose, methyl hydroxyethylcellulose, carboxymethyl ethylcellulose, ethyl cellulose, crystalline cellulose, microcrystalline cellulose, crystalline cellulose-carmellose sodium, carmellose, carmellose sodium, carmellose calcium, powdered cellulose, and a mixture of fumaric acid, stearic acid, poly(vinyl acetal)diethylaminoacetate, and hydroxypropyl methylcellulose.
(10) The preparation according to the above (6), wherein the polymer is an acrylic acid-based polymer, and the acrylic acid-based polymer is one or more selected from the group consisting of a methacrylic acid copolymer L, an aminoalkyl methacrylate copolymer E, a methacrylic acid copolymer LD, a methacrylic acid copolymer S, aminoalkyl methacrylate copolymer RS, ethyl acrylate-methyl methacrylate copolymer, ammonio alkyl methacrylate copolymer, a methyl acrylate-methacrylic acid-methyl methacrylate copolymer, and a 2-methyl-5-vinylpyridine methyl acrylate-methacrylic acid copolymer.
(11) The preparation according to the above (1), wherein the active ingredient is a crystal of a compound represented by Formula (I), a pharmaceutically acceptable salt thereof, or a solvate thereof.
(12) The preparation according to the above (11), wherein the active ingredient is an anhydrous crystal of the compound represented by Formula (I).
(13) The preparation according to any one of the above (1) to (12), further comprising: a disintegrant, a filler, and/or a lubricant.
(14) The preparation according to the above (13), wherein the disintegrant is one or more selected from the group consisting of croscarmellose sodium, carmellose, carmellose calcium, carmellose sodium, hydroxypropyl cellulose, low substituted hydroxypropylcellulose, powdered cellulose, partially pregelatinized starch, potato starch, cornstarch, hydroxypropyl starch, sodium carboxymethyl starch, low-substituted sodium carboxymethyl starch, sodium starch glycolate, pregelatinized starch, starch, polyvinyl alcohol, and crospovidone.
(15) The preparation according to the above (14), wherein the disintegrant is croscarmellose sodium.
(16) The preparation according to the above (14), wherein the disintegrant is crospovidone.
(17) The preparation according to any one of the above (13) to (16), wherein the filler is one or more selected from the group consisting of crystalline cellulose, silicified microcrystalline cellulose, lactose, anhydrous lactose, sucrose, glucose, fructose, sucrose, mannitol, sorbitol, erythritol, xylitol, maltodextrin, maltitol, starch, potato starch, corn starch, rice starch, partially pregelatinized starch, pregelatinized starch, porous starch, sodium carboxy starch, hydroxypropyl starch, low-substituted sodium carboxymethyl starch, powdered cellulose, carmellose sodium, carmellose, carmellose calcium, carboxymethyl ethylcellulose, low substituted hydroxypropylcellulose, silicate derivative, phosphate, carbonate, sulfate, magnesium oxide, titanium oxide, calcium lactate, synthetic hydrotalcite, talc, kaolin, dried aluminum hydroxide, magnesium oxide, bentonite, silicon dioxide such as hydrated silica and light anhydrous silicic acid, magnesium aluminometasilicate, synthetic aluminum silicate, calcium silicate, anhydrous dibasic calcium phosphate, calcium monohydrogen phosphate, calcium hydrogen phosphate, sodium hydrogen phosphate, dibasic potassium phosphate, potassium dihydrogen phosphate, calcium dihydrogen phosphate, sodium dihydrogen phosphate, precipitated calcium carbonate, calcium carbonate, magnesium carbonate, and calcium sulfate.
(18) The preparation according to any one of the above (13) to (17), wherein the lubricant is one or more selected from the group consisting of sodium stearyl fumarate, magnesium stearate, calcium stearate, stearic acid, stearyl alcohol, polyoxyl stearate 40, talc, light anhydrous silicic acid, hydrated silicon dioxide, magnesium carbonate, precipitated calcium carbonate, dried aluminum hydroxide gel, magnesium aluminometasilicate, magnesium silicate, synthetic aluminum silicate, magnesium oxide, magnesium sulfate, cocoa butter, carnauba wax, glycerine fatty acid ester, hydrogenated oil, bleached beeswax, hydrogenated soybean oil, beeswax, cetanol, sodium laurate, sucrose fatty acid ester, and polyethylene glycol (macrogol).
(19) The preparation according to any one of the above (1) to (18), further comprising a coating layer.
(20) The preparation according to the above (19), wherein the coating layer comprises a photostabilizing agent and a polymer.
(21) The preparation according to the above (20), wherein the photostabilizing agent in the coating layer is one or more selected from the group consisting of Food Red No. 2, Food Red No. 3, Food Red No. 102, Food Red No. 104, Food Red No. 105, Food Red No. 106, Food Yellow No. 4, Food Yellow No. 5, Food Green No. 3, Food Blue No. 1, Food Blue No. 2, Food Red No. 3 aluminum lake, Food Yellow No. 4 aluminum lake, Food Yellow No. 5 aluminum lake, Food Blue No. 1 aluminum lake, Food Blue No. 2 aluminum lake, carmine, sodium copper chlorophyllin, copper chlorophyll, red iron oxide, black iron oxide, yellow iron oxide, titanium oxide, red ferric oxide, yellow ferric oxide, and talc.
(22) The preparation according to the above (21), wherein the photostabilizing agent is red ferric oxide, yellow ferric oxide, and/or talc.
(23) The preparation according to any one of the above (20) to (22), wherein the polymer in the coating layer is one or more one selected from hypromellose, hydroxypropyl cellulose, carboxymethyl ethyl cellulose, hypromellose phthalate, hydroxypropyl methylcellulose acetate succinate, ethyl cellulose, or polyvinyl alcohol.
(24) The preparation according to the above (23), wherein the polymer in the coating layer is hypromellose.
(25) The preparation according to any one of the above (1) to (24), wherein the preparation is an oral preparation.
(26) The preparation according to the above (25), wherein the preparation is a tablet, a granule, a powder, or a capsule.
(27) The preparation according to the above (11), wherein the active ingredient is an ethyl acetate solvate crystal of the compound represented by Formula (I).
(28) The preparation according to the above (27), wherein the preparation is a capsule.
(29) An amorphous form of a compound represented by Formula (I): 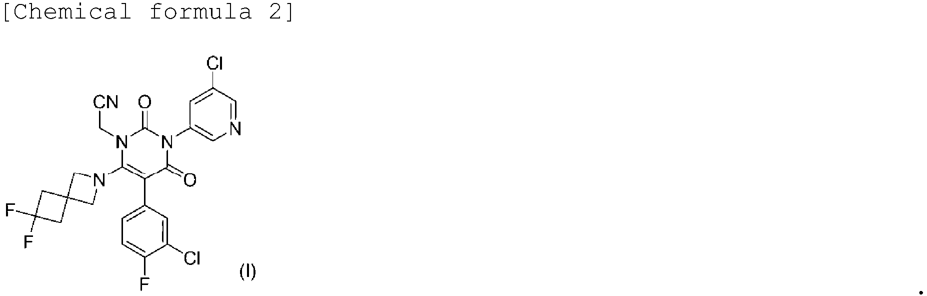
(30) An ethyl acetate solvate crystal of a compound represented by Formula (I): 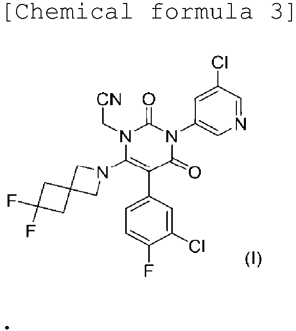
(31) A solid dispersion comprising a compound represented by Formula (I): 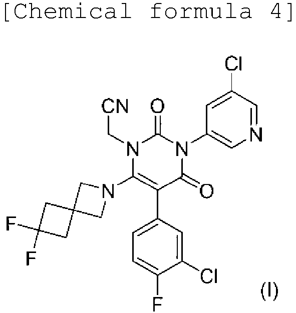 and a polymer.
(32) The solid dispersion according to the above (31), wherein the polymer is one or more selected from a vinyl-based polymer, a cellulose-based polymer, or an acrylic acid-based polymer.
(33) The solid dispersion according to the above (32), wherein the polymer is a vinyl-based polymer, and the vinyl-based polymer is one or more one selected from the group consisting of copovidone (polyvinyl pyrrolidone-vinyl acetate copolymer, PVPVA), polyvinyl pyrrolidone (povidone), polyvinylpyrrolidone, polyvinyl alcohol, copolymer of polyvinyl alcohol, acrylic acid, and methyl methacrylate, poly(vinyl alcohol)-graft-poly(ethylene glycol) copolymer, polyvinyl acetal diethylaminoacetate, fumaric acid-stearic acid-polyvinyl acetal diethylaminoacetate-hydroxypropyl methylcellulose mixture, and polyvinyl acetal diethylaminoacetate.
(34) The solid dispersion according to the above (33), wherein the vinyl-based polymer is copovidone.
(35) The solid dispersion according to the above (34), wherein a weight ratio of the compound represented by Formula (I) and the copovidone is 1:3.
(36) The preparation according to the above (25), wherein an amount of the compound represented by Formula (I) as the active ingredient is 20.0 mg.
(37) The ethyl acetate solvate crystal according to the above (30), wherein an X-Ray Powder diffraction pattern has peaks at diffraction angles (2θ) of 6.9°±0.2°, 8.8°±0.2°, 13.1°±0.2°, 16.3°±0.2°, and 23.7°±0.2°.
(38) The ethyl acetate solvate crystal according to the above (30) or (37), wherein a Raman spectrum has peaks at 421.2 cm⁻¹±2 cm⁻¹, 509.7 cm⁻¹±2 cm⁻¹, 1585.3 cm⁻¹±2 cm⁻¹, 1709.9 cm⁻¹±2 cm⁻¹, and 3052.9 cm⁻1±2 cm⁻¹.
(39) The preparation according to the above (17), wherein the filler is crystalline cellulose and/or mannitol.
(40) The preparation according to the above (18), wherein the lubricant is sodium stearyl fumarate and/or light anhydrous silicic acid.

### EFFECT OF THE INVENTION

The preparation according to the present invention has inhibitory activity against coronavirus 3CL protease and is useful as a therapeutic agent and/or prophylactic agent for coronavirus infections.

Further, the solid state (crystal or amorphous form) according to the present invention has inhibitory activity against coronavirus 3CL protease and is useful as an active pharmaceutical ingredient of the therapeutic agent and/or prophylactic agent for coronavirus infection.

### BRIEF DESCRIPTION OF THE DRAWINGS

[Fig. 1]

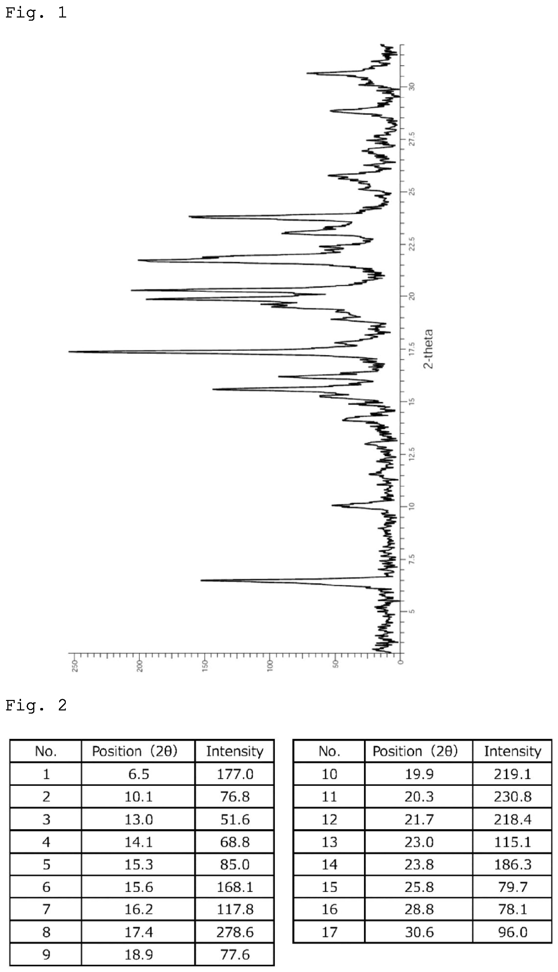

   Fig. 1 shows an X-Ray Powder diffraction pattern of an anhydrous crystal of the compound represented by Formula (I). The horizontal axis represents 2θ (°), and the vertical axis represents Intensity.
[Fig. 2]
   Fig. 2 shows a peak list of the X-Ray Powder diffraction pattern of Fig. 1. In the list, Position represents 2θ (°), and the Intensity represents the intensity.
[Fig. 3]

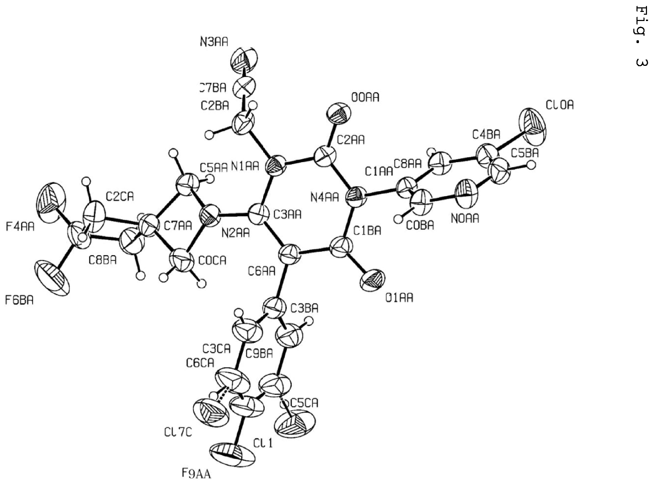

   Fig. 3 shows a crystal structure (structure in an asymmetric unit) of an anhydrous crystal of the compound represented by Formula (I).
[Fig. 4]

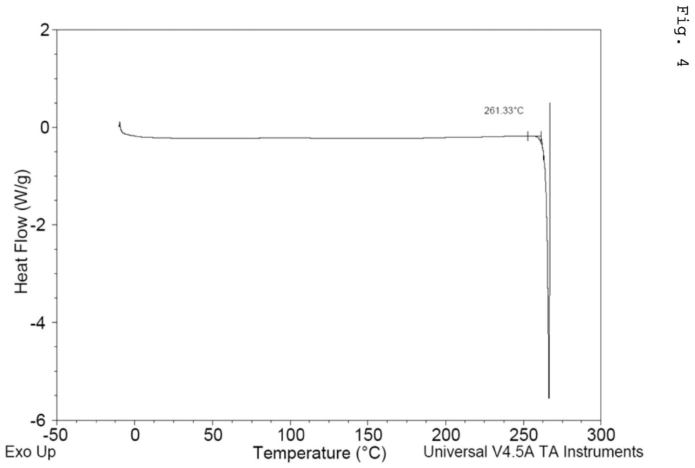

   Fig. 4 shows differential scanning calorimetry (DSC) results of the anhydrous crystal of the compound represented by Formula (I). The horizontal axis represents temperature (°C), and the vertical axis represents heat flow (W/g).
[Fig. 5]

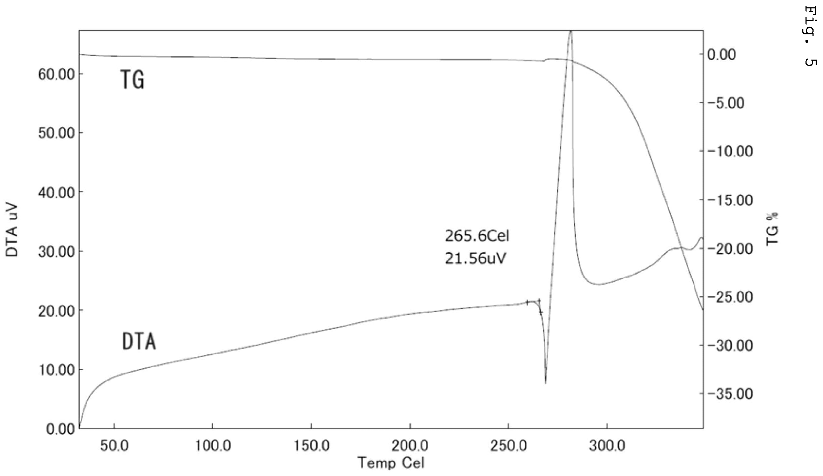

   Fig. 5 shows simultaneous thermogravimetric-differential thermal analysis (TG/DTA) results of the anhydrous crystal of the compound represented by Formula (I). The vertical axis represents heat flow (µV) or weight change (%), and the horizontal axis represents temperature (°C). In Fig. 5, Cel denotes degrees Celsius (°C).
[Fig. 6]

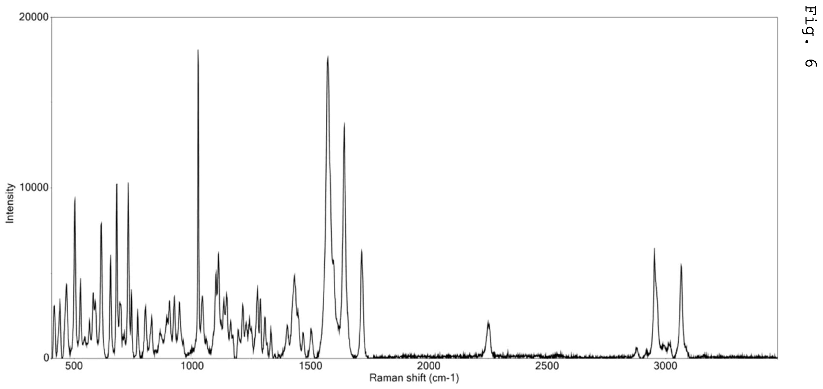

   Fig. 6 shows a Raman spectrum of the anhydrous crystal of the compound represented by Formula (I). The horizontal axis represents Raman shift (cm⁻¹), and the vertical axis represents peak intensity.
[Fig. 7]

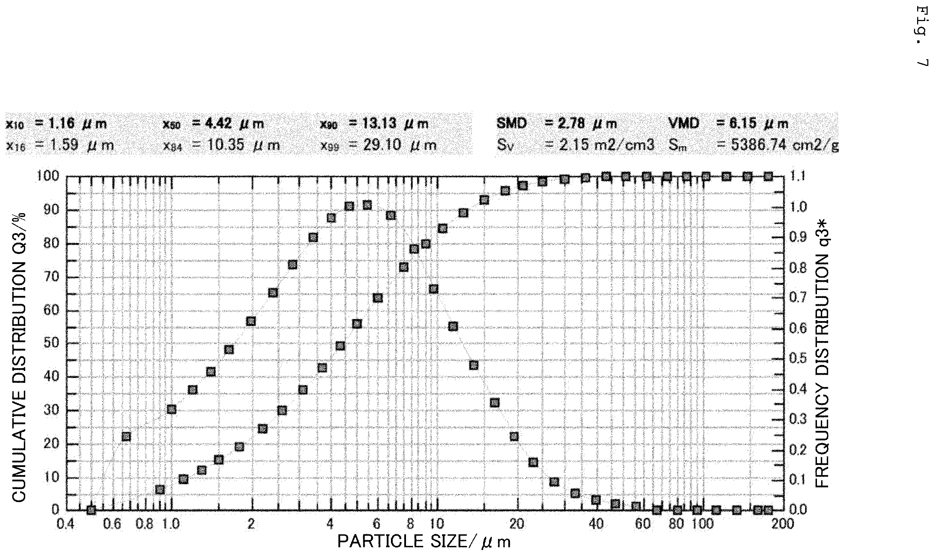

   Fig. 7 shows a particle size distribution of the anhydrous crystal of the compound represented by Formula (I) before milling, used in Example 2A.
[Fig. 8]

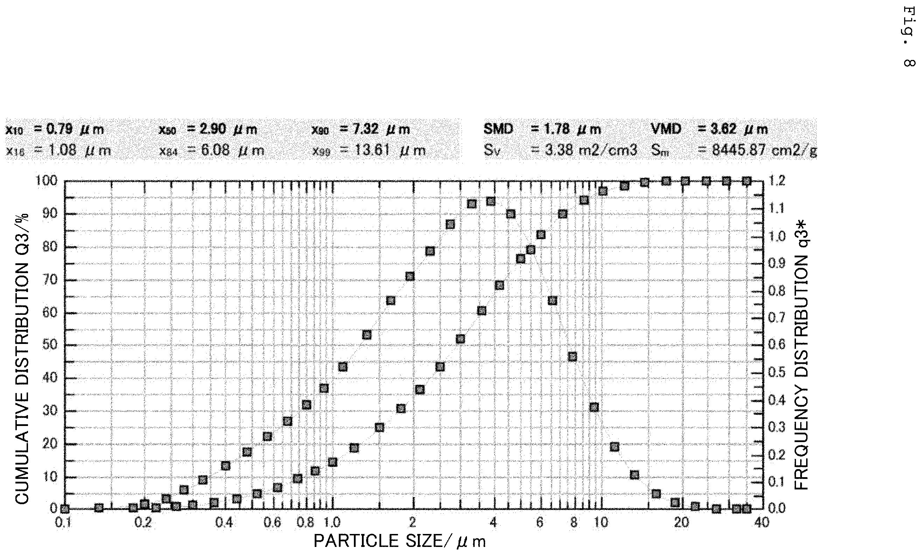

   Fig. 8 shows a particle size distribution of the anhydrous crystal of the compound represented by Formula (I) after milling, used in Example 2A.
[Fig. 9]

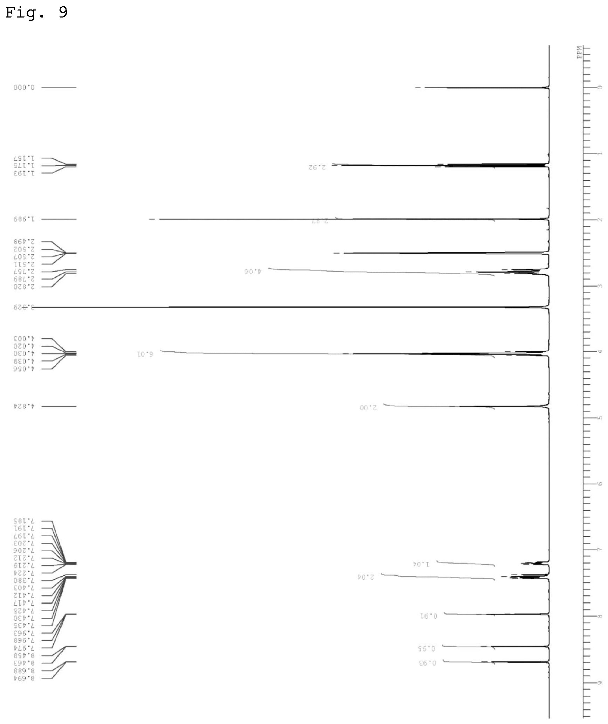

   Fig. 9 shows an NMR spectrum of an ethyl acetate solvate crystal of the compound represented by Formula (I). The horizontal axis represents the chemical shift (δ), and the vertical axis represents the relative intensity of proton signals.
[Fig. 10]

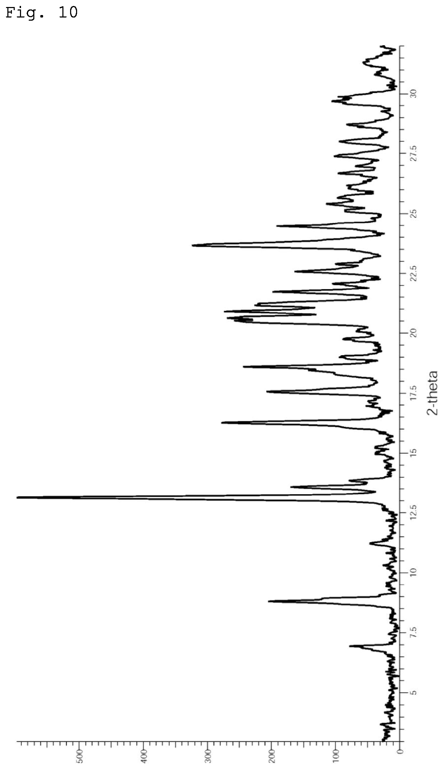

   Fig. 10 shows an X-Ray Powder diffraction pattern of the ethyl acetate solvate crystal of the compound represented by Formula (I). The horizontal axis represents 2θ (°), and the vertical axis represents intensity (Count).
[Fig. 11]

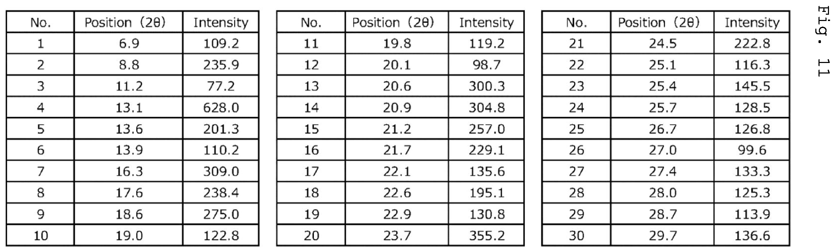

   Fig. 11 shows a peak list of the X-Ray Powder diffraction pattern of Fig. 10. In the list, Position represents 2θ (°), and the Intensity represents intensity.
[Fig. 12]

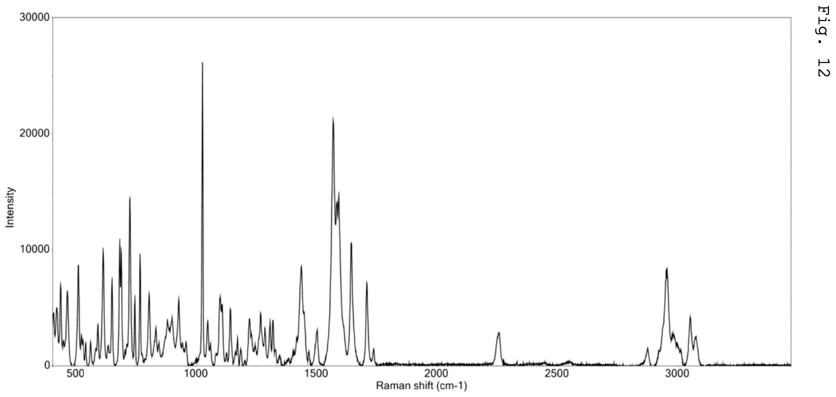

   Fig. 12 shows a Raman spectrum of the ethyl acetate solvate crystal of the compound represented by Formula (I). The horizontal axis represents Raman shift (cm⁻¹), and the vertical axis represents peak intensity.
[Fig. 13]

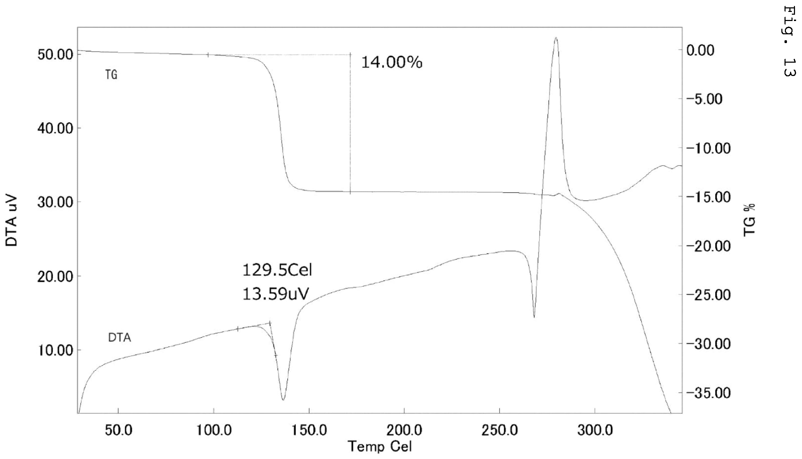

   Fig. 13 shows simultaneous thermogravimetric-differential thermal analysis (TG/DTA) results of the ethyl acetate solvate crystal of the compound represented by Formula (I). The vertical axis represents heat flow (µV) or weight change (%), and the horizontal axis represents temperature (°C). In Fig. 13, Cel denotes degrees Celsius (°C).
[Fig. 14]

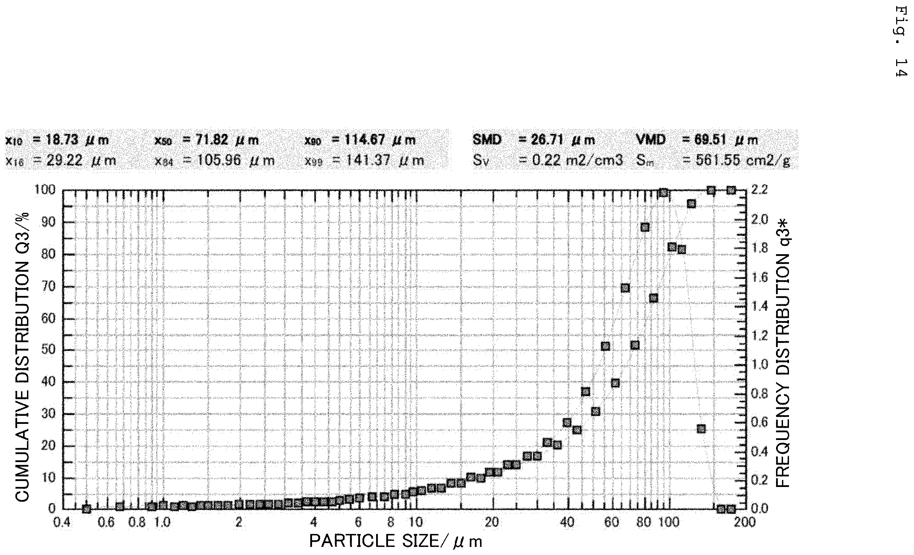

   Fig. 14 shows a particle size distribution of the ethyl acetate solvate crystal of the compound represented by Formula (I) before milling, used in Example 3E.
[Fig. 15]

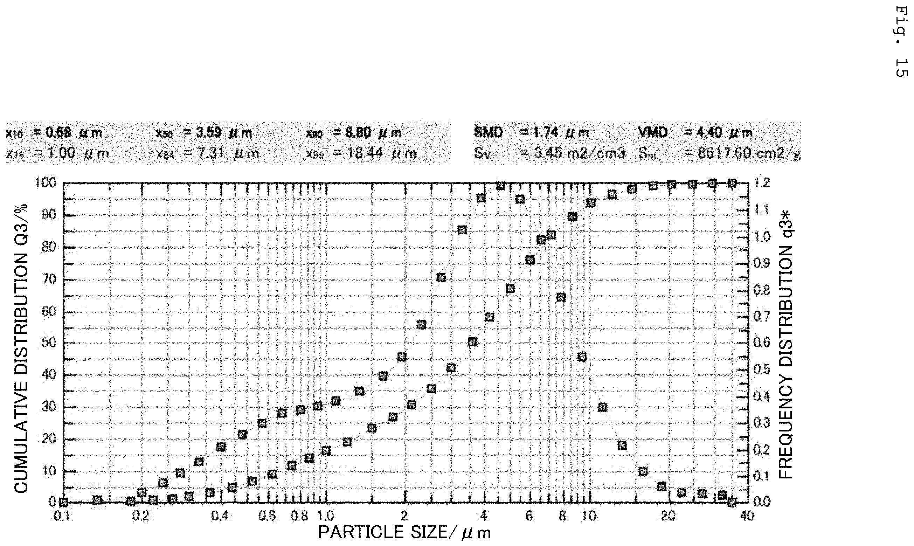

   Fig. 15 shows a particle size distribution of the ethyl acetate solvate crystal of the compound represented by Formula (I) after milling, used in Example 3E.
[Fig. 16]

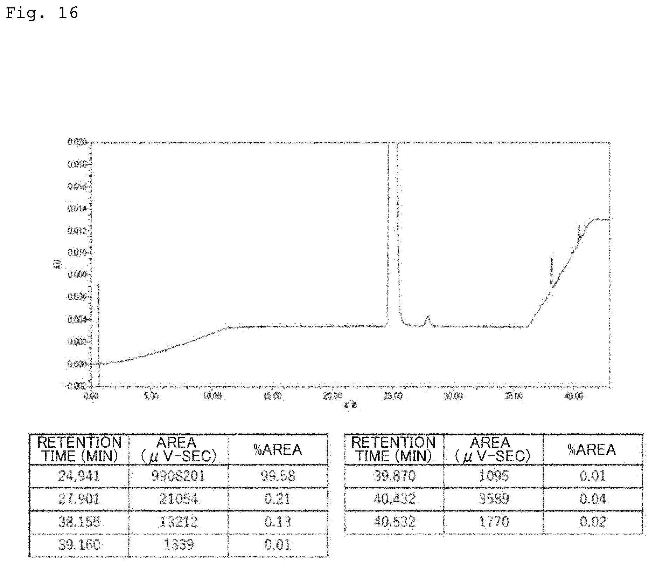

   Fig. 16 shows a chromatogram obtained by liquid chromatography of the anhydrous crystal of the compound represented by Formula (I) (having a particle size distribution of D10: 0.86 µm, D50: 3.29 µm, and D90: 10.15 µm).
[Fig. 17]

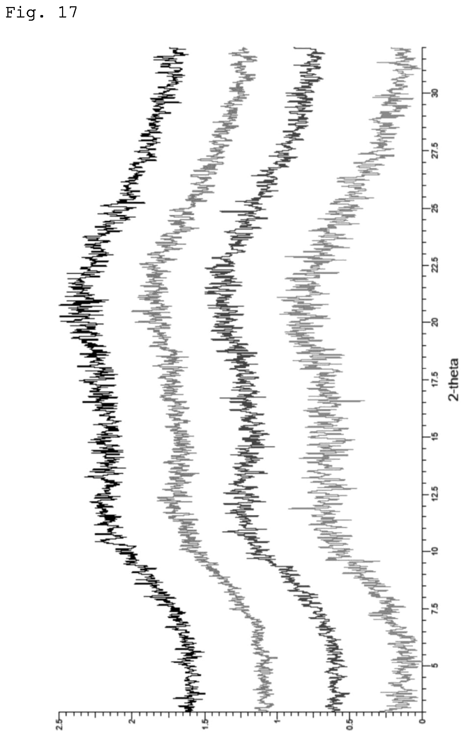

   Fig. 17 shows an X-Ray Powder diffraction pattern of the solid dispersion of Example 4B-1 (a solid dispersion comprising the compound represented by Formula (I) and copovidone). The horizontal axis represents 2θ (°), and the vertical axis represents Intensity. The four patterns, from top to bottom, correspond to a pattern after storage for one week in a closed glass bottle at 60°C, a pattern after storage for one week in a closed glass bottle at 40°C and 75% relative humidity, a pattern after storage for one week in an open glass bottle at 40°C and 75% relative humidity, and a pattern immediately after preparation.
[Fig. 18]

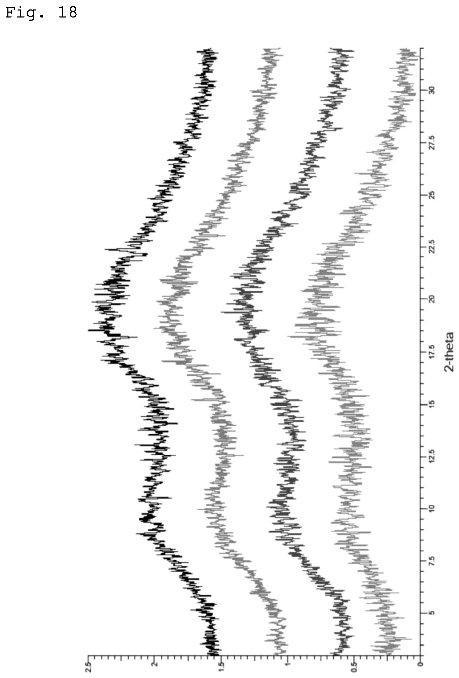

   Fig. 18 shows an X-Ray Powder diffraction pattern of the solid dispersion of Example 4B-2 (a solid dispersion comprising the compound represented by Formula (I) and hypromellose acetate succinate (grade MF)). The horizontal axis represents 2θ (°), and the vertical axis represents Intensity. The four patterns, from top to bottom, correspond to a pattern after storage for one week in a closed glass bottle at 60°C, a pattern after storage for one week in a closed glass bottle at 40°C and 75% relative humidity, a pattern after storage for one week in an open glass bottle at 40°C and 75% relative humidity, and a pattern immediately after preparation.
[Fig. 19]

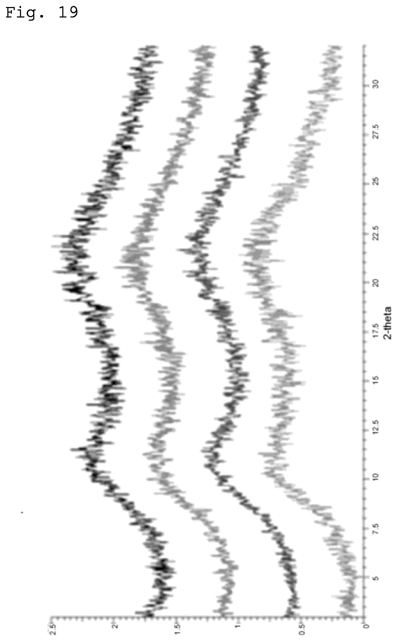

   Fig. 19 shows an X-Ray Powder diffraction pattern of the solid dispersion of Example 4B-3 (a solid dispersion comprising the compound represented by Formula (I) and povidone). The horizontal axis represents 2θ (°), and the vertical axis represents Intensity. The four patterns, from top to bottom, correspond to a pattern after storage for one week in a closed glass bottle at 60°C, a pattern after storage for one week in a closed glass bottle at 40°C and 75% relative humidity, a pattern after storage for one week in an open glass bottle at 40°C and 75% relative humidity, and a pattern immediately after preparation.
[Fig. 20]

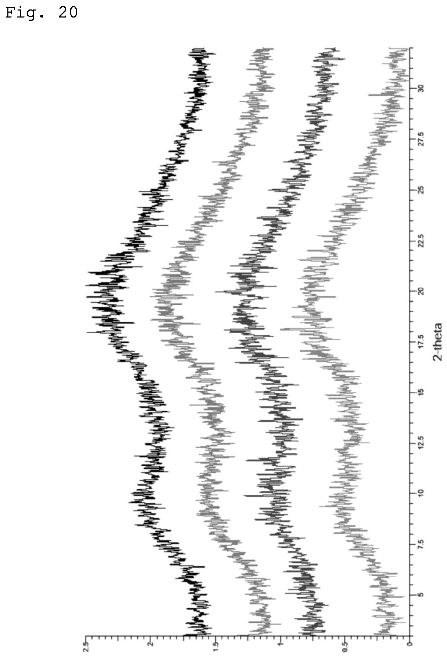

   Fig. 20 shows an X-Ray Powder diffraction pattern of the solid dispersion of Example 4B-4 (a solid dispersion comprising the compound represented by Formula (I) and hypromellose acetate succinate (grade LF)). The horizontal axis represents 2θ (°), and the vertical axis represents Intensity. The four patterns, from top to bottom, correspond to a pattern after storage for one week in a closed glass bottle at 60°C, a pattern after storage for one week in a closed glass bottle at 40°C and 75% relative humidity, a pattern after storage for one week in an open glass bottle at 40°C and 75% relative humidity, and a pattern immediately after preparation.
[Fig. 21]

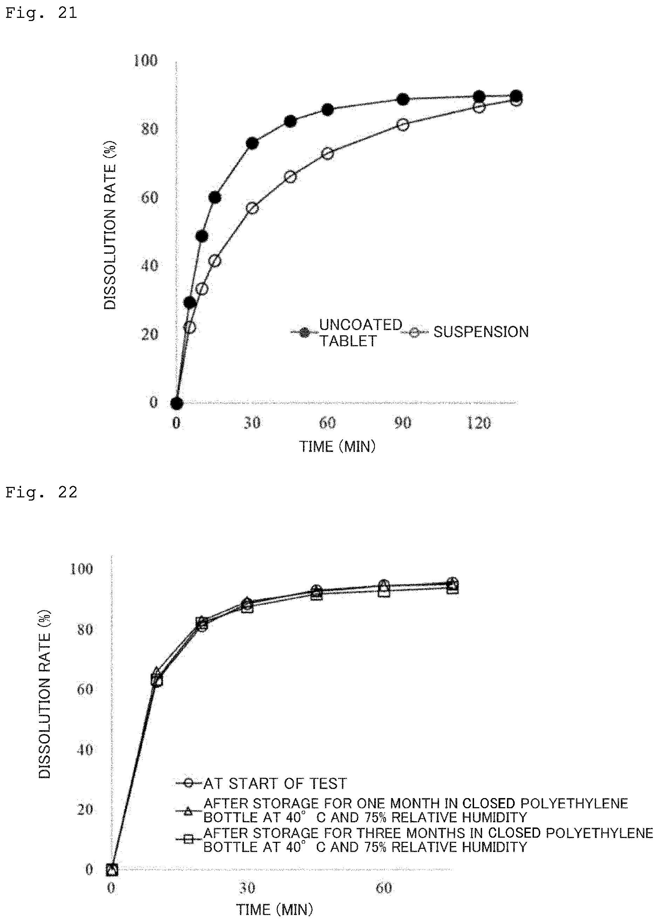

   Fig. 21 shows dissolution profiles of Example 5C-1 (uncoated tablet) and Example 5D-1 (suspension). The horizontal axis represents time (min), and the vertical axis represents dissolution rate (%).
[Fig. 22]
   Fig. 22 shows dissolution profiles of the tablet of Example 5E-1 at the start of the test, after storage for one month in a closed polyethylene bottle at 40°C and 75% relative humidity, and after storage for three months in a closed polyethylene bottle at 40°C and 75% relative humidity. The horizontal axis represents time (min), and the vertical axis represents dissolution rate (%).
[Fig. 23]

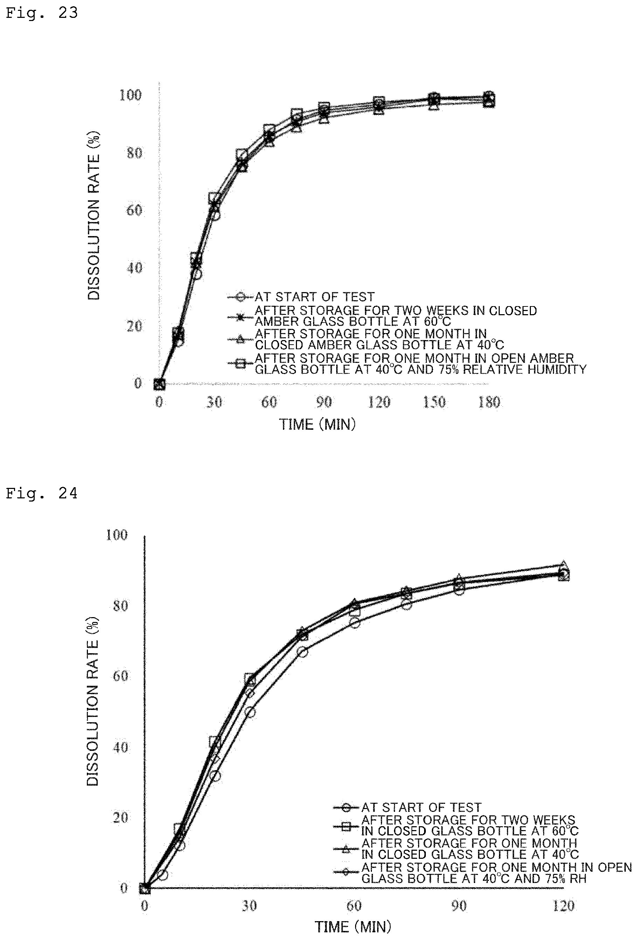

   Fig. 23 shows dissolution profiles of the tablet of Example 6B-1 at the start of the test, after storage for two weeks in a closed amber glass bottle at 60°C, after storage for one month in a closed amber glass bottle at 40°C, and after storage for one month in a closed amber glass bottle at 40°C and 75% relative humidity. The horizontal axis represents time (min), and the vertical axis represents dissolution rate (%).
[Fig. 24]
   Fig. 24 shows dissolution profiles of the tablet of Example 6B-2 at the start of the test, after storage for two weeks in a closed amber glass bottle at 60°C, after storage for one month in a closed amber glass bottle at 40°C, and after storage for one month in a closed amber glass bottle at 40°C and 75% relative humidity. The horizontal axis represents time (min), and the vertical axis represents dissolution rate (%).
[Fig. 25]

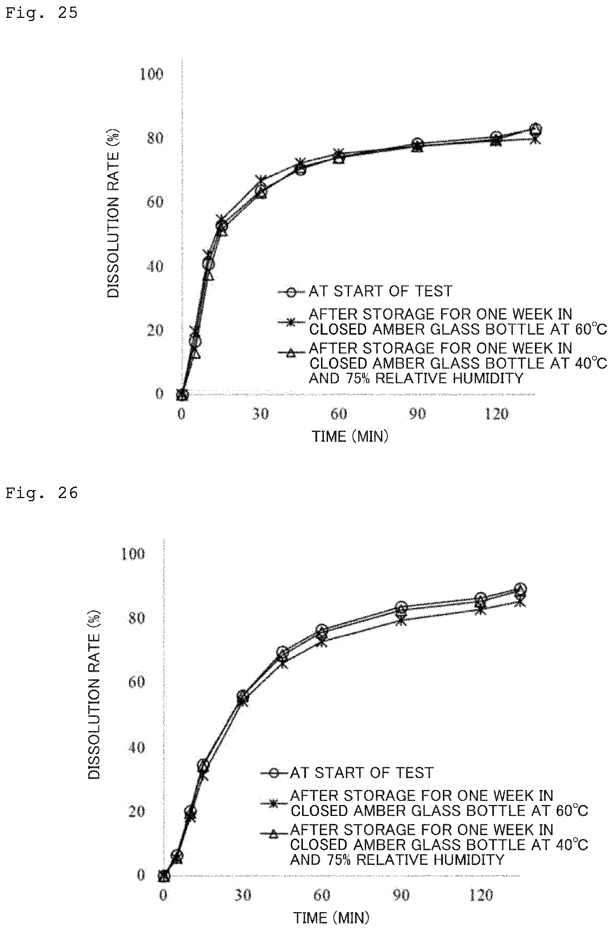

   Fig. 25 shows dissolution profiles of the tablet of Example 6C-1 at the start of the test, after storage for one week in a closed amber glass bottle at 60°C, and after storage for one week in a closed amber glass bottle at 40°C and 75% relative humidity. The horizontal axis represents time (minutes), and the vertical axis represents dissolution rate (%).
[Fig. 26]
   Fig. 26 shows dissolution profiles of the tablet of Example 6C-2 at the start of the test, after storage for one week in a closed amber glass bottle at 60°C, and after storage for one week in a closed amber glass bottle at 40°C and 75% relative humidity. The horizontal axis represents time (minutes), and the vertical axis represents dissolution rate (%).
[Fig. 27]

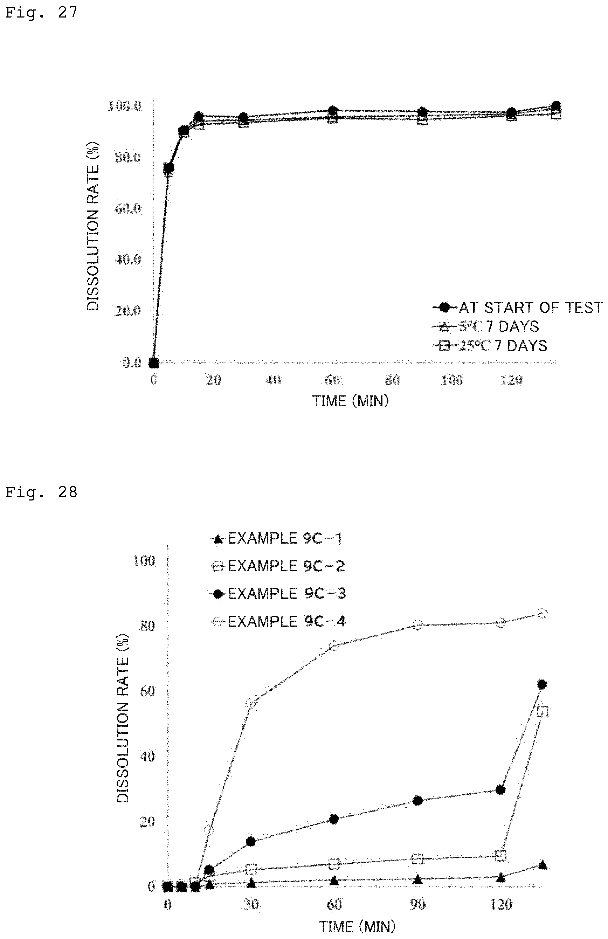

   Fig. 27 shows a dissolution profile of the solution preparation of Example 8-1. The horizontal axis represents time (minutes), and the vertical axis represents dissolution rate (%).
[Fig. 28]
   Fig. 28 shows dissolution profiles of capsules of Examples 9C-1, 9C-2, 9C-3, and 9C-4. The horizontal axis represents time (min), and the vertical axis represents dissolution rate (%).
[Fig. 29]

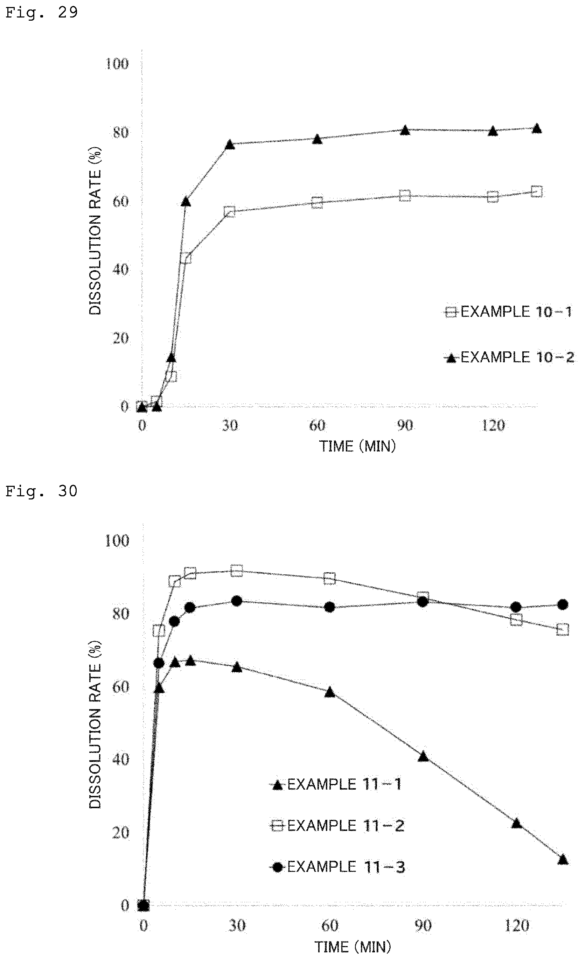

   Fig. 29 shows dissolution profiles of capsules of Examples 10-1 and 10-2. The horizontal axis represents time (min), and the vertical axis represents dissolution rate (%).
[Fig. 30]
   Fig. 30 shows dissolution profiles of solution preparations of Examples 11-1, 11-2, and 11-3 after storage at 5°C for three days. The horizontal axis represents time (min), and the vertical axis represents dissolution rate (%).

### MODE FOR CARRYING OUT THE INVENTION

Hereinafter, the meaning of each term used in the present specification will be described. Unless particularly stated otherwise, each term is used in the same sense, either alone or in combination with other terms.

The term "consist of" means having only the constituent elements. The term "comprise" and the term "encompass" mean that elements are not limited to the constituent elements, and elements that are not described are not excluded.

The present invention will be described below with reference to embodiments. Throughout this specification, it should be understood that the representation of the singular also includes the concept of the plural unless stated otherwise. Thus, unless stated otherwise, singular articles (e.g., "a", "an", "the", and the like in English) can be understood to include the concepts of the plural forms.

Unless otherwise defined, it should be understood that the terms used in the present specification are used in a meaning normally used in the art. Accordingly, unless otherwise defined, all terminologies and scientific and technical terms used in the present specification have the same meanings as commonly understood by those having ordinary skill in the art to which the present invention belongs. In the event of any inconsistency, the present specification (including the definitions) shall prevail.

Unless otherwise mentioned, numerical values in the specification and claims are approximate values. Variations in numerical values are caused by equipment calibration, equipment errors, substance purity, crystal size, sample size, temperature, and other factors.

A compound represented by Formula (I): 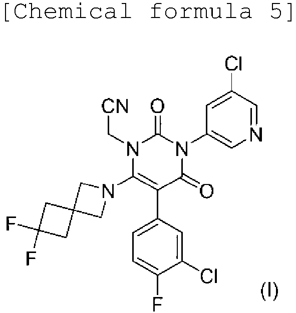 is a compound (I-077) described in WO 2023/195529 and WO 2023/195530 and can be produced by the synthesis method of Examples 5 and 6. The documents indicate that the compound represented by Formula (I) has coronavirus 3CL protease inhibitory activity and inhibits coronavirus 3CL protease. In the specification of the present application, the compound represented by Formula (I) is also referred to as Compound (I).

The compound represented by Formula (I) is not limited to specific isomers, but include all possible isomers (e.g., keto-enol isomer, imine-enamin isomers, diastereoisomers, optical isomers, rotational isomers, etc.), racemates or mixtures thereof.

One or more hydrogen atoms, carbon atoms, and/or other atoms of the compound represented by Formula (I) may be replaced with isotopes of the corresponding hydrogen atoms, carbon atoms, and/or other atoms. Examples of such isotopes include isotopes of hydrogen, carbon, nitrogen, oxygen, phosphorus, sulfur, fluorine, iodine, and chlorine, such as ²H, ³H, ¹¹C, ¹³C, ¹⁴C, ¹⁵N, ¹⁸O, ¹⁷O, ³¹P, ³²P, ³⁵S, ¹⁸F, ¹²³I, and ³⁶Cl. The compound represented by Formula (I) also encompasses compounds substituted with such isotopes (for example, deuterated compounds). Compounds substituted with such isotopes are also useful as pharmaceuticals. The compound represented by Formula (I) encompasses all radiolabeled forms substituted with radioactive isotopes among such isotopes. Furthermore, a "radiolabeling method" for producing the "radiolabeled form" is also encompassed in the present invention, and the "radiolabeled forms" are useful as tools for metabolic pharmacokinetics studies, studies on binding assay, and/or diagnostics.

Radiolabeled forms of the compound represented by Formula (I) can be prepared by methods well known in the art. For example, a tritium-labeled compound of Formula (I) can be prepared by introducing tritium into a compound of Formula (I) by a catalytic dehalogenation reaction using tritium. The method involves the reaction of tritium gas with an appropriately halogen-substituted precursor of compound represented by Formula (I) in the presence or absence of a base, in the presence of a suitable catalyst, e.g. Pd/C. Other suitable methods for preparing a tritium-labeled compound are described in "Isotopes in the Physical and Biomedical Sciences, Vol. 1, Labeled Compounds (Part A), Chapter 6 (1987)". ¹⁴C-labeled compounds can be prepared by using raw material having a ¹⁴C carbon.

The compound represented by Formula (I) as used herein may form a prodrug, and the present invention includes such various prodrugs. A prodrug is a derivative of the compound of the present invention having a chemically or metabolically cleavable group, and is a compound that becomes a pharmaceutically active compound of the present invention by solvolysis or in vivo under physiological conditions. Prodrugs encompass compounds that are enzymatically oxidized, reduced, hydrolyzed, or the like under physiological conditions in vivo to be converted into the compound represented by Formula (I), as well as compounds that are hydrolyzed by gastric acid or the like to be converted into the compound represented by Formula (I). Methods for selecting and producing suitable prodrug derivatives are described in, for example, "Design of Prodrugs, Elsevier, Amsterdam, 1985". A prodrug may have activity per se.

Since the compound according to the present invention has coronavirus 3CL protease inhibitory activity, the compound is useful as a therapeutic and/or prophylactic agent for a disease associated with coronavirus 3CL proteases. When the term "therapeutic agent and/or prophylactic agent" is used in the present invention, this also encompasses a symptom ameliorating agent. The disease associated with coronavirus 3CL proteases may be viral infections, and preferably coronavirus infections.

The coronavirus infections can be infections caused by HCoV-229E, HCoV-NL63, HCoV-OC43, HCoV-HKU1, SARS-CoV, MERS-CoV, and/or SARS-CoV-2. The coronavirus infections are preferably infections caused by HCoV-229E, HCoV-OC43, and/or SARS-CoV-2, more preferably infections caused by SARS-CoV-2.

The coronavirus infections may be particularly preferably novel coronavirus infections (COVID-19).

As used herein, the "compound represented by Formula (I)" may form salts, cocrystals, or solvates thereof.

As used herein, the "compound represented by Formula (I), a pharmaceutically acceptable salt thereof, or a solvate thereof" encompasses such various salts, cocrystals, and solvates thereof.

"Salt" as used in the present specification means that, for example, the "compound represented by Formula (I)" and a counter molecule are regularly arranged in the same crystal lattice, and may include any number of counter molecules. The salt refers to a form in which proton transfer occurs between the compound and the counter molecule in the crystal lattice, resulting in formation via ionic bonding.

As used herein, the term "cocrystal" refers to a form in which counter molecules (co-former molecules) are regularly arranged within the same crystal lattice, and may include any number of counter molecules (co-former molecules). In addition, a cocrystal refers to a form in which intermolecular interactions between a compound and counter molecules (co-former molecules) are mediated by non-covalent and non-ionic chemical interactions, such as hydrogen bonding and van der Waals forces.

Generally, salts are considered to cause proton transfer between the compound and the counter molecule, but in some cases, it is also known that proton transfer may not be complete. This state may also be referred to as cocrystals because it is not a true salt. It is also known that proton transfer may vary continuously with temperature.

Thus, the "pharmaceutically acceptable salt of the compound represented by Formula (I)" as used herein encompasses a cocrystal and refers to a pharmaceutically acceptable salt or cocrystal of the compound represented by Formula (I).

One embodiment herein is a pharmaceutically acceptable salt or cocrystal of the compound represented by Formula (I) in combination with hydrofluoric acid, hydrochloric acid, hydrobromic acid, orthophosphoric acid, hydroiodic acid, nitric acid, phosphoric acid, boric acid, methanesulfonic acid, ethanesulfonic acid, p-toluenesulfonic acid, benzenesulfonic acid, trifluoromethylbenzenesulfonic acid, chlorobenzenesulfonic acid, methoxybenzenesulfonic acid, acetic acid, propionic acid, lactic acid, citric acid, fumaric acid, malonic acid, malic acid, succinic acid, salicylic acid, maleic acid, glycerophosphoric acid, tartaric acid, benzoic acid, glutamic acid, aspartic acid, 2-naphthalenesulfonic acid, hexanoic acid, acetylsalicylic acid, and the like.

Studies of salt formation and cocrystal formation provide a means for modifying the physicochemical properties and resulting biological properties of a drug without altering its chemical structure. Salt formation and cocrystal formation can have a dramatic impact on the properties of the drug. In the selection of an appropriate salt or cocrystal, hygroscopicity, stability, solubility and processing properties are also important perspectives. Solubility of the salt or cocrystal may affect their suitability for use as a drug. When the aqueous solubility is low, the dissolution rate in vivo administration may be limited to the absorption process, resulting in low bioavailability. Also, their low water solubility may make administration by injection difficult, thus limiting the selection of the appropriate routes of administration.

The "compound represented by Formula (I)" can form a solvate with water (i.e., a hydrate) or a solvate with a common organic solvent. The "pharmaceutically acceptable salt of the compound represented by Formula (I)" can also form a solvate with water (i.e., a hydrate) or a solvate with a common organic solvent.

The "solvate" as used in the present specification means that, for example, with regard to "the compound represented by Formula (I)", the compound and any number of solvent molecules are arranged with regularity.

Examples of the solvent molecule include ethyl acetate, water, ethanol, acetone, 1,1-diethoxypropane, 1,1-dimethoxymethane, 2,2-dimethoxypropane, isooctane, isopropyl ether, methylisopropylketone, methyltetrahydrofuran, petroleum ether, trichloroacetic acid, trifluoroacetic acid, acetic acid, anisole, 1-butanol, 2-butanol, n-butyl acetate, t-butyl methyl ether, cumene, dimethyl sulfoxide, diethyl ether, ethyl formate, formic acid, heptane, isobutyl acetate, isopropyl acetate, methyl acetate, 3-methyl-1-butanol, methyl ethyl ketone, methyl isobutyl ketone, 2-methyl-1-propanol, pentane, 1-pentanol, 1-propanol, 2-propanol, propyl acetate, tetrahydrofuran, acetonitrile, chlorobenzene, chloroform, cyclohexane, 1,2-dichloroethene, dichloromethane, 1,2-dimethoxyethane, N,N-dimethylacetamide, N,N-dimethylformamide, 1,4-dioxane, 2-ethoxyethanol, ethylene glycol, formamide, hexane, methanol, 2-methoxyethanol, methylbutyl ketone, methylcyclohexane, N-methylpyrrolidone, nitromethane, pyridine, sulfolane, tetralin, toluene, 1,1,2-trichloroethene, xylene, and t-butanol.

The solvent molecule can be preferably ethyl acetate, water, ethanol, acetone, 1,1-diethoxypropane, 1,1-dimethoxymethane, 2,2-dimethoxypropane, isooctane, isopropyl ether, methylisopropylketone, methyltetrahydrofuran, petroleum ether, trichloroacetic acid, trifluoroacetic acid, acetic acid, anisole, 1-butanol, 2-butanol, n-butyl acetate, t-butyl methyl ether, cumene, dimethyl sulfoxide, diethyl ether, ethyl formate, formic acid, heptane, isobutyl acetate, isopropyl acetate, methyl acetate, 3-methyl-1-butanol, methyl ethyl ketone, methyl isobutyl ketone, 2-methyl-1-propanol, pentane, 1-pentanol, 1-propanol, 2-propanol, propyl acetate, or tetrahydrofuran.

The solvent molecule is more preferably ethyl acetate, water, ethanol, acetone, 1,1-diethoxypropane, 1,1-dimethoxymethane, 2,2-dimethoxypropane, isooctane, isopropyl ether, methylisopropylketone, methyltetrahydrofuran, petroleum ether, trichloroacetic acid, or trifluoroacetic acid.

The solvent molecule is most preferably ethyl acetate.

When the "compound represented by Formula (I)" is allowed to stand in the atmosphere, the compound may absorb water, resulting in attachment of adsorbed water or formation of a hydrate.

One embodiment herein is an "ethyl acetate solvate of the compound represented by Formula (I)". For example, the ethyl acetate solvate comprises about one molar equivalent of ethyl acetate molecules relative to the compound represented by Formula (I).

The present invention relates to a solid state (crystal or amorphous form) of the compound represented by Formula (I), a pharmaceutically acceptable salt thereof, or a solvate thereof. The solid state may be either a single state or a mixed state. The crystal may be either a single-phase crystal or a mixed crystal.

An active pharmaceutical ingredient may have substantially different physical properties depending on its solid state. Such differences in physical properties can significantly affect, for example, the bioavailability, purity, production method, pharmaceutical compositions (preparations) comprising the active pharmaceutical ingredient, or routes of administration, and therefore, selection of the solid state is extremely important in pharmaceutical development.

The present invention provides an ethyl acetate solvate crystal of the compound represented by Formula (I), an amorphous form of the compound represented by Formula (I), and a solid dispersion of the compound represented by Formula (I), which are highly useful as compared with other solid state. The present invention also provides a preparation comprising a solid dispersion of the compound represented by Formula (I), a preparation comprising an anhydrous crystal of the compound represented by Formula (I), and a preparation comprising an ethyl acetate solvate crystal of the compound represented by Formula (I), that are highly useful as compared with other formulations.

The solid states and the preparations at least have the following properties:
(1) having excellent stability against heat, humidity, solvent, light, and the like and high storage stability.
(2) exhibiting no noticeable coloring after light irradiation.
(3) having good solubility in water or organic solvents.
(4) having high dissolution rate in water or organic solvents.
(5) having high purity.
(6) having low ratio of residual organic solvents.
(7) having excellent operability in filtration, centrifugation, preparation, and the like.
(8) having small specific volume.
(9) being difficult to charge.
(10) being produced under the condition of low environmental load at high yield, and capable of being mass-produced.
(11) being useful as an active pharmaceutical ingredient for oral preparations, injectable preparations, and the like, or as a raw material for producing the same.
(12) being advantageous in that the pH can be controlled within a range suitable for intravenous injection without causing vascular pain, thereby facilitating control of the liquid volume during formulation and reduction of filler.
(13) having good fluidity.
(14) having a low compression degree (%).

"Crystal" as used in the present specification means a solid in which constituent atoms, ions, molecules, and the like are three-dimensionally arranged with regularity, and is distinguished from an amorphous solid that does not have such a regular internal structure. The crystal as used herein may be a single crystal, a twin crystal, a polycrystal, or the like.

Furthermore, the "crystal" may include "crystalline polymorphs" that have the same composition but different arrangements in the crystal, and crystals including those crystalline polymorphs are referred to as "crystal form".

The "compound represented by Formula (I), a pharmaceutically acceptable salt thereof, or a solvate thereof" encompasses a crystal polymorph.

The crystal as used herein may be a deuterated form. The crystal as used herein may be labeled with isotopes (for example, ³H, ¹⁴C, ³⁵S, ¹²⁵I, and the like).

The crystal form and/or degree of crystallinity can be confirmed, for example, by spectroscopy such as X-ray diffraction, Raman spectroscopy, infrared absorption spectroscopy, and solid-state NMR. In addition, the physical properties of the crystal can be confirmed by various techniques such as differential scanning calorimetry, moisture sorption/desorption measurements, and dissolution characteristics.

One embodiment herein is an anhydrous crystal of the compound represented by Formula (I).

As used herein, "anhydride" is synonymous with "non-solvated form", "unsolvated form", "non-hydrated form", and "anhydrous form".

In the anhydrous crystal of the compound represented by Formula (I), the theoretical content of crystalline water is 0 wt%. However, in the analysis of water content and/or solvent content, the measured value may be higher than the theoretical content of crystal water due to the influence of adsorbed water and/or adsorbed solvent adhering to the crystalline surface.

One embodiment herein is an anhydrous crystal of the compound represented by Formula (I), having characteristic peaks at diffraction angles (2θ) of 6.5°±0.2°, 15.6°±0.2°, 17.4°±0.2°, 19.9°±0.2°, and 20.3°±0.2° in an X-Ray Powder diffraction pattern (CuKα radiation, λ=1.5418 Å).

One embodiment herein is an anhydrous crystal of the compound represented by Formula (I), wherein when the anhydrous crystal is subjected to single crystal structure analysis using CuKα radiation (λ=1.5418 Å) at 298 K (25°C), it is characterized by having the following crystallographic data:
space group: Pbca
a=14.67Å±0.05Â
b=11.83Å±0.05Å
c=27.10Å+0.05Å
α=90°
β=90°
γ=90°.

One embodiment herein is an anhydrous crystal of the compound represented by Formula (I), having a melting point of 261.3°C±2°C in differential scanning calorimetry (DSC).

One embodiment herein is an anhydrous crystal of the compound represented by Formula (I), having a melting point of 265.6°C±2°C in simultaneous thermogravimetric-differential thermal analysis (TG/DTA).

One embodiment herein is an anhydrous crystal of the compound represented by Formula (I), having characteristic peaks at 415.2 cm⁻¹±2 cm⁻¹, 502.7 cm⁻¹±2 cm⁻¹, 1431.4 cm⁻¹±2 cm⁻¹, 1714.8 cm⁻¹±2 cm⁻¹, and 3065.4 cm⁻¹±2 cm⁻1 in a Raman spectrum.

The D50 of the anhydrous crystal of the compound represented by Formula (I) herein is, for example, 0.02 to 200 µm, preferably 0.1 to 150 µm, more preferably 0.2 to 100 µm. The D90 of the anhydrous crystal of the compound represented by Formula (I) herein is, for example, 0.05 to 300 µm, preferably 0.5 to 200 µm, more preferably 1.0 to 150 µm.

The D50 of the anhydrous crystal of the compound represented by Formula (I), for use in preparation of the present invention is, for example, 0.02 to 20 µm, preferably 0.1 to 10 µm, more preferably 0.2 to 8 µm. The D90 of the anhydrous crystal of the compound represented by Formula (I), for use in preparation of the present invention is, for example, 0.05 to 40 µm, preferably 0.2 to 20 µm, more preferably 0.5 to 16 µm. Depending on the type of preparation, particles of an anhydrous crystal of the compound represented by Formula (I) having a D50 of approximately 200 to 1500 nm may be used.

One embodiment of the present invention is an ethyl acetate solvate crystal of the compound represented by Formula (I). For example, the ethyl acetate solvate is a crystal comprising about one molar equivalent of ethyl acetate molecules relative to the compound represented by Formula (I).

One embodiment of the present invention is an "ethyl acetate solvate crystal of the compound represented by Formula (I)", comprising the compound represented by Formula (I) and ethyl acetate in the molar abundance ratio of 1:1. The theoretical content of ethyl acetate in the crystal is about 14.4 wt%.

The ethyl acetate content of the "ethyl acetate solvate crystal of the compound represented by Formula (I)" of the present invention is, for example, 4 to 20 wt%, preferably from 9 to 18 wt%, more preferably from 12 to 16 wt%. In the analysis of ethyl acetate content, the measured content may be higher due to the influence of water or ethyl acetate adhering to the crystal, or may be lower due to partial dissociation of ethyl acetate from the crystal prior to measurement.

One embodiment of the present invention is an ethyl acetate solvate crystal of the compound represented by Formula (I), having characteristic peaks at diffraction angles (2θ) of 6.9°±0.2°, 8.8°±0.2°, 13.1°±0.2°, 16.3°±0.2°, and 23.7°±0.2° in an X-Ray Powder diffraction pattern (CuKα radiation, λ=1.5418Å).

One embodiment of the present invention is an ethyl acetate solvate crystal of the compound represented by Formula (I), showing an endothermic peak and weight loss at 129.5°C±2°C in simultaneous thermogravimetric-differential thermal analysis (TG/DTA).

One embodiment of the present invention is an ethyl acetate solvate crystal of the compound represented by Formula (I), having characteristic peaks at 421.2 cm⁻¹±2 cm⁻¹, 509.7 cm⁻¹±2 cm⁻¹, 1585.3 cm⁻¹±2 cm⁻¹, 1709.9 cm⁻¹±2 cm⁻¹, and 3052.9 cm⁻¹±2 cm⁻¹ in a Raman spectrum.

The D50 of the ethyl acetate solvate crystal of the compound represented by Formula (I) of the present invention is, for example, from 0.1 to 200 µm, preferably from 0.3 to 150 µm, more preferably from 0.5 to 100 µm. The D90 of the ethyl acetate solvate crystal of the compound represented by Formula (I) of the present invention is, for example, from 0.2 to 300 µm, preferably from 0.5 to 200 µm, more preferably from 1 to 150 µm.

The D50 of the ethyl acetate solvate crystal of the compound represented by Formula (I), for use in preparation according to the present invention is, for example, from 0.1 to 25 µm, preferably from 0.5 to 10 µm, more preferably from 1 to 8 µm. The D90 of the ethyl acetate solvate crystal of the compound represented by Formula (I) of the present invention, for use in preparation according to the present invention is, for example, from 0.2 to 40 µm, preferably from 1 to 20 µm, more preferably from 2 to 15 µm.

One embodiment herein is an amorphous form of the compound represented by Formula (I).

As used herein, the term "amorphous form" is synonymous with "amorphous solid", "amorphous", and "glass", and refers to a solid state that does not have an ordered structure like that of a crystal. The constituent atoms, ions, or molecules do not have an ordered repeating periodicity in three dimensions. As used herein, the term "amorphous form" refers to a substantially amorphous form state. For example, it means that 80% or more, preferably 90% or more, more preferably 95% or more, most preferably 99% or more of the active pharmaceutical ingredient (drug) present in the composition is in an amorphous form. In addition, the degree of crystallinity means, for example, having a degree of crystallinity of about 20% or less, preferably about 10% or less, more preferably about 5% or less, and most preferably about 1% or less.

Since the amorphous solid lacks an ordered repeating periodicity in its structure, it does not exhibit distinct diffraction phenomena and can therefore be confirmed by X-Ray Powder diffraction spectrometry. The X-Ray Powder diffraction pattern of an amorphous solid is a broad and featureless XRPD pattern, which is also referred to as a halo pattern. In addition, since amorphous solid does not exhibit birefringence like crystals, it can be confirmed using a microscope or a digital microscope in a polarized observation mode. In addition, it can be confirmed by, for example, spectroscopy such as Raman spectroscopy, infrared absorption spectroscopy, and solid-state NMR, as well as techniques such as differential scanning calorimetry.

One embodiment of the present invention is a solid dispersion of the compound represented by Formula (I).

One embodiment of the present invention is a solid dispersion of the compound represented by Formula (I), wherein the compound represented by Formula (I) is an amorphous form.

One embodiment of the present invention is a solid dispersion of the compound represented by Formula (I), which exhibits a halo pattern in X-Ray Powder diffraction spectrometry.

One embodiment of the present invention is a solid dispersion comprising a compound represented by Formula (I) and a polymer.

The "solid dispersion" as used herein comprises an active pharmaceutical ingredient (drug) and a polymer. The solid dispersion refers to a matrix obtained by mixing the active pharmaceutical ingredient (drug) with the polymer and then solidifying the mixture, in which the amorphous active pharmaceutical ingredient (drug) is stably molecularly dispersed within the polymer. More specifically, the solid dispersion refers to a matrix obtained by once dissolving and mixing an active pharmaceutical ingredient (drug) and a polymer in a common solvent, followed by solidification, whereby the amorphous active pharmaceutical ingredient (drug) is stably molecularly dispersed within the polymer. The method for producing such a solid dispersion can be a spray-drying method (solvent removal method), a melt-heating method, a co-pulverizing method (mechanochemical method), or the like. For crystalline poorly soluble drugs, conversion into a solid dispersion allows the active pharmaceutical ingredient (drug) to be amorphized, thereby improving solubility and dissolution rate.

As used herein, the phrase "improving solubility" means an increase in the solubility of the compound represented by Formula (I) in water or a buffer solution. Specifically, for example, when the solid dispersion or a pharmaceutical composition (preparation) comprising the solid dispersion is evaluated by a dissolution test, it is defined that the solubility of solid dispersion comprising the compound represented by Formula (I) (or Compound (I) in the solid dispersion of the compound represented by Formula (I)) is at least 1.5 times the solubility of Compound (I) itself; in other embodiments, at least 2 times; in still other embodiments, at least 5 times; and in further embodiments, at least 10 times.

As used herein, the expression the "solid dispersion is stable" means that, in a stability test over time of the solid dispersion, the compound represented by Formula (I) present in an amorphous form state in the solid dispersion does not crystallize.

The polymer used in the solid dispersion according to the present invention may be any polymer that can be used in pharmacology, and it may be a mixture of two or more kinds. The polymer can be a vinyl-based polymer, cellulose-based polymer, acrylic acid-based polymer, or a polyether-based polymer. The polymer is preferably vinyl-based polymer, cellulose-based polymer, acrylic acid-based polymer, more preferably vinyl-based polymer.

The vinyl-based polymer used in the solid dispersion of the present invention can be copovidone (as used herein, polyvinyl pyrrolidone-vinyl acetate copolymer, hereinafter also referred to as PVPVA), polyvinyl pyrrolidone (as used herein, povidone), polyvinyl polypyrrolidone, polyvinyl alcohol, copolymer of polyvinyl alcohol, acrylic acid, and methyl methacrylate, poly(vinyl alcohol)-graft-poly(ethylene glycol) copolymer, polyvinyl acetal diethylaminoacetate, fumaric acid-stearic acid-polyvinyl acetal diethylaminoacetate-hydroxypropyl methylcellulose mixture, or polyvinyl acetal diethylaminoacetate. The vinyl-based polymer is preferably copovidone, polyvinyl pyrrolidone, particularly preferably copovidone.

One embodiment of the present invention is a solid dispersion comprising the compound represented by Formula (I) and copovidone.

The cellulose-based polymer used in the solid dispersion according to the present invention can be hypromellose acetate succinate (as used herein, also referred to as hydroxypropyl methylcellulose acetate succinate), hypromellose phthalate, hydroxypropyl cellulose (as used herein, also referred to as HPC), low substituted hydroxypropylcellulose, hypromellose (as used herein, hydroxypropyl methylcellulose, also referred to as HPMC), hydroxyethyl cellulose, hydroxyethyl methylcellulose, hydroxypropyl methylcellulose phthalate, methyl cellulose (as used herein, also referred to as MC), methyl hydroxyethylcellulose, carboxymethyl ethylcellulose, ethyl cellulose, crystalline cellulose, microcrystalline cellulose, crystalline cellulose-croscarmellose sodium, carmellose, carmellose sodium, carmellose calcium, powdered cellulose, or a fumaric acid-stearic acid-polyvinyl acetal diethylaminoacetate-hydroxypropyl methylcellulose mixture.

The cellulose-based polymer is preferably hypromellose acetate succinate, hypromellose phthalate, or hydroxypropyl cellulose, particularly preferably hypromellose acetate succinate.

The acrylic acid-based polymer used in the solid dispersion of the present invention can be methacrylic acid copolymer L, aminoalkyl methacrylate copolymer E, methacrylic acid copolymer LD, methacrylic acid copolymer S, aminoalkyl methacrylate copolymer RS, ethyl acrylate-methyl methacrylate copolymer, ammonio alkyl methacrylate copolymer, methyl acrylate-methacrylic acid-methyl methacrylate copolymer, or 2-methyl-5-vinylpyridine methyl acrylate-methacrylic acid copolymer. The acrylic acid-based polymer is preferably methacrylic acid copolymer L.

In the solid dispersion of the present invention, the weight ratio between the "compound represented by Formula (I), a pharmaceutically acceptable salt thereof, or a solvate thereof" and the polymer is, for example, 1:0.1 to 1:50, preferably 1:0.5 to 1:25, more preferably 1:1 to 1:10, particularly preferably 1:1 to 1:6.

One embodiment of the present invention relates to a solid dispersion, wherein the weight ratio between the compound represented by Formula (I) and copovidone is 1:3.

The solid dispersion of the present invention can be produced by a known method. For example, the solid dispersion can be produced by dissolving and/or suspending the "compound represented by Formula (I), a pharmaceutically acceptable salt thereof, or a solvate thereof" and a polymer in a pharmaceutically acceptable solvent, and distilling the solvent off.

The solvent is not particularly limited as long as it can maintain the compound represented by Formula (I) in an amorphous form state in the presence of a polymer. For example, Class 3 solvents as defined in ICH-Q3C (Guideline for Residual Solvents) (which are considered to have low toxicity and low risk to human health) may be used. Specific examples of the solvent include ketones such as acetone, alcohols such as methanol, ethanol, and propanol, methylene chloride, mixed solvent thereof, and mixed solvents thereof with water. The solvent is preferably acetone, and a mixed solvent of acetone and ethanol, more preferably acetone.

The amount of the solvent is not particularly limited as long as it is sufficient to allow the compound represented by Formula (I) to be in an amorphous form state. For example, the amount of the solvent is from 1 to 100 times by weight (w/w), preferably from 5 to 20 times by weight (w/w), relative to the total weight of the "compound represented by Formula (I), a pharmaceutically acceptable salt thereof, or a solvate thereof" and the polymer.

The method for distilling off the solvent is not particularly limited as long as it is a method for distilling off the solvent, but examples thereof include a spray drying method, a low-pressure drying method, and a ventilation drying method.

The D50 of the solid dispersion of the compound represented by Formula (I) of the present invention is, for example, from 0.1 to 500 µm, preferably from 0.5 to 200 µm, more preferably from 1 to 100 µm.

The X-Ray Powder diffraction pattern of the "solid dispersion of the compound represented by Formula (I)" of the present invention exhibits a halo pattern.

The present invention relates to a preparation comprising, as an active ingredient, "a compound represented by Formula (I), a pharmaceutically acceptable salt thereof, or a solvate thereof".

In the pharmaceutical composition of the present invention, the compound represented by Formula (I), or the like, may be present in an amount of from 1 to 100 mg, preferably from 5 to 50 mg. The amount is particularly preferably 20 to 40 mg. Examples of the preparation include oral preparations and parenteral preparations, preferably oral preparations, more preferably solid preparations for oral administration, particularly preferably tablets, granules, powders, or capsules.

Examples of oral preparations include solid preparations for internal use (for example, tablets, powders, granules, capsules, pills, films, and the like) and liquid preparations for internal use (for example, suspensions, emulsions, elixirs, syrups, lemonades, alcoholic preparations, aromatic waters, extracts, decoctions, tinctures, and the like). The tablet may be a sugar-coated tablet, a film-coated tablet, an enteric-coated tablet, a sustained release tablet, a troche, a sublingual tablet, a buccal tablet, a chewable tablet, or an orally disintegrating tablet; the powder preparation and granular preparation may be dry syrups; and the capsule may be a soft capsule, a microcapsule, or a sustained release capsule.

The parenteral preparation can be an injectable preparation, an infusion, or a preparation for external use (for example, an eye drop, a nasal drop, an ear drop, an aerosol, an inhalant, a lotion, an impregnating agent, a liniment, a gargling agent, an enema, an ointment, a plaster, a jelly, a cream, a patch, a poultice, a powder preparation for external use, or a suppository). The injectable preparation may be an O/W, W/O, O/W/O, or W/O/W type emulsion, or the like.

One embodiment of the present invention is a preparation comprising as active ingredient a compound represented by Formula (I), a pharmaceutically acceptable salt thereof, or a solvate thereof.

The "preparation comprising as active ingredient a compound represented by Formula (I), a pharmaceutically acceptable salt thereof, or a solvate thereof" of the present invention can be produced by the known method. This preparation may comprise a pharmacologically acceptable additive, such as a disintegrant, a filler, a lubricant, a binder, or a coating agent.

One embodiment of the present invention is a preparation comprising as an active ingredient a solid dispersion of the compound represented by Formula (I). One embodiment of the present invention is a preparation comprising, as an active ingredient, a solid dispersion including the compound represented by Formula (I) and copovidone.

One embodiment of the present invention is a preparation comprising as an active ingredient an anhydrous crystal of the compound represented by Formula (I).

One embodiment of the present invention is a preparation comprising as active ingredient an ethyl acetate solvate crystal of the compound represented by Formula (I).

The preferable form of the preparation can be a solid preparation, particularly preferably a granule, powder, capsule, or tablet.

The method for producing "a granule comprising, as an active ingredient, a compound represented by Formula (I), a pharmaceutically acceptable salt thereof, or a solvate thereof" is not particularly limited. Specifically, the method includes mixing the "compound represented by Formula (I), a pharmaceutically acceptable salt thereof, or a solvate thereof" with additives such as a disintegrant and a filler to prepare a mixed powder, and then granulating the mixed powder. Preferably, the granulation is performed by a wet granulation method in which water or an aqueous or solvent-based solution containing a binder is added, a dry granulation method in which granulation is performed by compression without using water, or a melt granulation method. As a mixer for mixing the active pharmaceutical ingredient (drug) and additives, a V-type mixer or a container blender may be used. In addition, granulation may be carried out using a wet extrusion granulator, a fluidized bed granulator, an agitation granulator, a dry crushing granulator, or a melt extrusion granulator.

The method for producing "a tablet comprising, as an active ingredient, a compound represented by Formula (I), a pharmaceutically acceptable salt thereof, or a solvate thereof" is not particularly limited. Specifically, the method includes producing granules by the above-described method, further mixing the granules with a filler, a disintegrant, a lubricant, and the like, and compressing the resulting mixed granules using a tableting machine. Alternatively, the method includes mixing an active pharmaceutical ingredient (drug) with a filler, a disintegrant, a lubricant, and the like, and directly compressing the resulting mixture using a tableting machine.

A V-type mixer or a container blender can be used as a mixer for mixing the active ingredient and additives. In addition, a single-punch tableting machine, a rotary tableting machine, or the like may be used as the tableting machine.

The disintegrant can be croscarmellose sodium, carmellose, carmellose calcium, carmellose sodium, hydroxypropyl cellulose, low substituted hydroxypropylcellulose, crystalline cellulose, powdered cellulose, partially pregelatinized starch, potato starch, cornstarch, hydroxypropyl starch, sodium carboxymethyl starch, low-substituted sodium carboxymethyl starch, sodium starch glycolate, pregelatinized starch, starch, polyvinyl alcohol, crospovidone, or the like. The disintegrant is preferably croscarmellose sodium, low substituted hydroxypropylcellulose, sodium starch glycolate, or crospovidone, particularly preferably croscarmellose sodium or crospovidone.

One embodiment of the present invention is a solid preparation comprising a solid dispersion of the compound represented by Formula (I) and croscarmellose sodium.

One embodiment of the present invention is a solid preparation comprising a solid dispersion including the compound represented by Formula (I) and copovidone and croscarmellose sodium.

One embodiment of the present invention is a solid preparation comprising the anhydrous crystal of the compound represented by Formula (I) and crospovidone.

A preferred form of the solid preparation can be a granule, a powder, a capsule, or a tablet, and a particularly preferred form is a tablet.

The filler can be crystalline cellulose, silicified microcrystalline cellulose, lactose, anhydrous lactose, sucrose, glucose, fructose, sucrose, mannitol, sorbitol, erythritol, xylitol, maltodextrin, maltitol, starch, potato starch, corn starch (as used herein, corn starch may also be referred to simply as "corn starch"), rice starch, partially pregelatinized starch, pregelatinized starch, porous starch, sodium carboxy starch, hydroxypropyl starch, low-substituted sodium carboxymethyl starch, powdered cellulose, carmellose sodium, carmellose, carmellose calcium, carboxymethyl ethylcellulose, low substituted hydroxypropylcellulose, silicate derivative, phosphate, carbonate, sulfate, magnesium oxide, titanium oxide, calcium lactate, synthetic hydrotalcite, talc, kaolin, dried aluminum hydroxide, magnesium oxide, bentonite, silicon dioxide such as hydrated silica and light anhydrous silicic acid, magnesium aluminometasilicate, synthetic aluminum silicate, calcium silicate, anhydrous dibasic calcium phosphate, calcium monohydrogen phosphate, calcium hydrogen phosphate, sodium hydrogen phosphate, dibasic potassium phosphate, potassium dihydrogen phosphate, calcium dihydrogen phosphate, sodium dihydrogen phosphate, precipitated calcium carbonate, calcium carbonate, magnesium carbonate, calcium sulfate, or the like. The filler is preferably crystalline cellulose and/or mannitol.

One embodiment of the present invention is a solid preparation comprising a solid dispersion of the compound represented by Formula (I), croscarmellose sodium, crystalline cellulose, and mannitol.

One embodiment of the present invention is a solid preparation comprising a solid dispersion including the compound represented by Formula (I) and copovidone, croscarmellose sodium, crystalline cellulose, and mannitol.

One embodiment of the present invention is a solid preparation comprising an anhydrous crystal of the compound represented by Formula (I), crospovidone, and crystalline cellulose.

A preferred form of the solid preparation can be a granule, a powder, a capsule, or a tablet, and a particularly preferred form is a tablet.

The lubricant can be sodium stearyl fumarate, light anhydrous silicic acid, magnesium stearate, calcium stearate, stearic acid, stearyl alcohol, polyoxyl stearate 40, talc, hydrated silica, magnesium carbonate, precipitated calcium carbonate, dried aluminum hydroxide gel, magnesium aluminometasilicate, magnesium silicate, synthetic aluminum silicate, magnesium oxide, magnesium sulfate, cocoa butter, carnauba wax, glycerine fatty acid ester, hydrogenated oil, bleached beeswax, hydrogenated soybean oil, beeswax, cetanol, sodium laurate, sucrose fatty acid ester, and polyethylene glycol (as used herein, the polyethylene glycol may also be referred to as macrogol), or the like. The lubricant is preferably sodium stearyl fumarate, light anhydrous silicic acid, magnesium stearate, or calcium stearate, particularly preferably sodium stearyl fumarate and/or light anhydrous silicic acid.

One embodiment of the present invention is a solid preparation comprising a solid dispersion of the compound represented by Formula (I), croscarmellose sodium, crystalline cellulose, mannitol, sodium stearyl fumarate, and light anhydrous silicic acid.

One embodiment of the present invention is a solid preparation comprising a solid dispersion including the compound represented by Formula (I) and copovidone, croscarmellose sodium, crystalline cellulose, mannitol, sodium stearyl fumarate, and light anhydrous silicic acid.

One embodiment of the present invention is a solid preparation comprising an anhydrous crystal of the compound represented by Formula (I), crospovidone, crystalline cellulose, and sodium stearyl fumarate.

A preferred form of the solid preparation can be a granule, a powder, a capsule, or a tablet, and a particularly preferred form is a tablet.

The "preparation comprising, as an active ingredient, a compound represented by Formula (I), a pharmaceutically acceptable salt thereof, or a solvate" of the present invention may further comprise a photostabilizing agent.

One embodiment of the present invention is a "preparation comprising a compound represented by Formula (I), a pharmaceutically acceptable salt thereof, or a solvate thereof, and a photostabilizing agent".

The photostabilizing agent in the preparation according to the present invention may be blended into the preparation or may be applied to coat the surface of the preparation; however, preferably, the preparation comprises a coating layer covering the surface of the preparation and containing the photostabilizing agent. By including the photostabilizing agent in the coating layer of the preparation, the photostability of the compound represented by Formula (I) comprised in the preparation can be improved, or discoloration of the preparation can be prevented.

The preparation according to the present invention comprises a coating layer, and the coating layer comprises a photostabilizing agent and a polymer. In the preparation of the present invention, after a granule or tablet is produced, the granule or tablet may be coated with a coating layer. The preparation according to the present invention may be a coated tablet or coated granule.

When the coating layer is formed on the granule, a fluidized bed granulating/coating machine, a fluidized bed rolling coating machine, or the like can be used. When the coating layer is formed on the tablet, a pan coating machine, an air-permeable coating machine, or the like can be used. The coating liquid is sprayed onto the granules or tablets while fluidizing in a coating machine, and dried to form a coating layer.

One embodiment of the present invention is "a solid preparation comprising, as active ingredient, a compound represented by Formula (I), a pharmaceutically acceptable salt thereof, or a solvate thereof, having a coating layer". One embodiment of the present invention is "a solid preparation comprising, as active ingredient, a compound represented by Formula (I), a pharmaceutically acceptable salt thereof, or a solvate thereof, having a coating layer comprising a photostabilizing agent and a polymer".

Preferred forms of "the compound represented by Formula (I), a pharmaceutically acceptable salt thereof, or a solvate thereof" include a solid dispersion of the compound represented by Formula (I), an anhydrous crystal of the compound represented by Formula (I), or an ethyl acetate solvate crystal of the compound represented by Formula (I).

A preferred form of the solid preparation can be a granule, a powder, a capsule, or a tablet, and a particularly preferred form is a tablet or a capsule.

The photostabilizing agent can be a light-shielding agent having a light-shielding effect or a light-absorbing agent having a light-absorbing effect. Examples of the photostabilizing agent include Food Red No. 2, Food Red No. 3, Food Red No. 102, Food Red No. 104, Food Red No. 105, Food Red No. 106, Food Yellow No. 4, Food Yellow No. 5, Food Green No. 3, Food Blue No. 1, Food Blue No. 2, Food Red No. 3 aluminum lake, Food Yellow No. 4 aluminum lake, Food Yellow No. 5 aluminum lake, Food Blue No. 1 aluminum lake, Food Blue No. 2 aluminum lake, carmine, sodium copper chlorophyllin, copper chlorophyll, red iron oxide, black iron oxide, yellow iron oxide, titanium oxide, red ferric oxide, yellow ferric oxide, and talc. The photostabilizing agent is preferably red ferric oxide, yellow ferric oxide, and/or talc.

The polymer in the coating layer can be hypromellose, hydroxypropyl cellulose, carboxymethyl ethylcellulose, hypromellose phthalate, hydroxypropyl methylcellulose acetate succinate, ethyl cellulose, or polyvinyl alcohol. The polymer is preferably hypromellose.

One embodiment of the present invention is a "solid preparation comprising, as an active ingredient, a solid dispersion of a compound represented by Formula (I), having a coating layer comprising red ferric oxide, yellow ferric oxide, and/or talc". One embodiment of the present invention is a "solid preparation comprising, as an active ingredient, a solid dispersion of a compound represented by Formula (I), having a coating layer comprising red ferric oxide, yellow ferric oxide, talc, and hypromellose".

One embodiment of the present invention is a "solid preparation comprising, as an active ingredient, an anhydrous crystal of the compound represented by Formula (I), having a coating layer comprising red ferric oxide, yellow ferric oxide, and/or talc". One embodiment of the present invention is a "solid preparation comprising, as an active ingredient, a anhydrous crystal of a compound represented by Formula (I), having a coating layer comprising red ferric oxide, yellow ferric oxide, talc, and hypromellose".

A preferred form of the solid preparation can be a granule, a powder, a capsule, or a tablet, and a particularly preferred form is a tablet.

The content of the coating layer in the solid preparation according to the present invention is not particularly limited, but is 0.1 to 20 wt%, preferably 0.5 to 10 wt%, more preferably 1 to 8 wt%, with respect to the total amount of preparation. When two or more types of the coating layers are used, it is sufficient that the total amount of the coating layer falls within the above-mentioned range.

The solid preparation of the present invention may further comprise a plasticizer together with the photostabilizing agent and the polymer, and as the plasticizer, any plasticizer described in the Japanese Pharmacopoeia, the Japanese Pharmaceutical Codex, the Japanese Pharmaceutical Excipients Standards, the Specifications and Standards for Food Additives, or the like can be used. Examples of the plasticizer include citric acid ester, glycerol fatty acid ester, surfactant, monostearin, diethyl phthalate, dibutyl phthalate, diethyl sebacate, and dibutyl sebacate.

One embodiment of the present invention is a "solid preparation comprising an ethyl acetate solvate crystal of the compound represented by Formula (I), croscarmellose sodium, mannitol, crystalline cellulose, and sodium stearyl fumarate". A preferred form of the solid preparation can be a granule, a powder, a capsule, or a tablet, and a particularly preferred form is a capsule.

### (X-Ray Powder Diffraction (XRPD))

X-Ray powder diffraction (XRPD) is one of the most sensitive analytical techniques for measuring crystal forms and crystallinity of solids. When the crystal is irradiated with X-rays, they are reflected by crystal lattice planes and interfere with each other, resulting in ordered diffracted lines corresponding to the periodicity of the crystal structure. In contrast, amorphous solids generally lack an ordered repeating periodicity in their structure, and therefore do not exhibit diffraction phenomena, instead showing a broad and featureless XRPD pattern (also referred to as a halo pattern).

Characteristic diffraction peaks used in the present specification are peaks selected from an observed diffraction pattern. Characteristic diffraction peaks are preferably selected from about 10 peaks, more preferably about 5 peaks in a diffraction pattern.

When distinguishing a plurality of crystals, a peak that is identified in the relevant crystal and is not identified in other crystals becomes a characteristic peak preferable for characterizing the crystal, rather than the intensity of a peak. With such characteristic peaks, even one or two peaks can characterize the crystal. When the patterns obtained by measurement are compared and these characteristic peaks are found to coincide, it can be said that the X-Ray powder diffraction patterns substantially coincide.

Generally, since the diffraction angle (2θ) in X-Ray powder diffraction may cause an error within the range of ±0.2°, it should be understood that a value of the diffraction angle of X-Ray powder diffraction also includes numerical values within the range of about ±0.2°. Therefore, not only crystals in which the diffraction angles of peaks in X-Ray powder diffraction perfectly coincide, but also crystals in which the diffraction angles of peaks coincide with an error of about ±0.2°, are included in the present invention.

It is known that the intensity of a peak indicated in the following drawings may fluctuate due to many factors, for example, the effect of selective orientation of crystals with respect to an X-ray beam, the influence of coarse particles, the purity of the substance to be analyzed, or the crystallinity of a sample. In addition, the peak positions may shift depending on height variations in sample. Furthermore, when measurements are performed using different wavelengths, different shifts are obtained in accordance with Bragg's equation (nλ = 2d sinθ); however, such XRPD patterns obtained using different wavelengths are also included within the scope of the present invention.

By combining an X-Ray powder diffraction apparatus with a temperature and/or relative humidity control device, X-Ray powder diffraction spectrometry can be performed under a specific temperature and/or relative humidity.

For example, in the cases where an anhydrous crystal is converted into a hydrate crystal due to moisture absorption, where a hydrate crystal is converted into an anhydrous crystal due to dehydration, where a solvate crystal undergoes crystal transition into an anhydrous crystal due to solvent desorption, or where an anhydrous crystal and a hydrate crystal reversibly interconvert due to moisture absorption and dehydration, measurements under a specific temperature and/or relative humidity, or measurements in which the temperature and/or relative humidity are continuously varied, are suitable. In such cases, it is possible to infer the relative humidity and amount of crystal water and/or crystal solvent where the change occurs.

The crystal form of the compound represented by Formula (I) can be identified by an X-Ray powder diffraction pattern and the diffraction angles (2θ) of characteristic peaks. The crystal form of the compound represented by Formula (I) (for example, the anhydrous form of the compound represented by Formula (I) or the ethyl acetate solvate crystal of the compound represented by Formula (I)) can be distinguished from other crystal forms by the presence of characteristic peaks.

### (Simultaneous X-Ray Powder Diffraction-Differential Scanning Calorimetry (XRD-DSC))

Simultaneous measurement of X-Ray powder diffraction and differential scanning calorimetry (XRD-DSC measurement) allows simultaneous observation of changes in crystal form (crystal structure) and heat flow in response to temperature and/or relative humidity. For example, this method is suitable for observing changes in crystal form (crystal structure) in the cases where an anhydrous crystal undergoes crystal transition to a hydrate crystal due to moisture absorption, where a hydrate crystal undergoes crystal transition to an anhydrous crystal due to dehydration, where a solvate crystal undergoes crystal transition to an anhydrous crystal due to solvent desorption, or where anhydrous and hydrate crystals undergo reversible crystal transition due to moisture absorption and dehydration.

### (Single Crystal Structure Analysis)

Single crystal structure analysis is one method for identifying a crystal form and enables the determination of crystallographic parameters, as well as atomic coordinates (values indicating the spatial relationships of individual atoms) and a three-dimensional structural model. For single crystal structure analysis, reference may be made to, for example, Toshio Sakurai, Guide to X-ray Structure Analysis, Shokabo Publishing (1983), and Stout & Jensen, X-Ray Structure Determination: A Practical Guide, Macmillan Co., New York

(1968). Single crystal structure analysis is useful for identifying the structures of optical isomers, tautomers, geometric isomers, salts, cocrystals, and solvates (hydrates).

It is known that the quality of data obtained by single crystal X-ray diffraction experiments is improved by performing measurements at low temperature; therefore, measurements are carried out at room temperature or at low temperature depending on the crystal to be measured. In addition, for single crystal X-ray diffraction experiments of hydrates and/or solvates, it is known that coating the crystal with paraffin oil or the like prior to measurement improves data quality.

### (Raman Spectroscopy)

Raman spectroscopy shows the characteristics of the oscillation of a molecular or composite system. Its origin lies in the inelastic collision between molecules and photons, which are particles of light including light rays. Collision between molecules and photons results in the exchange of energy, which results in a change in energy, and thereby the wavelength of the photons changes. That is, since the Raman spectrum is a spectral line of very narrow wavelengths, which is emitted when photons are incident on a molecule of interest, lasers and the like are used as light sources. The wavelength of each Raman line is indicated by the wavenumber shift from incident light, and this is the difference between the Raman line and the reciprocal of the wavelength of incident light. Raman spectroscopy is to measure the state of oscillation of molecules, and this is determined by the molecular structure thereof.

In general, the wavelength shift (cm⁻¹) in a Raman spectrum may involve an error within a range of ±2 cm⁻¹; therefore, the values of Raman spectral peaks should be understood to include values within a range of approximately ±2 cm⁻¹. Therefore, not only the crystals whose Raman spectral peaks in the Raman spectra perfectly coincide, but also the crystals whose Raman spectral peaks coincide with errors of about ±2 cm⁻¹, are included in the present invention.

### (Infrared Absorption Spectroscopy (IR Spectroscopy))

Infrared absorption spectroscopy is a method for measuring, at each wavenumber, the degree to which infrared radiation is absorbed as it passes through a sample. An infrared absorption spectrum is typically presented as a graph with wavenumber on the horizontal axis and transmittance or absorbance on the vertical axis. The wavenumbers and transmittance (or absorbance) of absorption peaks can be read directly from the graph, or values calculated by a data processing device may be used. An infrared absorption spectrum is determined by the chemical structure of the substance. Accordingly, a substance can be identified or quantified by measuring absorption at various wavenumbers. Discrimination of crystal polymorphs can be carried out by comparing absorption bands attributable to functional groups characteristic of each polymorph, that is, functional groups mainly involved in hydrogen bonding within the crystal structure, such as C=O bonds, OH bonds, and NH bonds, as well as other characteristic functional groups, for example, C-X (halogen) bonds, C=C bonds, and C≡C bonds. About 20 absorption peaks corresponding to characteristic functional groups, preferably about 10 absorption peaks, and most preferably about 5 absorption peaks are selected. Typically, the absorption spectrum of a sample is measured over a wavenumber range of 4000 cm⁻¹ to 400 cm⁻¹. Measurement of the absorption spectrum is performed under the same operating conditions as those used for confirming the instrument resolution, wavenumber scale, and wavenumber accuracy.

In general, absorption bands (cm⁻¹) in infrared absorption spectroscopy may involve an error within a range of ±2 cm⁻¹; therefore, the above absorption peak values should be understood to include values within a range of approximately ±2 cm⁻¹. Accordingly, the present invention includes not only crystals in which the absorption band peaks in infrared absorption spectroscopy exactly coincide, but also crystals in which the absorption band peaks coincide within an error of approximately ±2 cm⁻¹.

Methods for measuring infrared absorption spectra include the potassium bromide pellet method, solution method, paste method, liquid film method, thin film method, gas sample measurement method, attenuated total reflection (ATR) method, and diffuse reflectance method, among others. Among these, the ATR method, also referred to as a total reflection measurement method, is one type of reflection method. In this method, a sample is brought into close contact with the surface of a prism made of a material having a high refractive index, such as KRS-5, and light is incident on the prism at an angle greater than the critical angle. The absorption spectrum is obtained by measuring the light totally reflected at the interface between the prism and the sample. One of the conditions for measurement by the ATR method is that the refractive index of the prism is higher than that of the sample; therefore, it is necessary to change the prism material depending on the sample. Another condition is that the prism and the sample must be in close contact. Accordingly, the ATR method is suitable for measuring liquids, powders, plastics, soft rubbers, and the like, and has the advantage that measurements can be performed without chemically or physically treating the sample. On the other hand, the diffuse reflectance method is a technique in which a powder sample is measured as it is, without preparing a potassium bromide pellet. When light is irradiated onto a powder sample, light that is specularly reflected from the powder surface and light that enters the interior of the sample, undergoes repeated transmission and diffusion, and then emerges from the surface as diffusely reflected (scattered) light are generated. In the diffuse reflectance method, the latter diffusely reflected light is used to obtain the absorption spectrum.

### (Solid-state ¹³C-NMR (Nuclear Magnetic Resonance))

Solid-state ¹³C-NMR is useful for identifying crystal forms because (i) the number of signals corresponds to the number of carbon atoms in the target compound, (ii) the chemical shift range is wider than that of ¹H-NMR, (iii) the signals are sharper than those in solid-state ¹H-NMR, and (iv) even in the presence of additives, the chemical shifts do not change when no interaction is present, among other reasons. It is noted that the chemical shifts observed may slightly vary depending on the specific spectrometer used and the sample preparation technique employed by the analyst. The error range in a solid-state ¹³C-NMR spectrum is approximately ±0.5 ppm.

### (Differential Scanning Calorimetry (DSC))

Differential scanning calorimetry (DSC) is one of important measurement methods for thermal analysis and is a method of measuring the thermal properties of a substance as an aggregate of atoms and molecules.

By DSC, changes in heat flow of an active pharmaceutical ingredient with respect to temperature or time are measured, and a differential scanning calorimetry curve is obtained by plotting the resulting data against temperature or time. From the differential scanning calorimetry curve, information regarding the onset temperature of melting (extrapolated onset temperature), the maximum of the endothermic peak curve associated with melting, and enthalpy of the active pharmaceutical ingredient can be obtained.

With regard to DSC, it is known that the temperature to be observed may depend on the rate of temperature change as well as the sample preparation technique and the particular apparatus used. Therefore, the "melting point" in DSC refers to the onset temperature (extrapolated onset temperature) that is not likely to be affected by the sample preparation technique. The error range for the onset temperature (extrapolated onset temperature) obtainable from the differential scanning calorimetry curve is approximately ±2°C. For the recognition of identity of crystals, not only the melting point but also the overall pattern are important, and there may be some variation depending on the measurement conditions and the measuring equipment.

### (Simultaneous Thermogravimetric-Differential Thermal Analysis (TG/DTA))

Simultaneous thermogravimetric-differential thermal analysis (TG/DTA) is one of important measurement methods for thermal analysis and is a method of measuring the weight and thermal properties of a substance as an aggregate of atoms and molecules.

TG/DTA is a method of measuring the temperature-related or time-related changes in weight and calorific value of an active pharmaceutical ingredient, and a TG (thermogravimetric) and DTA (differential thermal) curve is obtained by plotting obtained data with respect to temperature or time. From the TG/DTA curve, information on the changes in weight and calorific value in relation to degradation, dehydration, oxidation, reduction, sublimation, and evaporation of an active pharmaceutical ingredient can be obtained.

With regard to TG/DTA, it is known that the temperature to be observed and the weight change may depend on the rate of temperature change as well as the sample preparation technique and the particular apparatus used. Therefore, the "melting point" in TG/DTA refers to the onset temperature (extrapolated onset temperature) that is not likely to be affected by the sample preparation technique. For the recognition of identity of crystals, not only the melting point but also the overall pattern are important, and there may be some variation depending on the measurement conditions and the measuring equipment.

### (Dynamic Vapor Sorption (DVS))

Dynamic vapor sorption (DVS) is a measurement method in which changes in weight of a solid under various relative humidity conditions are measured, thereby measuring moisture adsorption and desorption behavior.

As a basic measurement method, the dry weight at 0% RH (0% relative humidity) is used as a reference, the relative humidity is increased in increments of 5% or 10%, and, after the weight stabilizes at each relative humidity, the amount of adsorbed water can be determined from the increase in weight relative to the reference value. Similarly, the amount of desorbed water can be measured by decreasing the relative humidity in increments of 5% or 10% from 100% RH or 95% RH.

An adsorption/desorption isotherm can be obtained by plotting the weight change values at each relative humidity. From these results, it is possible to discuss the adsorption and desorption behavior of adhering moisture at each humidity level.

Since the adsorption and desorption of adhering water and crystalline water are affected by particle size, degree of crystallinity, crystal habit, and the like, the measurement results may vary to some extent.

Differential scanning calorimetry (DSC), simultaneous thermogravimetric-differential thermal analysis (TG/DTA), dynamic vapor sorption (DVS), Karl Fischer titration, and gas chromatography are analytical methods that also detect adhering water and/or adhering solvent (residual solvent) on the crystal surface. In such analyses, when a sample having adhering water and/or adhering solvent on the crystal surface is measured, the measured water content and/or solvent content may be higher than the theoretical content of crystal water in a hydrate crystal and/or the theoretical content of solvent of crystallization in a solvate crystal.

In contrast, X-Ray powder diffraction and single crystal structure analysis are measurement methods for analyzing the "internal structure" of a crystal, and therefore show characteristic peaks at the same positions regardless of the presence or absence of adhering water and/or adhering solvent (residual solvent) on the crystal surface. Raman spectroscopy and infrared absorption spectroscopy (IR) are similar in this respect. Accordingly, in X-Ray powder diffraction, single crystal structure analysis, Raman spectroscopy, and infrared absorption spectroscopy (IR), even when a higher water content and/or solvent content than the theoretical content in the crystal is observed, the crystal can be interpreted as being substantially the same crystal, as long as it exhibits the characteristic peaks described in the present specification.

### (Particle Size Distribution)

As used herein, "D10, D50, and D90" refer to particle diameters at which a cumulative curve reaches 10%, 50%, and 90%, respectively, when the total volume of a powder population is taken as 100%. These values can be measured by a dry method or a wet method.

### Examples

Hereinafter, the present invention will be described in more detail by way of Examples, Reference Examples, and Test Examples; however, the present invention is not intended to be limited by these. With respect to numerical values (e.g., amounts, temperatures, etc.), some degree of error and deviation should be taken into consideration.

Unless otherwise specified, percentage (%) refers to weight percentage (wt%) based on the weight of the component and the total weight of the composition, and pressure is atmospheric pressure or pressure close thereto.

### (Method for Identifying Compound)

The NMR analysis obtained in each Example was performed at 400 MHz, and measurement was made using DMSO-d₆ or CDCl₃.

Furthermore, when NMR data are shown, there are occasions in which not all the measured peaks are described.

The term "RT" in the specification indicates retention time in an LC/MS: liquid chromatography/mass spectrometry, and the retention time was measured under the following conditions.

### (Measurement Conditions A)

Column: ACQUITY UPLC (registered trademark) BEH C18 (1.7 µm, i.d. 2.1 × 50 mm) (Waters)
Flow rate: 0.8 mL/min
UV detection wavelength: 254 nm
Mobile phase: [A] is 0.1% formic acid-containing aqueous solution, and [B] is 0.1% formic acid-containing acetonitrile solution.
Gradient: A linear gradient of 5% to 100% solvent [B] was carried out for 3.5 minutes, and then 100% solvent [B] was maintained for 0.5 minutes.

### (Measurement Conditions B)

Column: ACQUITY UPLC (registered trademark) BEH C18 (1.7 µm, i.d. 2.1 × 50 mm) (Waters)
Flow rate: 0.8 mL/min
UV detection wavelength: 254 nm
Mobile phase: [A] is 0.1% formic acid-containing aqueous solution, and [B] is 0.1% formic acid-containing acetonitrile solution.
Gradient: A linear gradient of 5% to 100% solvent [B] was carried out for 3.5 minutes, and then 100% solvent [B] was maintained for 0.5 minutes.

In the specification, the term MS (m/z) indicates a value measured by mass spectrometry.

### (Method for Producing Compound Represented by Formula (I))

The "compound represented by Formula (I)" is a compound (I-077) described in WO 2023/195529 and WO 2023/195530 and can be produced by the synthesis method of Examples 5 and 6. The compound can be synthesized with reference to methods known in the field. Regarding extraction, purification, and the like, the treatments carried out in ordinary experiments of organic chemistry may be carried out.

### (Example 1: Synthesis of Compound Represented by Formula (I) (Compound (I)))

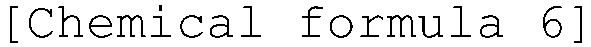

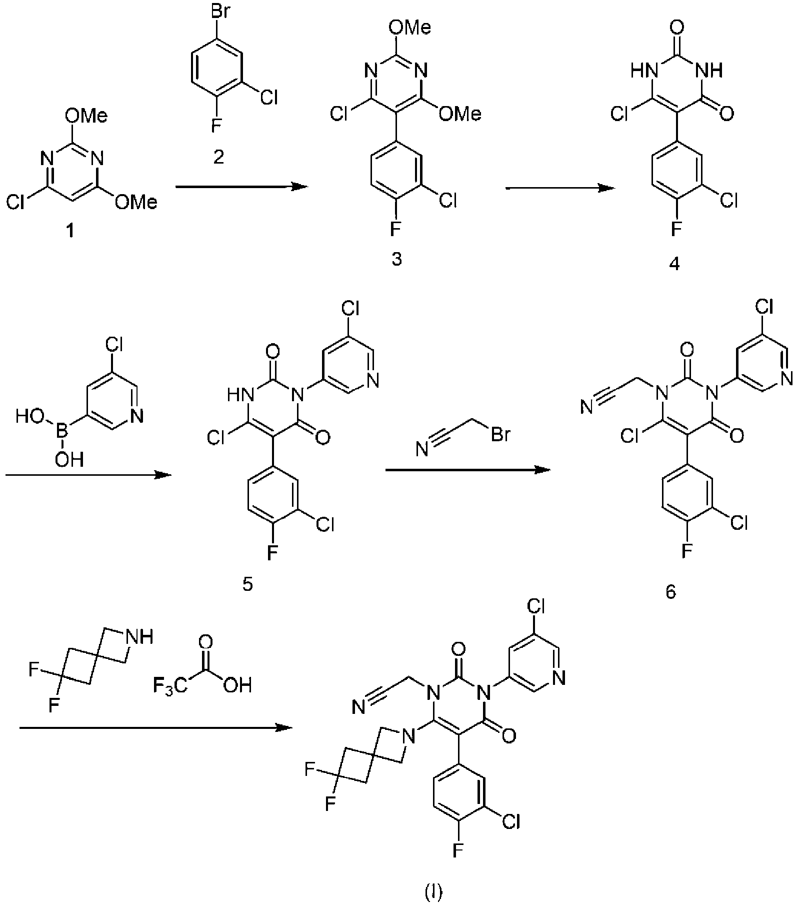

### Step 1: Synthesis of Compound 3

To a mixture solution of a 2.64 mol/L n-butyllithium solution in hexane (31 mL, 82.4 mmol) and tetrahydrofuran (20 mL), a solution of Compound 1 (12 g, 68.7 mmol) in tetrahydrofuran (70 mL) was added dropwise over 15 minutes at -78°C, and the resultant was stirred at -78°C for 1 hour. A solution of 1.9 mol/L zinc chloride in 2-methyltetrahydrofuran (43 mL, 82.4 mmol) was added dropwise over 5 minutes. The resultant was stirred at room temperature for 2 hours. Compound 2 (9.1 mL, 75.6 mmol) and tetrakis(triphenylphosphine)palladium (4.0 g, 3.44 mmol) were added and stirred at 80°C for 1.5 hours. The reaction mixture was cooled to room temperature, water (80 mL) and 2 mol/L hydrochloric acid (40 mL) were added and subjected to extraction with ethyl acetate. The organic layer was concentrated under reduced pressure and to the resulting residue, isopropanol (40 mL) was added. The precipitate was filtered off and washed with isopropanol. After air drying, Compound 3 (14.8 g, 49 mmol) was obtained.
¹H-NMR(CDCl₃)δ:3.95(s,3H), 4.05(s,3H), 7.18-7.23(m,2H), 7.35(d,J=6.8Hz,1H)
LC/MS(ESI):m/z=303, RT=2.70min, LC/MS Measurement Conditions A

### Step 2: Synthesis of Compound 4

To Compound 3 (14.8 g, 48.8 mmol) acetic acid (40 mL) and concentrated hydrochloric acid (41 mL) were added and stirred at 110°C for 5 hours. The reaction solution was cooled to room temperature and then water (80 ml) was added. The precipitate was filtered off and washed with water. After air drying, Compound 4 (11.6 g, 42.2 mmol) was obtained.
¹H-NMR(DMSO-d6)δ:7.31(ddd,J=8.8,4.9,2.1Hz,1H), 7.45(t,J=8.8Hz,1H), 7.53(dd,J=7.3,2.1Hz,1H), 11.57(s,1H), 12.24(brs,1H)
LC/MS(ESI):m/z=275, RT=1.81min, LC/MS Measurement Conditions A

### Step 3: Synthesis of Compound 5

Compound 4 (1.00 g, 3.64 mmol), 5-chloropyridine-3-boronic acid (1.14 g, 7.27 mmol), copper(II) acetate (0.99 g, 5.45 mmol), acetonitrile (10 mL), triethylamine (5.04 mL, 36.4 mmol), and pyridine (7.34 mL, 91.0 mmol) were mixed, and the solution was stirred overnight at room temperature. An aqueous saturated sodium bicarbonate solution (5 mL) was added to the reaction solution, and the mixture was subjected to extraction with ethyl acetate. The organic layer was washed with water, dried over sodium sulfate and filtered. The filtrate was concentrated and the obtained residue was purified by silica gel column chromatography (chloroform:methanol = 100:0 to 90:10), and the solvent was distilled off under reduced pressure. The resulting residue was dried in vacuo to yield Compound 5 (1.15 g, 2.97 mmol, yield: 82%).
¹H-NMR(DMSO-d₆)δ:7.35-7.37(1H,m),7.49(1H,t,J=9.0Hz),7.54-7.56(1H,m),8.07(1H,t,J=2.1Hz),8.54(1H,d,J=2.0Hz),8.70(1H,d,J=2 .3Hz).
LC/MS(ESI):m/z=386, RT=1.98min, LC/MS Measurement Conditions A

### Step 4: Synthesis of Compound 6

To a solution obtained by mixing Compound 5 (520 mg, 1.345 mmol), N,N-diisopropylethylamine (0.705 mL, 4.04 mmol), and DMF (5.2 mL) was added 2-bromoacetonitrile (269 µL, 4.04 mmol) and stirred overnight at room temperature. 2 mol/L hydrochloric acid (2 mL) was added to the reaction mixture under ice-cooling, and the mixture was subjected to extraction with ethyl acetate. The organic layer was washed with water, dried over sodium sulfate and filtered. The filtrate was concentrated and the obtained residue was purified by silica gel column chromatography (chloroform:methanol=100:0 to 99:1), and the solvent was distilled off under reduced pressure. The resulting residue was dried in vacuo to yield Compound 6 (291 mg, 0.684 mmol, yield: 51%).
¹H-NMR(CDCl₃)δ:5.13(2H,s),7.23-7.24(2H,m),7.42(1H,d,J=7.3Hz),7.67(1H,t,J=2.1Hz),8.45(1H,d, J=2.3Hz),8.67(1H,d,J=2.3Hz).
LC/MS(ESI):m/z=425, RT=2.17min, LC/MS Measurement Conditions A

### Step 5: Synthesis of Compound (I)

Compound 6 (25.0 mg, 0.059 mmol), 6,6-difluoro-2-azaspiro[3.3]heptane trifluoroacetate (17.4 mg, 0.070 mmol), N,N-diisopropylethylamine (20.5 µL, 0.117 mmol), and DMF (0.5 mL) were mixed and the solution was stirred at 60°C for 2 hours. Water (2 mL) was added to the reaction solution and subjected to extraction with ethyl acetate. The organic layer was washed with water, dried over sodium sulfate, and filtered. The filtrate was concentrated and ethyl acetate (0.05 mL), hexane (0.125 mL) and diisopropylether (0.125 mL) were added. The resulting precipitate was filtered off and washed with diisopropylether. The resulting solid was dried in vacuo to yield Compound (I) (22.0 mg, 0.042 mmol, yield: 72%) .
¹H-NMR(CDCl₃)δ:2.75(4H,t,J=12.0Hz),4.02(4H,s),4.74(2H,s),7.16-7.18(2H,m),7.32-7.35(1H,m),7.65(1H,t,J=2.1Hz),8.43(1H,d,J=2.3Hz),8.61(1H,d,J=2 .3Hz).
LC/MS(ESI):m/z=522, RT=2.27min, LC/MS Measurement Conditions A

### (Example 1A: Biotest of Compound (I))

Biotest Examples for the compounds according to the present invention will be described below.

It is desirable that the compounds represented by Formula (I) according to the present invention are compounds having coronavirus 3CL protease inhibitory activity and inhibiting coronavirus 3CL proteases.

Specifically, in the evaluation method described below, the IC₅₀ is preferably 50 µM or less, more preferably 1 µM or less, and even more preferably 100 nM or less. EC₅₀ is preferably 10 µM or less, more preferably 1 µM or less, and even more preferably 100 nM or less.

Biotest results of the compound represented by Formula (I) are described in WO 2023/195529 and WO 2023/195530 as a compound (I-077).

### Test Example 1: Cytopathic Effect (CPE) Suppression Effect Confirmation Test Using HEK293T Cells Expressing Human TMPRSS2, ACE2 (HEK293T/ACE2-TMPRSS2 Cells)

### <Operational Procedure>

### · Dilution and dispensing of sample to be tested

The sample to be tested is diluted in advance to an appropriate concentration with DMSO, and a 2- to 5-fold series of serial dilutions is prepared and then dispensed into a 384-well plate.

### · Dilution and dispensing of cells and SARS-CoV-2

HEK293T/ACE2-TMPRSS2 cells (GCP-SL222, 5×10³ cells/well) and SARS-CoV-2 (200-600 TCID₅₀/well) are mixed in a medium (MEM, 2% FBS, penicillin-streptomycin), the mixture is dispensed into the wells in which the sample to be tested has been introduced, and then the cells are cultured for 3 days in a CO₂ incubator.

### · Dispensing of CellTiter-Glo (registered trademark) 2.0 and measurement of luminescence signals

The plate that has been cultured for 3 days is returned to room temperature, subsequently CellTiter-Glo (registered trademark) 2.0 is dispensed into each well, and the plate is mixed using a plate mixer. After standing for a predetermined period of time, the luminescence signal (Lum) is measured using a plate reader.

### <Calculation of Each Measurement Item Value>

### · Calculation of 50% SARS-CoV-2 infected cell death inhibitory concentration (EC₅₀)

When x denotes the logarithmic value of the compound concentration and y denotes %Efficacy, the inhibition curve is approximated by the following Logistic regression equation, and the value of x when y = 50 (%) is inputted is calculated as EC₅₀. $y = min + \left(max-min\right) / \left\{1+{\left(X50/x\right)}^{∧}Hill\right\}$ 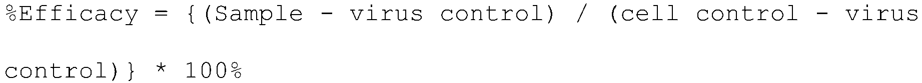 %Efficacy = {(Sample - virus control) / (cell control - virus control)} * 100% cell control: the average of Lum of cell control wells virus control: the average of Lum of virus control wells min: lower limit of y axis, max: upper limit of y axis, X50: x coordinate of inflection point, Hill: Slope of curve at midpoint between min and max

The compound represented by Formula (I) according to the present invention was tested essentially as described above. The EC₅₀ values are shown in the following.

### (Results)

Compound (I): 1.66 nM

### Test Example 2: Inhibitory Activity Rest on SARS-CoV-2 3CL Protease

### <Materials>

· Commercially available Recombinant SARS-CoV-2 3CL Protease
· Commercially available substrate peptide 
Dabcyl-Lys-Thr-Ser-Ala-Val-Leu-Gln-Ser-Gly-Phe-Arg-Lys-Met-

Glu(Edans)-NH2 (SEQ ID NO: 1)
· Internal Standard peptide

Dabcyl-Lys-Thr-Ser-Ala-Val-Leu(¹³C₆,¹⁵N)-Gln (SEQ ID NO: 2) Dabcyl-Lys-Thr-Ser-Ala-Val-Leu(¹³C₆,¹⁵N)-Gln can be synthesized with reference to documents (Atherton, E.; Sheppard, R.C., "In Solid Phase Peptide Synthesis, A Practical Approach", IRL Press at Oxford University Pres, 1989.; Bioorg.Med.Chem., Vol. 5, No. 9, 1997, pp. 1883-1891; and the like). An example will be shown below.

Using Rink amide resin, H-Lys-Thr-Ser-Ala-Val-Leu(¹³C₆, ¹⁵N)-Glu(resin)-OαOtBu was synthesized by Fmoc solid-phase peptide synthesis. The Lys side chain was protected with Boc, the Thr side chain was protected with a tert-butyl group, and the Ser side chain was protected with a tert-butyl group. In addition, the C-terminal OH of Glu was protected with a tert-butyl group, and the peptide was immobilized on the resin by coupling the side-chain carboxylic acid of Glu to the resin. N-terminal Dabcyl group modification was carried out by coupling 4-dimethylaminoazobenzene-4'-carboxylic acid (Dabcyl-OH) on the resin using EDC/HOBT. Final deprotection and cleavage from the resin were carried out by treatment with TFA/EDT (95:5). Thereafter, purification is performed by reverse phase HPLC.

### · RapidFire Cartridge C4 type A

### <Operational Procedure>

### · Preparation of assay buffer

In this test, an assay buffer consisting of 20 mM Tris-HCl, 1 mM EDTA, 10 mM DTT, and 0.01% BSA is used.

### · Dilution and dispensing of sample to be tested

The sample to be tested is diluted in advance to an appropriate concentration with DMSO, and a 2- to 5-fold series of serial dilutions is prepared and then dispensed into a 384-well plate.

### · Addition of enzyme and substrate, and enzymatic reaction

To the prepared compound plate, 8 µM of substrate and 6 nM or 0.6 nM of enzyme solution are added, followed by incubation at room temperature for 3 to 5 hours. Thereafter, a reaction stopping solution (0.067 µM Internal Standard, 0.1% formic acid, and 10 or 25% acetonitrile) is added to stop the enzymatic reaction.

### · Measurement of reaction product

The plate in which the reaction has been completed is measured using RapidFire System 360 and a mass analyzer (Agilent, 6550 iFunnel Q-TOF), or RapidFire System 365 and a mass analyzer (Agilent, 6495C Triple Quadrupole). Solution A (75% isopropanol, 15% acetonitrile, 5 mM ammonium formate) and solution B (0.01% trifluoroacetic acid, 0.09% formic acid) are used as a mobile phase at the measurement.

The reaction product detected by the mass analyzer is calculated using RapidFire Integrator or an equivalent program capable of analysis and is taken as Product area value. Furthermore, the Internal Standard that has been detected at the same time is also calculated and is designated as Internal Standard area value.

### <Calculation of Each Measurement Item Value>

### · Calculation of P/IS

The area values obtained in the previous items are calculated by the following formula, and P/IS is calculated. $P / IS = Product area value / Internal Standard area value$

### · Calculation of 50% SARS-CoV-2 3CL protease inhibitory concentration (IC₅₀)

When x denotes the logarithmic value of the compound concentration and y denotes %Inhibition, the inhibition curve is approximated by the following Logistic regression equation, and the value of x obtainable when y = 50 (%) is inputted is calculated as IC₅₀. $y = min + \left(max-min\right) / \left\{1+{\left(X50/x\right)}^{∧}Hill\right\}$ 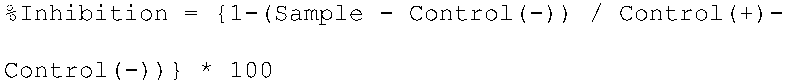 %Inhibition = {1-(Sample - Control(-)) / Control(+)-Control(-))} * 100 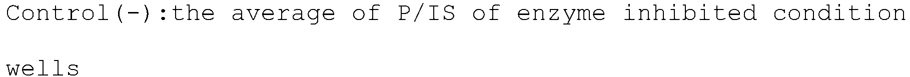 Control(-):the average of P/IS of enzyme inhibited condition wells Control(+):the average of P/IS of DMSO control wells min: lower limit of y axis, max: upper limit of y axis, X50: x coordinate of inflection point, Hill: Slope of curve at midpoint between min and max

The compound represented by Formula (I) according to the present invention was tested essentially as described above. The IC₅₀ values are shown in the following.

### (Results)

Compound (I): 0.00036 µM

Test Example 3: X-Ray Powder Diffraction (XRPD) Experiment X-Ray powder diffraction spectrometry of the solid state (crystal and amorphous form) and the solid dispersion obtained in each Example was performed according to the X-Ray powder diffraction spectrometry method described in the General Testing Methods of the Japanese Pharmacopoeia. Measurement conditions are shown below.

### Measurement Conditions 1:

X-Ray powder diffraction apparatus: SmartLab manufactured by Rigaku Corporation
Measurement method: Reflection method
Wavelength used: CuKα radiation (A = 1.5418Å)
Tube current: 200 mA
Tube voltage: 45 kV
Sample plate: Aluminum
Incident angle of X-rays: 2.5°
Sampling width: 0.02°
Detector: HyPix-3000 (two-dimensional detection mode)

Test Example 4: Measurement of X-Ray Powder Diffraction Pattern Under Temperature and/or Relative Humidity Control Using an attachment equipped with the X-ray diffraction apparatus to control the temperature and relative humidity of the sample measurement section, X-Ray powder diffraction spectrometry was performed in accordance with the X-Ray powder diffraction spectrometry described in the General Tests of the Japanese Pharmacopoeia. Measurement conditions are shown below.

### Measurement Conditions 2:

### (Measurement of X-Ray Diffraction)

X-Ray powder diffraction apparatus: RINT 2100 Ultima+ manufactured by Rigaku Corporation
Measurement method: Reflection method
Wavelength used: CuKα radiation (A = 1.5418Å)
Tube current: 40 mA
Tube voltage: 40 kV
Sample plate: Aluminum
X-ray measurement range: 5° to 35°
Sampling width: 0.02°
Scan speed: 60°/min

### (Control of Temperature and Relative Humidity)

Temperature controller: Rigaku ThermoPlus
Humidity controller: Rigaku HUM-1

### Test Example 5: Measurement and Analysis Method for Single Crystal Structure Analysis

The crystals obtained in the Examples were subjected to single crystal structure analysis. The measurement conditions and analysis method will be described below.

### (Apparatus)

XtaLAB P200 MM007 manufactured by Rigaku Corporation

### (Measurement Conditions)

Measurement temperature: 25°C
Temperature controller: Sample spraying low temperature apparatus manufactured by Rigaku Corporation
Wavelength used: CuKα radiation (λ = 1.5418Å)
Software: CrysAlisPro 1.171.39.46e (Rigaku Oxford Diffraction, 2018)

### (Data Processing)

Software: CrysAlisPro 1.171.39.46e (Rigaku Oxford Diffraction, 2018)
The data were subjected to Lorentz and polarization correction and absorption correction.

### (Crystal Structure Analysis)

Phase determination was performed using a direct method program, ShelXT (Sheldrick, G.M., 2015), and regarding refinement, a full-matrix least squares method was carried out using ShelXL (Sheldrick, G.M., 2015). The temperature factors of non-hydrogen atoms were all subjected to refinement with anisotropy. Hydrogen atoms were computationally introduced using the default parameters of ShelXL and were treated as riding atoms. The hydrogen atoms were subjected to refinement with isotropic parameters.

PLATON (Spek, 1991)/ORTEP (Johnson, 1976) was used to draw the following structural drawing (30% PROBABILITY level).

### Test Example 6: Measurement of Raman Spectrum

Measurement of the Raman spectrum of crystals obtained in Examples was performed.

### Measurement Conditions 1

Measurement method: Microscopic laser Raman spectrometry Laser wavelength: 671 nm
Cumulative number: Once
Exposure time: 1 second

### Test Example 7: Differential Scanning Calorimetry (DSC)

The DSC measurement of the crystals obtained in the Examples was performed. The sample was weighed into an aluminum pan and measured by simple sealing. The measurement conditions are shown below. In the measurement using the differential scanning calorimetry (DSC), an error may occur in the range of ±2°C.
Apparatus: Discovery DSC/TA Instrument
Measured temperature range: -10°C to 270°C
Rate of temperature increase: 10 °C/min
Atmosphere: N₂ 50 mL/min

### Test Example 8: Simultaneous Thermogravimetric-Differential Thermal Analysis (TG/DTA)

The solid states (crystal and amorphous forms) and the solid dispersion obtained in each Example were subjected to simultaneous thermogravimetric-differential thermal analysis (TG/DTA). Samples obtained in each Example were weighed and packed into an aluminum pan, and measurement was performed in an open system. Measurement conditions are as follows.
Apparatus: Hitachi High-Technologies TG/DTA STA7200RV
Measured temperature range: Room temperature to 350°C
Rate of temperature increase: 10 °C/min

### Test Example 9: Dynamic Vapor Sorption (DVS)

The crystals obtained in the Examples were subjected to dynamic vapor sorption (DVS). The crystals obtained in the Examples were weighed into aluminum pans and allowed to stand at 25°C and 0% relative humidity. After the compound was sufficiently dried and its weight had stabilized, the measurement was initiated, and the weight was recorded as the relative humidity was increased from 0% to 95% in increments of 5%. The weight was then recorded when the relative humidity varied in 5% increments from 95% to 0%.
Apparatus: DVS Adventure manufactured by Surface Measurement Systems

### Test Example 10: Particle Size Distribution/Dry Method

The particle size distributions of the crystals and solid dispersions obtained in the Examples were measured. The particle size distributions were measured by a dry method using a laser diffraction particle size distribution measuring apparatus.

### Measurement Conditions 1

Apparatus: HELOS & RODOS (manufactured by Sympatec Corporation)
Range: R1
Dispersion pressure: 2 bar
Trigger condition: 2-second stop measured concentration ≤ 0.5%, or a 10-second real-time measurement

### Measurement Conditions 2

Apparatus: HELOS & RODOS (manufactured by Sympatec Corporation)
Range: R3
Dispersion pressure: 2 bar
Trigger condition: 2-second stop measured concentration ≤ 0.5%, or a 10-second real-time measurement

### Measurement Conditions 3

Apparatus: MT 3200II type microtrucks (manufactured by MicrotracBEL)

### Test Example 11: Particle Size Distribution/Wet Method

The particle size distributions of the crystals obtained in the Examples were measured. The particle size distributions were measured by a wet method using a laser diffraction/scattering particle size distribution analyzer, Microtrac MT3200II (manufactured by MicrotracBEL).
Measurement range: 0.243 to 1408 µm
Solvent: Water
Solvent refractive index: 1.333
Measurement time: 30 seconds
Particle shape: Non-spherical
Particle permeability: Transparent
Particle refractive index: 1.81

### (Example 1B: Analysis of Solid State of Compound (I))

For Compound (I) produced by the synthesis method of Example 1 described above, X-Ray powder diffraction experiment and simultaneous thermogravimetric-differential thermal analysis (TG/DTA) were performed, thereby confirming that the compound represented by Formula (I) was an anhydrous crystal.

### (Example 2: Analysis of Anhydrous Crystal of Compound Represented by Formula (I))

The anhydrous crystal of the compound represented by Formula (I) was pulverized and then subjected to X-Ray powder diffraction experiment, single crystal structure analysis, differential scanning calorimetry (DSC), simultaneous thermogravimetric-differential thermal analysis (TG/DTA), dynamic vapor sorption (DVS), Raman spectroscopy, and particle size distribution measurement.

### (Example 2A: Milling of Anhydrous Crystal of Compound Represented by Formula (I))

The anhydrous crystal of the compound represented by Formula (I) was sieved through a 1000 µM mesh and then pulverized under the following conditions.
Apparatus: A-O jet mill (Seishin Kigyo Co., Ltd.)
Feeding method: Feeder
Feed rate: 20 g/hour
Pulverizing pressure: 0.30 MPa
Supply pressure: 0.40 MPa

### (Example 2B: X-Ray Powder Diffraction Experiment of Anhydrous Crystal of Compound Represented by Formula (I))

An X-Ray powder diffraction experiment was performed on the anhydrous crystal of the compound represented by Formula (I) after milling under measurement conditions 1 described in Test Example 3.

The X-Ray powder diffraction pattern is shown in Fig. 1, and the peak list of the X-Ray powder diffraction pattern is shown in Fig. 2. Hereinafter, in the table of the peak list of the X-Ray powder diffraction pattern, "Position" indicates 2θ (°), and "Intensity" indicates intensity.

The X-Ray powder diffraction pattern has peaks at diffraction angles (2θ) of 6.5°±0.2°, 10.1°±0.2°, 13.0°±0.2°, 14.1°±0.2°, 15.3±0.2°, 15.6°±0.2°, 16.2°±0.2°, 17.4°±0.2°, 18.9°±0.2°, 19.9°±0.2°, 20.3°±0.2°, 21.7°±0.2°, 23.0°±0.2°, 23.8°±0.2°, 25.8°±0.2°, 28.8°±0.2°, and 30.6°±0.2°.

For the anhydrous crystal of the compound represented by Formula (I), the X-Ray powder diffraction pattern has characteristic peaks at diffraction angles (2θ) of 6.5°±0.2°, 10.1°±0.2°, 15.6°±0.2°, 16.2°±0.2°, 17.4°±0.2°, 19.9°±0.2°, 20.3°±0.2°, 21.7°±0.2°, 23.0°±0.2°, and 23.8°±0.2°.

For the anhydrous crystal of the compound represented by Formula (I), the X-Ray powder diffraction pattern has characteristic peaks at diffraction angles (2θ) of 6.5°±0.2°, 15.6°±0.2°, 17.4°±0.2°, 19.9°±0.2°, and 20.3°±0.2°.

### (Example 2C: Single Crystal Structure Analysis of Anhydrous Crystal of Compound Represented by Formula (I))

### <Method of Preparing Single Crystal>

To 1 mg of the crystal of the compound represented by Formula (I), 400 µL of methanol was added, and the mixture was heated to 50°C to dissolve the crystal. The solution was dispensed into a 1.5 mL HPLC vial, the HPLC vial was capped, a syringe needle was inserted through the cap, and the HPLC vial was allowed to stand at room temperature. Single crystals were prepared by solvent evaporation.

### <Single Crystal Structure Analysis>

The single-crystal diffraction experiment and analysis were performed according to the method described in Test Example 5 above. It should be noted that Cl1 and Cl7C, and H5CA and H6CA are in a disorder relationship; therefore, the structure was analyzed with occupancies of Cl1:Cl7C = 0.75:0.25 and H5CA:H6CA = 0.25:0.75.

The results of the single crystal structure analysis are shown below. The R₁ (I>2.00s(I)) was 0.0555, and it was confirmed from the final difference Fourier map that no missing or misplaced electron density was present.

Crystallographic data are shown in Table 1.

**[Table 1]**

| Space Group | Pbca |
|---|---|
| a (Å) | 14.6653 (7) |
| b (Å) | 11.8338 (5) |
| c (Å) | 27.1024 (10) |
| 0 (°) | 90 |
| β (°) | 90 |
| γ (°) | 90 |
| V (Å³) | 4703.5 (3) |
| Z | 8 |
| Density (Measured Value) (g/cm³) | 1.475 |
| Measured Temperature(K) | 298 |

Here, V indicates the unit lattice volume, Z indicates chemical unit number per unit cell.

The fractional atomic coordinates x, y, and z (Å×10⁴) and the equivalent isotropic displacement parameters U(eq) (Equivalent Isotropic Displacement Parameters, Å²×10³) for non-hydrogen atoms are shown in Table 2. Here, U(eq) is defined as one third of the trace of the orthogonalized Uᵢⱼ tensor.

**[Table 2]**

| **Atom** | **x** | **y** | **z** | **U(eq)** |
|---|---|---|---|---|
| Cl0A | 1162.1(10) | 9350.8(10) | 9005.2(4) | 134.2(5) |
| Cl1 | 754.6(13) | 6240.2(15) | 5416.9(8) | 158.6(8) |
| O0AA | 4165.4(12) | 8688.3(14) | 7965.5(7) | 62.4(5) |
| N4AA | 3011.5(14) | 8849.3(16) | 7408.6(8) | 55.0(5) |
| N1AA | 4332.8(13) | 7814.7(16) | 7227.4(8) | 53.0(5) |
| N2AA | 4551.4(15) | 6837.4(17) | 6491.3(8) | 59.9(5) |
| O1AA | 1886.9(15) | 9038(2) | 6844.4(8) | 97.5(8) |
| N0AA | 2003.5(18) | 11399.8(18) | 7952.6(10) | 76.3(7) |
| C1AA | 2516.9(17) | 9553.6(18) | 7746.5(9) | 52.9(6) |
| C2AA | 3850.1(17) | 8463.9(19) | 7561.7(10) | 53.1(6) |
| C3AA | 3996.7(17) | 7532.2(19) | 6757.1(9) | 54.5(6) |
| F4AA | 6218(2) | 3242(2) | 5837.2(11) | 158.4(11) |
| C5AA | 4960(2) | 5752(2) | 6663.9(10) | 62.6(7) |
| C6AA | 3179.9(18) | 7974(2) | 6609.7(9) | 61.2(7) |
| C7AA | 4998.9(18) | 5350(2) | 6131.3(10) | 61.4(7) |
| C8AA | 2142.6(19) | 9118(2) | 8169.5(10) | 63.0(7) |
| F9AA | 1731(3) | 7503(2) | 4713.3(10) | 186.6(16) |
| C0BA | 2430(2) | 10692(2) | 7651.1(11) | 66.1(7) |
| C1BA | 2644(2) | 8653(2) | 6937.4(10) | 65.7(7) |
| C2BA | 5281.1(18) | 7639(2) | 7371.6(12) | 69.6(8) |
| C3BA | 2787(2) | 7819(2) | 6105(1) | 70.5(8) |
| C4BA | 1677(2) | 9846(2) | 8473.2(11) | 70.1(7) |
| C5BA | 1632(2) | 10977(2) | 8357.7(12) | 73.9(8) |
| F6BA | 5565(3) | 3960(3) | 5212.6(9) | 180.9(14) |
| C7BA | 5403(2) | 6691(3) | 7713.5(13) | 79.8(9) |
| N3AA | 5508(2) | 5941(3) | 7971.4(13) | 114.7(12) |
| CBBA | 4770(2) | 4133(3) | 5977.5(13) | 83.8(9) |
| C9BA | 2054(3) | 7103(3) | 6022.4(13) | 89(1) |
| C0CA | 4369(3) | 6326(3) | 6001.4(11) | 89.6(11) |
| C1CA | 5647(3) | 4080(3) | 5706.8(12) | 92.1(11) |
| C2CA | 5924(2) | 5216(3) | 5873.1(14) | 94.9(11) |
| C3CA | 3122(3) | 8436(3) | 5721.9(12) | 94.8(11) |
| C4CA | 2070(4) | 7603(4) | 5176.6(16) | 120.8(18) |
| C5CA | 1702(3) | 7000(4) | 5550.8(18) | 113.8(15) |
| C6CA | 2768(4) | 8325(4) | 5246.0(14) | 121.0(17) |
| Cl7C | 2963(4) | 9116(5) | 4816.4(15) | 140.4(18) |

Next, the atomic coordinates x, y, and z (Å×10⁴) and the isotropic displacement parameters U(eq) (Isotropic Displacement Parameters, Å²×10³) for hydrogen atoms are shown in Table 3.

**[Table 3]**

| Atom | x | y | z | U(eq) |
|---|---|---|---|---|
| H5AA | 4557.64 | 5307.5 | 6871.27 | 75 |
| H5AB | 5554.99 | 5838.46 | 6816.05 | 75 |
| H8AA | 2201.83 | 8356.07 | 8247.86 | 76 |
| H0BA | 2680.93 | 10977.51 | 7361.78 | 79 |
| H2BA | 5508.61 | 8323.16 | 7525.87 | 84 |
| H2BB | 5642.53 | 7502.71 | 7077.87 | 84 |
| H5BA | 1329.65 | 11462.88 | 8572.27 | 89 |
| H8BA | 4731.05 | 3609.27 | 6252.06 | 101 |
| H8BB | 4237.54 | 4073.65 | 5766.58 | 101 |
| H9BA | 1801.24 | 6695.23 | 6281.91 | 107 |
| H0CA | 4582.75 | 6779.79 | 5726.84 | 108 |
| H0CB | 3738.83 | 6101.51 | 5953.94 | 108 |
| H2CA | 6021.1 | 5746.94 | 5605.7 | 114 |
| H2CB | 6439.24 | 5211.91 | 6097.8 | 114 |
| H3CA | 3596.24 | 8942.43 | 5778.24 | 114 |
| H5CA | 1213.16 | 6517.45 | 5491.34 | 137 |
| H6CA | 3008.16 | 8737.79 | 4984.33 | 145 |

The structure in the asymmetric unit of the crystal structure is shown in Fig. 3.

It should be noted that the label numbers of non-hydrogen atoms shown in Fig. 3 correspond to the numbers of non-hydrogen atoms in Table 2.

Since only one molecule of the compound represented by Formula (I) was present in the asymmetric unit of the crystal structure, the crystal was identified as an anhydrous crystal of the compound represented by Formula (I).

It was confirmed that the X-Ray powder diffraction pattern (λ=1.5418 Å) calculated from the crystal structure using Mercury (The Cambridge Crystallographic Data Centre, Ver. 4.0.0) generally corresponds to the X-Ray powder diffraction pattern of Example 2B (Fig. 1).

### (Example 2D: Differential Scanning Calorimetry of Anhydrous Crystal of Compound Represented by Formula (I))

About 2 mg of the pulverized anhydrous crystal of the compound represented by Formula (I) was weighed into an aluminum pan, and measurement was performed according to the method described in Test Example 7 above. The results are shown in Fig. 4. An endothermic peak with an onset temperature of about 261.3°C was observed.

### (Example 2E: Simultaneous Thermogravimetric-Differential Thermal Analysis of Anhydrous Crystal of Compound Represented by Formula (I))

For the pulverized anhydrous crystal of the compound represented by Formula (I), measurement was performed according to the method described in Test Example 8 above.

The results are shown in Fig. 5. An endothermic peak with an onset temperature of about 265.6°C was observed. No weight loss was observed.

### (Example 2F: Measurement of Raman Spectrum of Anhydrous Crystal of Compound Represented by Formula (I))

For the pulverized anhydrous crystal of the compound represented by Formula (I), Raman spectroscopy was performed under measurement conditions 1 described in Test Example 6 above. The results are shown in Fig. 6. The main Raman peaks are shown below.

**[Table 4]**

| Roman Peak (cm⁻¹) | Raman Peak (cm⁻¹) | Raman Peak (cm⁻¹) | Raman Peak (cm⁻¹) |
|---|---|---|---|
| 415.2 | 729.1 | 1100.1 | 1400.8 |
| 439.0 | 742.7 | 1109.7 | 1431.4 |
| 466.2 | 768.7 | 1133.3 | 1468.0 |
| 502.7 | 801.4 | 1145.0 | 1502.3 |
| 526.2 | 827.2 | 1162.0 | 1572.4 |
| 543.7 | 864.0 | 1194.9 | 1595.2 |
| 565.8 | 892.9 | 1213.9 | 1641.0 |
| 582.1 | 902.9 | 1227.6 | 1714.8 |
| 589.1 | 922.7 | 1241.2 | 2249.0 |
| 615.7 | 945.9 | 1275.8 | 2952.9 |
| 654.9 | 1024.5 | 1287.2 | 3065.4 |
| 681.2 | 1041.9 | 1307.0 | |
| 696.1 | 1059.2 | 1332.9 | |

For the anhydrous crystal of the compound represented by Formula (I), the Raman spectrum showed characteristic peaks at 415.2 cm⁻¹±2 cm⁻¹, 502.7 cm⁻¹±2 cm⁻¹, 1431.4 cm⁻¹± 2 cm⁻¹, 1714.8 cm⁻¹±2 cm⁻¹, and 3065.4 cm⁻¹ ± 2 cm⁻¹.

### (Example 2G: Particle Size Distribution of Anhydrous Crystal of Compound Represented by Formula (I))

For the anhydrous crystal of the compound represented by Formula (I) before milling, which was used in Example 2A, the particle size distribution was measured according to the method described in measurement conditions 2 of Test Example 10. As a result, D10, D50, and D90 were 1.16 µm, 4.42 µm, and 13.13 µm, respectively. The particle size distribution is shown in Fig. 7.

### (Example 2H: Particle Size Distribution of Anhydrous Crystal of Compound Represented by Formula (I))

For the anhydrous crystal of the compound represented by Formula (I) after milling, obtained in Example 2A, the particle size distribution was measured in the method described in measurement conditions 1 of Test Example 10.

As a result, D10, D50, and D90 were 0.79 µm, 2.90 µm, and 7.32 µm, respectively. The particle size distribution is shown in Fig. 8.

### (Example 3: Preparation and Analysis of Ethyl Acetate Solvate Crystal of Compound Represented by Formula (I))

Ethyl acetate solvate crystal of the compound represented by Formula (I) were prepared, and each crystal was subjected to NMR measurement, X-Ray powder diffraction experiments, single crystal structure analysis, differential scanning calorimetry (DSC), simultaneous thermogravimetric-differential thermal analysis (TG/DTA), dynamic vapor sorption (DVS), Raman spectroscopy, and particle size distribution measurement.

### (Example 3A: Preparation of Ethyl Acetate Solvate Crystal of Compound Represented by Formula (I))

Compound 6 described in Example 1 (800 mg, 1.880 mmol) and 6,6-difluoro-2-azaspiro[3.3]heptane trifluoroacetate (557 mg, 2.256 mmol) were dissolved in DMF (8.0 mL), and N,N-diisopropylethylamine (985 µL, 5.64 mmol) was added. The mixture was stirred at 60°C for 2 hours. Water was added to the reaction mixture, followed by extraction with ethyl acetate. The organic layer was washed with water and then with brine, dried over magnesium sulfate, and filtered. The filtrate was concentrated under reduced pressure to remove the solvent, and the residue was subjected to silica gel column chromatography. Dissolution was carried out with hexane/ethyl acetate, and fractions containing the desired compound were collected. After concentration under reduced pressure, diisopropyl ether was added, and the precipitated solid was collected by filtration. The obtained residue was dried under reduced pressure to give Compound (I) (954 mg, 1.563 mmol, yield: 83%).

NMR measurement was performed according to the method described above (the method for identifying compound). Peaks attributable to ethyl acetate were observed in the NMR chart.
¹H-NMR (DMSO-D₆)δ:1.18(3H,t , J=7.0Hz),1.99(3H,s),2.79(4H,t,J=12.4Hz),4.03-4.06(6H,m),4.82(2H,s),7.21(1H,s),7.39-7.42(2H,m),7.96(1H,s),8.46(1H,s),8.69(1H,s).
LC/MS(ESI) :m/z=522, RT=2.30min, LC/MS Measurement conditions B

### (Example 3B: X-Ray Powder Diffraction Experiment of Solid Prepared by Method of Example 3A)

For the sample prepared by the method of Example 3A, a X-Ray powder diffraction experiment was performed under measurement conditions 1 described in Test Example 3 above. As a result, it was confirmed that the sample prepared by the method of Example 3A was a crystal different from the anhydrous crystal of the compound represented by Formula (I).

Based on the NMR measurement of Example 3A and the results of Example 3B, the sample prepared by the method of Example 3A was presumed to be an "ethyl acetate solvate crystal of the compound represented by Formula (I)".

### (Example 3C: Single Crystal Structure Analysis of Ethyl Acetate Solvate Crystal of Compound Represented by Formula (I))

For the ethyl acetate solvate crystals of the compound represented by Formula (I), single-crystal diffraction experiment and analysis were performed according to the method described in Test Example 5 above. As a result, it was confirmed that the crystal was an ethyl acetate solvate crystals having a molar ratio of 1:1 between the compound represented by Formula (I) and ethyl acetate. It should be noted that a portion of the compound represented by Formula (I) exhibited a disordered structure.

### (Example 3D: NMR Measurement of Ethyl Acetate Solvate Crystal of Compound Represented by Formula (I))

For the ethyl acetate solvate crystal of the compound represented by Formula (I) that had been air-dried overnight at room temperature, NMR measurement was performed according to the method described above (the method for identifying compound). The ¹H-NMR results are shown in Fig. 9.

In the NMR chart, peaks attributable to ethyl acetate were observed. From the integration ratio of the ¹H-NMR spectrum, the molar ratio of the compound represented by Formula (I) to ethyl acetate was about 1:1. Since the crystal was air-dried at room temperature prior to measurement, it was presumed that ethyl acetate was not merely adhering to the crystal surface, but was incorporated into the crystal lattice, and therefore the crystals were identified as ethyl acetate solvate crystals. This result was consistent with the single crystal structure analysis of Example 3C.

### (Example 3E: Milling of Ethyl Acetate Solvate Crystal of Compound Represented by Formula (I))

The ethyl acetate solvate crystal of the compound represented by Formula (I) was pulverized under the following conditions. Apparatus: Hosokawa/ALPINE SPIRAL JET MILL50AS (Hosokawa micron Co., Ltd.)
Feeding method: Manual
Feed rate: 30.15 g/25 min
Pulverizing pressure: 0.10 MPa
Supply pressure: 0.20 MPa

### (Example 3F: X-Ray Powder Diffraction Experiment of Ethyl Acetate Solvate Crystal of Compound Represented by Formula (I))

An X-Ray powder diffraction experiment was performed on the ethyl acetate solvate crystal of the compound represented by Formula (I) after milling under measurement conditions 1 described in Test Example 3. The obtained X-Ray powder diffraction pattern is shown in Fig. 10, and the peak list of the X-Ray powder diffraction pattern is shown in Fig. 11.

The X-Ray powder diffraction pattern has peaks at diffraction angles (2θ) of 6.9°±0.2°, 8.8°±0.2°, 11.2°±0.2°, 13.1°±0.2°, 13.6°±0.2°, 13.9°±0.2°, 16.3°±0.2°, 17.6°±0.2°, 18.6°±0.2°, 19.0°±0.2°, 19.8°±0.2°, 20.1°±0.2°, 20.6°±0.2°, 20.9°±0.2°, 21.2°±0.2°, 21.7°±0.2°, 22.1°±0.2°, 22.6°±0.2°, 22.9°±0.2°, 23.7°±0.2°, 24.5°±0.2°, 25.1°±0.2°, 25.4°±0.2°, 25.7°±0.2°, 26.7°±0.2°; 27.0°±0.2°, 27.4°±0.2°, 28.0°±0.2°, 28.7°±0.2°, and 29.7°±0.2°.

For the ethyl acetate solvate crystal of the compound represented by Formula (I), the X-Ray powder diffraction pattern has characteristic peaks at diffraction angles (2θ) of 6.9°±0.2°, 8.8°±0.2°, 13.1°±0.2°, 13.6°±0.2°, 16.3°±0.2°, 17.6°±0.2°, 18.6±0.2°, 20.9±0.2°, 21.7±0.2°, and 23.7°±0.2°. For the ethyl acetate solvate crystal of the compound represented by Formula (I), the X-Ray powder diffraction pattern has characteristic peaks at diffraction angles (2θ) of 6.9°±0.2°, 8.8°±0.2°, 13.1°±0.2°, 16.3°±0.2°, and 23.7°±0.2°.

### (Example 3G: Raman Spectrum Measurement of Ethyl Acetate Solvate Crystal of Compound Represented by Formula (I))

For the pulverized ethyl acetate solvate crystal of the compound represented by Formula (I), Raman spectroscopy was performed under measurement conditions 1 described in Test Example 6 above. The results are shown in Fig. 12. The main Raman peaks are shown below.

**[Table 5]**

| Raman Peak (cm⁻¹) | Ramen Peak (cm⁻¹) | Raman Peak (cm⁻¹) | Raman Peak (cm⁻¹) |
|---|---|---|---|
| 406.9 | 726.8 | 1100.1 | 1469.0 |
| 421.2 | 747.3 | 1106.5 | 1503.3 |
| 436.6 | 768.7 | 1125.8 | 1570.4 |
| 465.0 | 805.9 | 1141.8 | 1585.3 |
| 509.7 | 833.9 | 1171.6 | 1593.2 |
| 541.4 | 846.2 | 1185.3 | 1644.9 |
| 563.5 | 881.8 | 1221.2 | 1709.9 |
| 593.7 | 900.6 | 1246.5 | 1737.2 |
| 615.7 | 928.3 | 1268.5 | 2258.0 |
| 636.5 | 957.9 | 1287.2 | 2956.1 |
| 652.6 | 1026.7 | 1309.1 | 3052.9 |
| 684.7 | 1048.4 | 1321.5 | |
| 689.2 | 1059.2 | 1436.6 | |

For the ethyl acetate solvate crystal of the compound represented by Formula (I), the Raman spectrum has characteristic peaks at 421.2 cm⁻¹±2 cm⁻¹, 509.7 cm⁻¹±2 cm⁻¹, 1585.3 cm⁻¹±2 cm⁻¹, 1709.9 cm⁻¹±2 cm⁻¹, and 3052.9 cm⁻¹±2 cm⁻¹.

### (Example 3H: Simultaneous Thermogravimetric-Differential Thermal Analysis of Ethyl Acetate Solvate Crystal of Compound Represented by Formula (I))

For the ethyl acetate solvate crystals of the compound represented by Formula (I), measurement was performed according to the method described in Test Example 8 above.

The results are shown in Fig. 13. An endothermic peak with an onset temperature of about 129.5°C was observed, and a weight loss of about 14% was confirmed almost simultaneously. This change is considered to be due to desolvation (release) of "ethyl acetate from the ethyl acetate solvate crystal of the compound represented by Formula (I)".

The "ethyl acetate solvate crystal of the compound represented by Formula (I)" having a molar ratio of 1:1 between the compound represented by Formula (I) and ethyl acetate, has a theoretical ethyl acetate content of 14.4 wt%, which was generally consistent with the result of the present thermogravimetric (TG) measurement.

For the ethyl acetate solvate crystal of the compound represented by Formula (I), multiple lots were measured according to the method described in Test Example 8 above. In each measurement, endothermic peaks at 92.5°C, 95.7°C, and 116.7°C, respectively, were observed, and weight loss was confirmed at the temperatures of the endothermic peaks.

The X-Ray powder diffraction experiment was also performed for the sample under measurement conditions 1 described in Test Example 3 above, and the obtained X-Ray powder diffraction pattern was very similar. The main peaks coincided within a range of ±0.2°, although slight differences in relative intensity were observed.

For each sample, the X-Ray powder diffraction pattern has characteristic peaks at diffraction angles (2θ) of 6.9°±0.2°, 8.8°±0.2°, 13.1°±0.2°, 16.3°±0.2°, and 23.7°±0.2°.

### (Example 3I: Particle Size Distribution of Ethyl Acetate Solvate Crystal of Compound Represented by Formula (I))

For the ethyl acetate solvate crystal of the compound represented by Formula (I) before milling, which was used in Example 3E, the particle size distribution was measured according to the method described in measurement conditions 2 of Test Example 10. As a result, D10, D50, and D90 were 18.73 µm, 71.82 µm, and 114.67 µm, respectively. The particle size distribution is shown in Fig. 14.

### (Example 3J: Particle Size Distribution of Ethyl Acetate Solvate Crystal of Compound Represented by Formula (I))

For the ethyl acetate solvate crystal of the compound represented by Formula (I) after milling, obtained in Example 3E, the particle size distribution has been measured in the manner described in measurement conditions 1 of Test Example 10.

As a result, D10, D50, and D90 were 0.68 µm, 3.59 µm, and 8.80 µm, respectively. The particle size distribution is shown in Fig. 15.

Examples, Reference Examples, and Test Examples relating to preparation of the compound represented by Formula (I) are described below in order to explain the present invention in detail. The present invention is not limited thereto. As the anhydrous crystal of the compound represented by Formula (I), the following compounds were used: (1) a compound having D10=0.78 µm, D50=2.09 µm, and D90=4.77 µm; (2) a compound having particle sizes of D10=0.86 µm, D50=3.29 µm, and D90=10.15 µm; and (3) a compound having particle sizes of D10=0.7 µm, D50=2.7 µm, and D90=8.8 µm, wherein the purities (determination values) of compounds (2) and (3) were 101.7% and 99.7%, respectively.

A chromatogram obtained when the compound having particle sizes of D10 = 0.86 µm, D50 = 3.29 µm, and D90 = 10.15 µm of (2) was measured by the liquid chromatography method described in Test Example 12 is shown in Fig. 16. The compound represented by Formula (I) was detected at a retention time of 24.941 minutes with a purity (pa%) of 99.58%.

### Test Example 12: Analysis 1 of Related Substances

The amount of the related substances was measured by liquid chromatography using the following method and conditions. Detector: Ultraviolet absorptiometer (measurement wavelength: 247 nm)
Column: ACQUITY UPLC BEH C18 (1.7 µm, 2.1 × 100 mm, Waters)
Column temperature: constant temperature around 40°C
Mobile phase A: water/formic acid mixture (1000:1)
Mobile phase B: an acetonitrile/formic acid mixed solution for liquid chromatography (2000:1)
Mobile phase feeding: The mixing ratio of mobile phase A and mobile phase B was varied as follows to control a concentration gradient.

**[Table 6]**

| Time After Injection (Min) | Mobile Phase A (vol%) | Mobile Phase B (vol%) |
|---|---|---|
| 0~10 | 85→65 | 15→35 |
| 10~35 | 65 | 35 |
| 35~40 | 65→20 | 35→80 |
| 40~43 | 20 | 80 |
| 43~43. 1 | 20→85 | 80→15 |
| 43.1~55(Equilibration)* | 85 | 15 |

| | | |
|---|---|---|
| * Equilibration time may be adjusted as appropriate. | | |

Flow rate: 0.4 mL/min
Injection volume: 5 µL
Sample cooler temperature: constant temperature around 10°C Needle wash solvent or autoinjector wash liquid: acetonitrile
Area measurement range: Up to 43 min after sample solution injection

### (Example 4: Production and Analysis of Solid Dispersion of Compound Represented by Formula (I))

A solid dispersion of the compound represented by Formula (I) was prepared and for each solid dispersion observed by digital microscope, X-Ray powder diffraction experiments, differential scanning calorimetry (DSC), measurement of particle size distribution, measurement of related substances were carried out.

### (Example 4A: Selection of Polymer to Be Used for Solid Dispersion of Compound Represented by Formula (I))

After dissolving an anhydrous crystal of the compound represented by Formula (I) and a polymer in acetone, ethanol, or a mixed solvent thereof, the solution was dropped onto a glass slide. A solid dispersion was obtained by solvent evaporation.

The anhydrous crystal of the compound represented by Formula (I) was pulverized under the following conditions, and the pulverized product was used.
Apparatus: Hosokawa/ALPINE SPIRAL JET MILL50AS (Hosokawa micron Co., Ltd.)
Feeding method: Feeder
Feed rate: 60 g/hour
Pulverizing pressure: 0.05 MPa
Supply pressure: 0.10 MPa

The particle size distribution of the pulverized anhydrous crystal of the compound represented by Formula (I) was measured according to the method described in measurement conditions 1 of Test Example 10. As a result, D10, D50, and D90 were 0.78 µm, 2.09 µm, and 4.77 µm, respectively.

### <Preparation of Solid Dispersion>

The content of the compound represented by Formula (I) in the solid dispersion was examined at 10 wt%, 25 wt%, and 50 wt%. The polymers used were copovidone (polyvinyl pyrrolidone-vinyl acetate copolymer, PVPVA) (manufactured by BASF), polyvinyl pyrrolidone (povidone) (manufactured by BASF), hypromellose acetate succinate (hydroxypropyl methylcellulose acetate succinate) (manufactured by Shin-Etsu Chemical Co., Ltd., grades LF and MF), hypromellose phthalate (manufactured by Shin-Etsu Chemical Co., Ltd.), hydroxypropyl cellulose (manufactured by NIPPON SODA CO., LTD.), and methacrylic acid copolymer L (manufactured by Evonik Japan Co., Ltd.). An exemplary formulation is shown below.

**[Table 7]**

| Polymer | Solvent |
|---|---|
| Copovidone | Acetone |
| Povidone | Acetone /Ethanol = 8/2 |
| Hypromellose Acetate Succinate (Grade MF) | Acetone /Ethanol= 20/1 |
| Hypromellose Acetate Succinate (Grade LF) | Acetone /Ethanol= 20/1 |
| Hypromellose Phthalate | Acetone /Ethanol= 8/2 |
| Hydroxypropyl Cellulose | Acetone /Ethanol= 8/2 |
| Methacrylic Acid Copolymer L | Acetone /Ethanol= 1/1 |

### <Evaluation of Presence or Absence of Crystal Precipitation>

Immediately after dropping onto the glass slide, after storage for 1 week at 40°C and 75% relative humidity, and after storage for 1 week at 60°C, the presence or absence of crystal precipitation was observed using a digital microscope (VHX-7000, KEYENCE) in polarized observation mode.

As a result, the solid dispersion using polyvinylpyrrolidone showed no crystal precipitation at contents of the compound represented by Formula (I) of 10 wt%, 25 wt%, and 50 wt%, both immediately after preparation and after storage under each condition, indicating favorable results. The solid dispersion using copovidone showed no crystal precipitation at contents of the compound represented by Formula (I) of 10 wt% and 25 wt%, both immediately after preparation and after storage, indicating favorable results.

The solid dispersions using hypromellose acetate succinate (grades LF and MF) showed no precipitation of coarse crystals of 100 µm or larger at contents of the compound represented by Formula (I) of 10 wt% and 25 wt%, both immediately after preparation and after storage, indicating favorable results.

### (Example 4B: Preparation 1 of Solid Dispersion of Compound Represented by Formula (I))

The anhydrous crystal of the compound represented by Formula (I) (pulverized product of Example 4A) and each polymer were dissolved in acetone, and after confirming complete dissolution, solid dispersions were produced using a spray dryer (organic solvent spray dryer, ADVANCED B-290, manufactured by BUCHI) under the conditions of an inlet temperature of 90°C, a feed pump setting of 20%, and a nitrogen flow rate setting of 40.

As the polymer, copovidone (manufactured by BASF), povidone (manufactured by BASF), and hypromellose acetate succinate (manufactured by Shin-Etsu Chemical Co., Ltd., grades MF and LF) were used. The content of the compound represented by Formula (I) in the solid dispersion was set to 25 wt%. An exemplary formulation is shown below.

**[Table 8]**

| Component (g) | Example 4B-1 | Example 4B-2 | Example 4B-3 | Example 4B-4 |
|---|---|---|---|---|
| Anhydrous Crystal of Compound Represented by Formula (I) | 1 | 1 | 1 | 1 |
| Copovidone | 3 | - | - | - |
| Hypromellose Acetate Succinate (Grade MF) | - | 3 | - | - |
| Povidone | - | - | 3 | - |
| Hypromellose Acetate Succinate (Grade LF) | | | - | 3 |
| Total (g) | 4 | 4 | 4 | 4 |

### (Example 4C: X-Ray Powder Diffraction Experiment of Solid Dispersion of Compound Represented by Formula (I))

For the solid dispersion obtained in Example 4B, X-Ray powder diffraction experiments were performed under measurement conditions 1 described in Test Example 3 immediately after preparation, after storage for one week in an open glass bottle at 40°C and 75% relative humidity, after storage for one week in a closed glass bottle at 40°C and 75% relative humidity, and after storage for one week in a closed glass bottle at 60°C.

The results of Example 4B-1 are shown in Fig. 17, the results of Example 4B-2 are shown in Fig. 18, the results of Example 4B-3 are shown in Fig. 19, and the results of Example 4B-4 are shown in Fig. 20. Each of the solid dispersions obtained in Example 4B showed no diffraction peaks and exhibited only a halo pattern, both immediately after preparation and after storage under each conditions. It was confirmed that all of these solid dispersions remained amorphous form.

In addition, the anhydrous crystal alone of the compound represented by Formula (I) exhibited a clear X-Ray powder diffraction pattern, as described in Example 2B above.

### (Example 4D: Preparation 2 of Solid Dispersion of Compound Represented by Formula (I))

The anhydrous crystal of the compound represented by Formula (I) and copovidone (manufactured by ASHLAND) were dissolved in acetone, and after confirming complete dissolution, spray drying was carried out using a spray dryer under conditions of an outlet temperature of 50°C, a feed rate of about 6 kg/hour, and a spray pressure of 1.0 bar. It was then dried in vacuum for about 40 hours in a bench top vacuum drier to obtain a solid dispersion. A formulation is shown below.

**[Table 9]**

| Component | Example 4 D - 1 | |
|---|---|---|
| Anhydrous Crystal of Compound Represented by Formula (I) | 25% | 1425 g |
| Copovidone | 75% | 4275g |
| Total | 100% | 5700 g |

### <Particle Size Distribution of Solid Dispersion of Compound Represented by Formula (I)>

For the solid dispersion of Example 4D-1, the particle size distribution was measured under measurement conditions 3 (dry method) described in Test Example 10, and D10, D50, and D90 were 2.08 µm, 5.01 µm, and 11.00 µm, respectively.

### <Particle Size Distribution of Anhydrous Crystal of Compound Represented by Formula (I)>

For the anhydrous crystal of the compound represented by Formula (I) used in Example 4D-1, the particle size distribution was measured under measurement conditions 3 (dry method) described in Test Example 10, and D10, D50, and D90 were 0.86 µm, 3.29 µm, and 10.15 µm, respectively. For the anhydrous crystal of the compound represented by Formula (I) used in Example 4D-1, the particle size distribution was measured in the same manner as in Test Example 11 (wet method), and D10, D50, and D90 were 3.94 µm, 9.18 µm, and 20.32 µm, respectively.

### (Example 4E: Stability Test Over Time of Solid Dispersion of Compound Represented by Formula (I))

The solid dispersions obtained in Example 4D were stored for 3 months at 25°C and 60% relative humidity and for 3 months at 40°C and 75% relative humidity, and the increase in related substances was measured.

### Test Example 13: Analysis 2 of Related Substances

The amount of the related substances was measured by liquid chromatography using the following method and conditions. Detector: Ultraviolet absorptiometer (measurement wavelength: 247 nm)
Column: ACQUITY UPLC BEH C18 (1.7 µm, 2.1 × 100 mm, Waters)
Column temperature: constant temperature around 40°C
Mobile phase A: water/formic acid mixture (1000:1)
Mobile phase B: an acetonitrile/formic acid mixed solution for liquid chromatography (2000:1)
Mobile phase feeding: The mixing ratio of mobile phase A and mobile phase B was varied as follows to control a concentration gradient.

**[Table 10]**

| Time After Injection (Min) | Mobile Phase A (vol%) | Mobile Phase B (vol%) |
|---|---|---|
| 0~10 | 85→65 | 15→35 |
| 10~35 | 65 | 35 |
| 35~50 | 65→20 | 35→80 |
| 50~53 | 20 | 80 |
| 53~53.1 | 20→2 | 80→98 |
| 53.1~58 | 2 | 98 |
| 58~58.1 | 2→85 | 98→15 |
| 58.1~70(Equilibration)* | 85 | 15 |

| | | |
|---|---|---|
| *Equilibration time may be adjusted as appropriate. | | |

Flow rate: 0.4 mL/min
Injection volume: 5 µL
Sample cooler temperature: Constant temperature around 25°C
Needle wash solvent or autoinjector wash liquid: acetonitrile
Area measurement range: Up to 53 min after sample solution injection

The amounts of the related substances with a relative retention time of 0.44 at the start of the test, after storage for 3 months at 25°C and 60% relative humidity, and after storage for 3 months at 40°C and 75% relative humidity are shown below.

**[Table 11]**

| Amount of Related Substances With Relative Retention Time of 0.44 (%) | Example 4 D - 1 |
|---|---|
| At Start of Test | Not Detected |
| After Storage for 3 Months at 25° C and 60% Relative Humidity | 0.03 |
| After Storage for 3 Months at 40° C and 75% Relative Humidity | 0.08 |

Although the amount of the related substances with a relative retention time of 0.44 slightly increased compared with that at the start of the test, it was confirmed that the solid dispersion was stable.

### (Example 4F: Solubility Test of Solid Dispersion of Compound Represented by Formula (I))

The solubilities of the solid dispersion obtained in Example 4D and the anhydrous crystal alone of the compound represented by Formula (I) were measured.

### Test Example 14: Solubility Test

The solid dispersion obtained in Example 4D and the anhydrous crystal alone of the compound represented by Formula (I) (Example 4F-1) were added to various test media to prepare suspensions of 30 to 50 mL. The prepared suspensions were placed in centrifuge tubes and shaken at a shaking speed of 100 rpm or higher. After 60 minutes from the start of shaking, 5 mL of each suspension was collected and immediately filtered through a filter (Chromadisc, 0.45 µm, 25A) to obtain test samples.

The solubilities in various test solutions are shown below. The solid dispersion of the compound represented by Formula (I) exhibited a markedly improved solubility compared with that of the anhydrous crystal alone of the compound represented by Formula (I).

**[Table 12]**

| | Example 4 D - 1 | Example4 F - 1 |
|---|---|---|
| Purified Water | 43.97 | 0.71 |
| First Dissolution Test Medium in Japanese Pharmacopoeia (pH1.2) | 63.17 | 1.08 |
| Diluted McIlvaine Buffer (pH4.0) | 41.42 | 0.64 |
| Second Dissolution Test Medium in Japanese Pharmacopoeia (pH6.8) | 38.76 | 0.61 |

### (Example 4G: Rat PK Study of Solid Dispersion of Compound Represented by Formula (I))

A suspension of a solid dispersion comprising the anhydrous crystal alone of the compound represented by Formula (I) and copovidone was produced, and oral absorption in rats was evaluated.

### Test Example 15: Rat PK Study

After feeding, test samples were orally administered to male rats. The administration volume of each suspension was adjusted so that the dose of the compound represented by Formula (I) was 3 mg/kg body weight.

After administration of Example 4G-1, Example 4G-2, and Example 4G-3 described below, blood samples were collected at each predetermined sampling time point. Using LC/MS/MS, the maximum plasma drug concentration (Cmax), the time to reach the maximum plasma drug concentration (Tmax), and the area under the plasma drug concentration-time curve from the time of administration to 24 hours after administration (AUC) were calculated.

### <Example 4G-1: Preparation of Suspension of Solid Dispersion of Compound Represented by Formula (I)>

The compound represented by Formula (I) and copovidone (manufactured by ASHLAND) were dissolved in acetone at a weight ratio of 1:3, and after confirming complete dissolution, spray drying was performed using a spray dryer under conditions of an outlet temperature of 50°C, a feed rate of about 6 kg/hour, and a spray pressure of 1.0 bar. The obtained product was vacuum-dried in a bench-top vacuum dryer for about 40 hours to obtain a solid dispersion.

The resulting solid dispersion was suspended in an aqueous solution containing 0.5% methylcellulose to a concentration of 6 mg/mL, thereby obtaining a suspension.

### <Example 4G-2: Preparation of Suspension of Anhydrous Crystal of Compound Represented by Formula (I)>

The anhydrous crystal of the compound represented by Formula (I) was suspended in an aqueous solution containing 0.5% methylcellulose to a concentration of 6 mg/mL, thereby obtaining a suspension.

### <Example 4G-3: Preparation of Suspension of Ethyl Acetate Solvate Crystal of Compound Represented by Formula (I)>

The ethyl acetate solvate crystal of the compound represented by Formula (I) was suspended in an aqueous solution containing 0.5% methylcellulose to a concentration of 6 mg/mL, thereby obtaining a suspension.

The results of the Cmax, Tmax, and AUC of the compound represented by Formula (I) in each Example are shown below.

**[Table 13]**

| Specimen | Cmax (ng/mL) | Tmax (h r) | AUC (ng·hr/mL) |
|---|---|---|---|
| Example 4 G-1 (Suspension of Solid Dispersion) | 214 | 1.7 | 2920 |
| Example 4G-2 (Suspension of Anhydrous Crystal) | 43.2 | 3.3 | 613 |
| Example 4G-3 (Suspension of Ethyl Acetate Solvate Crystal ) | 37.2 | 4.7 | 542 |

Example 4G-1 showed increased Cmax and AUC compared with Examples 4G-2 and 4G-3, indicating a marked improvement in oral absorption. In addition, Example 4G-1 exhibited a shorter Tmax than Examples 4G-2 and 4G-3, demonstrating more rapid absorption.

The method for producing and evaluation results of the "preparation comprising, as an active ingredient, a compound represented by Formula (I)" are shown below.

### (Example 5: Preparation Comprising Anhydrous Crystal of Compound Represented by Formula (I))

The method for producing and evaluation results of the "preparation comprising, as an active ingredient, an anhydrous crystal of the compound represented by Formula (I)" are shown below.

### (Example 5A: Examination 1 of Uncoated Tablet Comprising Anhydrous Crystal of Compound Represented by Formula (I))

Uncoated tablets containing 50 wt% anhydrous crystal of the compound represented by Formula (I) and 5 wt% disintegrant were produced, and a dissolution test was conducted.

### <Method of Producing Uncoated Tablet>

An anhydrous crystal of the compound represented by Formula (I), D-mannitol (manufactured by ROQUETTE), crystalline cellulose (manufactured by Asahi Kasei Corp.), croscarmellose sodium (manufactured by Dupont), low-substituted hydroxypropyl cellulose (manufactured by Shin-Etsu Chemical Co., Ltd.), sodium starch glycolate (manufactured by JRS Pharma), crospovidone (grades CLM and CL, manufactured by BASF), and magnesium stearate (manufactured by MALLINCKRODT) were used. The formulation is shown below (units: mg).

**[Table 14]**

| Component (mg) | Example 5 A - 1 | Example 5 A - 2 | Example 5 A - 3 | Example 5 A - 4 | Example 5 A - 5 |
|---|---|---|---|---|---|
| Anhydrous Crystal of Compound Represented by Formula (I) | 30.0 | 30.0 | 30.0 | 30.0 | 30.0 |
| D-Mannitol | 12.9 | 12.9 | 12.9 | 12.9 | 12.9 |
| Crystalline Cellulose | 12.9 | 12.9 | 12.9 | 12.9 | 12.9 |
| Crospovidone (Grade CLM) | 3.0 | - | - | - | - |
| Low-Substituted Hydroxypropyl Cellulose | - | 3.0 | - | - | - |
| Sodium Starch Glycolate | - | - | 3.0 | - | - |
| Crospovidone (Grade CL) | - | - | - | 3.0 | - |
| Croscarmellose Sodium | - | - | - | - | 3.0 |
| Magnesium Stearate | 1.2 | 1.2 | 1.2 | 1.2 | 1.2 |
| Total in Uncoated Tablet (mg) | 60.0 | 60.0 | 60.0 | 60.0 | 60.0 |

The anhydrous crystal of the compound represented by Formula (I), a filler, a disintegrant, and half an amount of magnesium stearate were mixed with a spatula and sieved with a wire mesh. Thereafter, the plate was tableted using a simple tablet forming machine (model: HANDTAB-200, manufactured by Ichihashi Seiki Co., Ltd.). The mixture was then sized with a 20 mesh sieve, and the remaining magnesium stearate was added and mixed with a spatula. The obtained tableting granules were tableted with a simple tablet forming machine (Model: HANDTAB-200, manufactured by Ichihashi Seiki Co., Ltd.) to obtain uncoated tablets.

### <Dissolution Test>

### Test Example 16: Dissolution Test

For each preparation obtained in the Examples (one tablet in the case of tablets), a dissolution test was performed in accordance with the Dissolution Test described in the Japanese Pharmacopoeia 18th Edition. As the test solution, a second dissolution test medium containing a surfactant was used, and the test was performed by the paddle method at a paddle rotation speed of 50 rpm (which was changed to 250 rpm after 120 minutes from the start of the test).

As a result, Example 5A-1 using crospovidone (grade CLM) exhibited the most preferable dissolution profile.

### (Example 5B: Examination 2 of Uncoated Tablet Comprising Anhydrous Crystal of Compound Represented by Formula (I))

Uncoated tablets containing 50 wt% anhydrous crystal of the compound represented by Formula (I) and 10 wt% disintegrant were produced, and a dissolution test was conducted.

### <Method of Producing Uncoated Tablet>

The tablets were produced in the same manner as in Example 5A. A formulation is shown below.

**[Table 15]**

| Component (mg) | Example 5 B - 1 | Example 5 B - 2 | Example 5 B - 3 |
|---|---|---|---|
| Anhydrous Crystal of Compound Represented by Formula (I) | 30.0 | 30.0 | 30.0 |
| D-Mannitol | 11.4 | 11.4 | 11.4 |
| Crystalline Cellulose | 11.4 | 11.4 | 11.4 |
| Crospovidone (Grade CLM) | 6.0 | - | - |
| Low-Substituted Hydroxypropyl Cellulose | - | 6.0 | - |
| Croscarmellose Sodium | - | - | 6.0 |
| Magnesium Stearate | 1.2 | 1.2 | 1.2 |
| Total in Uncoated Tablet (mg) | 60.0 | 60.0 | 60.0 |

### <Dissolution Test>

The dissolution test of Test Example 16 was performed. As a result, Examples 5B-1, 5B-2, and 5B-3 (disintegrant mixing content: 10 wt%) exhibited improved dissolution rates for all disintegrants as compared with Examples 5A-1, 5A-2, and 5A-5 (disintegrant mixing content: 5 wt%). In addition, Example 5B-1 using crospovidone as the disintegrant exhibited the most rapid dissolution profile.

### (Example 5C: Examination 3 of Uncoated Tablet Comprising Anhydrous Crystal of Compound Represented by Formula (I))

Uncoated tablets containing 10 wt% anhydrous crystal of the compound represented by Formula (I) and 10 wt% disintegrant were produced, and a dissolution test was conducted.

### <Method of Producing Uncoated Tablet>

An anhydrous crystal of the compound represented by Formula (I), D-mannitol (manufactured by ROQUETTE), crystalline cellulose (manufactured by Asahi Kasei Corp.), crospovidone (grade INF-10, manufactured by ASHLAND), croscarmellose sodium (manufactured by Dupont), and sodium stearyl fumarate (manufactured by JRS Pharma) were used.

The tablets were produced in the same manner as in Examples 5A and 5B. A formulation is shown below.

**[Table 16]**

| Component (mg) | Example 5 C - 1 | Example 5 C - 2 |
|---|---|---|
| Anhydrous Crystal of Compound Represented by Formula (I) | 20.0 | 20.0 |
| D-Mannitol | 121.6 | 121.6 |
| Crystalline Cellulose | 30.4 | 30.4 |
| Crospovidone | 20.0 | - |
| Croscarmellose Sodium | - | 20.0 |
| Sodium Stearyl Fumarate | 8.0 | 8.0 |
| Total in Uncoated Tablet (mg) | 200.0 | 200.0 |

### <Dissolution Test>

The dissolution test of Test Example 16 was performed. As a result, Example 5C-1 using crospovidone as the disintegrant exhibited preferable dissolution profile. Accordingly, it is considered that crospovidone is optimal as the disintegrant when the concentration of the anhydrous crystal of the compound represented by Formula (I) is 10 to 50 wt%.

### (Example 5D: Suspension Comprising Anhydrous Crystal of Compound Represented by Formula (I))

A suspension containing an anhydrous crystal of the compound represented by Formula (I) was prepared. A dissolution test was performed for this suspension and Example 5C-1 (uncoated tablet containing an anhydrous crystal of the compound represented by Formula (I)).

### <Method for Preparing Suspension>

An anhydrous crystal of the compound represented by Formula (I), hydroxypropyl cellulose (manufactured by Nippon Soda Co., Ltd.), and titanium oxide (manufactured by Merck) were mixed using a spatula, and then kneaded with a spatula while adding a small amount of water for injection (manufactured by Otsuka Pharmaceutical Factory, Inc.). Thereafter, the remaining water for injection was added while applying ultrasonic irradiation, thereby obtaining a suspension. A formulation is shown below.

**[Table 17]**

| Component | Example 5 D - 1 |
|---|---|
| Anhydrous Crystal of Compound Represented by Formula (I)(mg) | 20 |
| Hydroxypropyl Cellulose (mg) | 300 |
| Titanium Oxide (mg) | 60 |
| Water for Injection (mL) | 30 |

### <Dissolution Test>

The dissolution test of Test Example 16 was performed. The dissolution test results of Example 5C-1 and Example 5D-1 are shown in Fig. 21. As a result, Example 5C-1 (uncoated tablet) exhibited more rapid dissolution as compared with Example 5D-1 (suspension).

### (Example 5E: Examination 1 of Tablet Comprising Anhydrous Crystal of Compound Represented by Formula (I))

In order to examine the influence of photostabilizing agent, uncoated tablets containing an anhydrous crystal of the compound represented by Formula (I) were coated with photostabilizing agents and a polymer, and the amounts of related substances of the preparations and the appearance thereof were evaluated. For the uncoated tablets and the coated tablets, the stability test under heated and humidified conditions was performed, and dissolution profile were evaluated.

### <Method of Producing Tablets>

An anhydrous crystal of the compound represented by Formula (I), D-mannitol (ROQUETTE), crystalline cellulose (Asahi Kasei Corp.), crospovidone (grade INF-10, ASHLAND), and half of the sodium stearyl fumarate (JRS Pharma) were mixed in a polyethylene bag and sieved through a 30-mesh sieve. Thereafter, granulation was carried out using a roller compactor under the conditions of roll pressure of 7 MPa, roll speed of about 4 rpm, and screw speed of about 30 rpm. Thereafter, sizing was performed using a sizing machine at a rotational speed of about 1800 rpm. The sized granules and the remaining half of the sodium stearyl fumarate were mixed in a mixer for 5 minutes, and then compressed using a rotary tableting machine at a turret rotation speed of 20 to 30 rpm to produce uncoated tablets.

Thereafter, coating was performed using a coating machine under conditions of an inlet air flow rate of 0.80 m3/min, an inlet air temperature setting of 60°C, a liquid flow rate of 2.0 to 2.9 g/min, a spray pressure of about 0.18 MPa, and a spray air flow rate of about 50 NL/min, thereby obtaining tablets of Example 5E-1. As the photostabilizing agent, talc, red ferric oxide, and yellow ferric oxide were used, and hypromellose was used as the polymer. A formulation is shown below.

**[Table 18]**

| Component (mg) | Example 5 E -1 |
|---|---|
| Anhydrous Crystal of Compound Represented by formula (I) | 20.0 |
| D-Mannitol | 121.6 |
| Crystalline Cellulose | 30.4 |
| Crospovidone | 20.0 |
| Sodium Stearyl Fumarate | 8.0 |
| Total in Uncoated Tablet (mg) | 200.0 |
| Hypromellose | 4.78 |
| Talc | 1.97 |
| Red Ferric Oxide | 0.02 |
| Yellow Ferric Oxide | 0.02 |
| Total in Coating Layer (mg) | 6.8 |
| Total in Coated Tablet (mg) | 206.8 |

### <Dissolution Test>

The tablets of Example 5E-1 were stored for 1 month in a closed polyethylene bottle at 40°C and 75% relative humidity, and for 3 months in a closed polyethylene bottle at 40°C and 75% relative humidity. At the start of the test and after storage under each condition, a dissolution test was performed according to the method of Test Example 16.

The results are shown in Fig. 22. It was confirmed that the dissolution rate did not decrease even after storage for 3 months under heated and humidified conditions.

### <Stability Test>

An uncoated tablet containing an anhydrous crystal of the compound represented by Formula (I) (Example 5C-1 above) and a tablet coated with a stabilizing substance and a polymer (Example 5E-1 above) were irradiated with light at a total illumination of 1.2 million lux·hr, and the amounts of related substances of the preparations and the appearance thereof were evaluated at the start of the test and after irradiation with light of 1.2 million lux·hr. The amounts of related substances measured were those having relative retention times of 0.84 and the total amount of related substances. In addition, the appearance properties were visually evaluated.

### Test Example 17: Analysis 3 of Related Substances

The amount of the related substances was measured by liquid chromatography using the following method and conditions. Detector: Ultraviolet absorptiometer (measurement wavelength: 247 nm)
Column: ACQUITY UPLC BEH C18 (1.7 µm, 2.1 × 100 mm, Waters)
Column temperature: constant temperature around 40°C
Mobile phase A: water/formic acid mixture (1000:1)
Mobile phase B: an acetonitrile/formic acid mixed solution for liquid chromatography (2000:1)
Mobile phase feeding: The mixing ratio of mobile phase A and mobile phase B was varied as follows to control a concentration gradient.

**[Table 19]**

| Time After Injection (Min) | Mobile Phase A (vol%) | Mobile Phase B (vol%) |
|---|---|---|
| 0~10 | 85→65 | 15→35 |
| 10~35 | 65 | 35 |
| 35~40 | 65→20 | 35→80 |
| 40~43 | 20 | 80 |
| 43~43.1 | 20→2 | 80→98 |
| 43.1~48 | 2 | 98 |
| 48~48.1 | 2→85 | 98→15 |
| 48.1~60 (Equilibration) * | 85 | 15 |

| | | |
|---|---|---|
| * Equilibration time may be adjusted as appropriate. | | |

Flow rate: 0.4 mL/min
Injection volume: 5 µL
Sample cooler temperature: Constant temperature around 25°C
Needle wash solvent or autoinjector wash liquid: acetonitrile
Area measurement range: Up to 43 min after sample solution injection

The amounts of the related substance having a relative retention time of 0.84 at the start of the test and after light irradiation with a total illumination of 1.2 million lux·hr are shown below.

**[Table 20]**

| Amount of Related Substances With Relative Retention Time of 0.84 (%) | Example 5 C - 1 (Uncoated Tablet) | Example 5E - 1 (Tablet Having Coating Layer) |
|---|---|---|
| At Start of Test | Not Detected | Not Detected |
| 1.2 Million lux·hr (Light Exposure) | 0.28 | 0.04 |
| 1.2 Million lux·hr (Dark Control) | Not Detected | Not Detected |

The total amount of related substances at the start of the test and after light irradiation with a total illumination of 1.2 million lux·hr is shown below.

**[Table 21]**

| Total Amount of Related Substances (%) | Example 5 C - 1 (Uncoated Tablet) | Example 5 E - 1 (Tablet Having Coating Laver) |
|---|---|---|
| At Start of Test | 0.43 | 0.41 |
| 1.2 Million lux·hr (Light Exposure) | 2.44 | 0.66 |
| 1.2 Million lux·hr (Dark Control) | 0.46 | 0.47 |

After light irradiation with a total illumination of 1.2 million lux·hr, Example 5E-1, as compared with Example 5C-1, showed a marked suppression of both the amount of the related substance having a relative retention time of 0.84 and the total amount of related substances. With respect to appearance after light irradiation with a total illumination of 1.2 million lux·hr, Example 5C-1 exhibited yellow discoloration as compared with before irradiation, whereas Example 5E-1 exhibited no observable change in appearance as compared with before irradiation.

### (Example 5F: Examination of Capsule and Uncoated Tablet Comprising Anhydrous Crystal of Compound Represented by Formula (I))

Granules containing an anhydrous crystal of the compound represented by Formula (I) were filled into capsules to prepare capsules. The amount of related substances in the capsules and uncoated tablets were evaluated. As related substances, the amount of the substance having a relative retention time of 0.98 and the total amount of related substances were measured.

### <Method for Producing Capsule and Uncoated Tablet>

An anhydrous crystal of the compound represented by Formula (I), D-mannitol (ROQUETTE), crystalline cellulose (Asahi Kasei Corp.), croscarmellose sodium (Dupont), and half of the magnesium stearate (MALLINCKRODT) were mixed using a spatula and sieved through a wire mesh. The mixture was then compressed using a simple tablet forming machine (model: HANDTAB-200, manufactured by Ichihashi Seiki Co., Ltd.). Thereafter, the tablets were sized through a 20-mesh sieve, the remaining half of the magnesium stearate was added and mixed using a spatula to obtain granules.

The granules were manually filled into hypromellose capsules (manufactured by Qualicaps) using a spatula to prepare the capsules of Example 5F-1. In addition, the granules were compressed using the simple tablet forming machine (model: HANDTAB-200, manufactured by Ichihashi Seiki Co., Ltd.) to prepare the uncoated tablets of Example 5F-2. Each exemplary formulation is shown below.

**[Table 22]**

| Component (mg) | Example 5 F - 1 (Capsule) | Example 5 F *- 2* (Uncoated Tablet) |
|---|---|---|
| Anhydrous Crystal of Compound Represented by Formula (I) | 30.0 | 30.0 |
| D-Mannitol | 54.8 | 54.8 |
| Crystalline Cellulose | 54.8 | 54.8 |
| Croscarmellose Sodium | 7.5 | 7.5 |
| Magnesium Stearate | 3.0 | 3.0 |
| **Total in Granule (mg)** | **150.0** | **150.0** |
| | **Filled Into Hypromellose Capsule** | - |
| **Total in Uncoated Tablet (mg)** | **-** | **150.0** |

### Test Example 18: Analysis 4 of Related Substances

The amount of the related substances was measured by liquid chromatography using the following method and conditions. Detector: Ultraviolet absorptiometer (measurement wavelength: 254 mm)
Column: ACQUITY UPLC BEH C18 (1.7 µm, 2.1 × 100 mm, Waters)
Column temperature: constant temperature around 40°C
Mobile phase A: water/trifluoroacetic acid mixture (1000:1)
Mobile phase B: Acetonitrile for high performance liquid chromatography
Mobile phase feeding: The mixing ratio of mobile phase A and mobile phase B was varied as follows to control a concentration gradient.

**[Table 23]**

| Time After Injection (Min) | Mobile Phase A (vol%) | Mobile Phase B (vol%) |
|---|---|---|
| 0~2 7 | 90→10 | 10→90 |
| 2 7~30 | 10 | 90 |
| 30~30.1 | 10→90 | 90→10 |
| 30.1~39.5 | 90 | 10 |

Flow rate: 0.3 mL/min
Injection volume: 4 µL
Sample cooler temperature: constant temperature around 10°C Needle wash solvent: Methanol

### <Stability Test>

The amounts of the related substance having a relative retention time of 0.98 at the start of the test and after light irradiation with a total illumination of 1.2 million lux·hr are shown below.

**[Table 24]**

| Amount of Related Substances With Relative Retention Time of 0.98 (%) | Example 5 F - 1 (Capsule) | Example 5 F - 2 (Uncoated Tablet) |
|---|---|---|
| At Start of Test | 0.02 | 0.03 |
| 1.2 Million lux·hr (Light Exposure) | 0.02 | 0.35 |
| 1.2 Million lux·hr (Dark Control) | 0.02 | 0.01 |

The total amount of related substances at the start of the test and after light irradiation with a total illumination of 1.2 million lux·hr is shown below.

**[Table 25]**

| Total Amount of Related Substances (%) | Example 5 F - 1 (Capsule) | Example 5 F - 2 (Uncoated Tablet) |
|---|---|---|
| At Start of Test | 5.32 | 5.52 |
| 1.2 Million lux·hr (Light Exposure) | 5.33 | 6.73 |
| 1.2 Million lux·hr (Dark Control) | 5.30 | 5.90 |

It was confirmed that Example 5F-1, as compared with Example 5F-2, exhibited a marked suppression of both the amount of the related substance having a relative retention time of 0.98 and the total amount of related substances. It was confirmed that a light-shielding effect achieved by capsule filling.

### (Example 6: Preparation Comprising Solid Dispersion of Compound Represented by Formula (I))

Formulation studies of a preparation comprising a solid dispersion of the compound represented by Formula (I) are shown below.

### (Example 6A: Examination of Uncoated Tablet Comprising Solid Dispersion of Compound Represented by Formula (I))

Uncoated tablets containing a solid dispersion of the compound represented by Formula (I) were produced, and a dissolution test was conducted.

### <Method of Producing Uncoated Tablet>

An anhydrous crystal of the compound represented by Formula (I) and copovidone were dissolved in acetone, and after confirming complete dissolution, spray drying was performed using a spray dryer under conditions of an outlet temperature of 50°C, a feed rate of about 6 kg/hr, and a spray pressure of 1.0 bar. It was then dried in vacuum for about 40 hours in a bench top vacuum drier to obtain a solid dispersion.

The obtained solid dispersion, D-mannitol (manufactured by ROQUETTE), crystalline cellulose (manufactured by Asahi Kasei Corp.), croscarmellose sodium (manufactured by Dupont) or crospovidone (manufactured by ASHLAND), light anhydrous silicic acid (manufactured by Cabot), and half of the sodium stearyl fumarate (manufactured by JRS Pharma) were mixed using a spatula and sieved through a wire mesh. The mixture was then compressed using a simple tablet forming machine (model: HANDTAB-200, manufactured by Ichihashi Seiki Co., Ltd.). Thereafter, the tablets were sized through a 20-mesh sieve, the remaining half of the sodium stearyl fumarate was added and mixed using a spatula, and the resulting granules were compressed using the simple tablet forming machine (model: HANDTAB-200, manufactured by Ichihashi Seiki Co., Ltd.) to obtain uncoated tablets. A formulation is shown below.

**[Table 26]**

| Component (mg) | Example 6 A - 1 | Example 6 A - 2 |
|---|---|---|
| Solid Dispersion of Compound Represented by Formula (I) | 80.0 | 30.0 |
| Compound Represented by Formula (I) in Solid Dispersion) | (20.0) | (20.0) |
| Copovidone in Solid Dispersion) | (60.0) | (60.0) |
| D-Mannitol | 74.4 | 74.4 |
| Crystalline Cellulose | 18.6 | 18.6 |
| Croscarmellose Sodium | 20.0 | - |
| Crospovidone | - | 20.0 |
| Light Anhydrous Silicic Acid | 1.0 | 1.0 |
| Sodium Stearyl Fumarate | 6.0 | 6.0 |
| Total in Uncoated Tablet (mg) | 200.0 | 200.0 |

### <Dissolution Test>

The dissolution test of Test Example 16 was performed. As a result, Example 6A-1 using croscarmellose sodium exhibited preferable dissolution profile. Accordingly, it is considered that croscarmellose sodium is suitable as the disintegrant for tablets containing a solid dispersion.

### (Example 6B-1: Examination 1 of Tablet Comprising Solid Dispersion of Compound Represented by Formula (I))

Tablets were produced by coating uncoated tablets containing a solid dispersion of the compound represented by Formula (I) with a photostabilizing agent and a polymer. The tablets were subjected to a stability test under heated and humidified conditions.

### <Method of Producing Tablets>

An anhydrous crystal of the compound represented by Formula (I) and copovidone were dissolved in acetone, and after confirming complete dissolution, spray drying was performed using a spray dryer under conditions of an outlet temperature of 50°C, a feed rate of about 6 kg/hr, and a spray pressure of 1.0 bar. It was then dried in vacuum for about 40 hours in a bench top vacuum drier to obtain a solid dispersion.

The obtained solid dispersion was mixed with D-mannitol (manufactured by ROQUETTE), crystalline cellulose (manufactured by Asahi Kasei Corp.), croscarmellose sodium (manufactured by Dupont), light anhydrous silicic acid (manufactured by Cabot), and half of the sodium stearyl fumarate (manufactured by JRS Pharma) in a mixer for 8 minutes, and sieved through a 30-mesh sieve. Thereafter, dry granulation was carried out using a roller compactor under the conditions of roll pressure of 7 MPa, roll speed of about 4 rpm, and screw speed of about 40 rpm. Thereafter, dry sizing was carried out using a sizing machine at a rotational speed of about 3000 rpm. The sized granules and the remaining half of the sodium stearyl fumarate were mixed in a mixer for 5 minutes, and then compressed using a rotary tableting machine at a turret rotation speed of about 30 rpm to produce uncoated tablets.

Thereafter, coating was performed using a coating machine under conditions of an inlet air flow rate of 0.80 m3/min, an inlet air temperature setting of 60°C, a liquid flow rate of 2.0 to 2.9 g/min, a spray pressure of about 0.18 MPa, and a spray air flow rate of about 50 NL/min, thereby obtaining tablets. A formulation is shown below.

**[Table 27]**

| Component (mg) | Example 6 B - 1 |
|---|---|
| Solid Dispersion of Compound Represented by formula (I) | 80.0 |
| (Compound Represented by Formula (I) in Solid Dispersion) | (20.0) |
| (Copovidone in Solid Dispersion) | (60.0) |
| D-Mannitol | 74.4 |
| Crystalline Cellulose | 18.6 |
| Croscarmellose Sodium | 20.0 |
| Light Anhydrous Silicic Acid | 1.0 |
| Sodium Stearyl Fumarate | 6.0 |
| Total in Uncoated Tablet (mg) | 200.0 |
| Hypromellose | 5.63 |
| Talc | 2.32 |
| Red Ferric Oxide | 0.03 |
| Yellow Ferric Oxide | 0.03 |
| Total in Coating Layer (mg) | 8.0 |
| Total in Coated Tablet (mg) | 208.0 |

### <Stability Test and Dissolution Test>

The tablets of Example 6B-1 were stored under the following conditions: 2 weeks at 60°C in a closed amber glass bottle; 1 month at 40°C in a closed amber glass bottle; and 1 month at 40°C and 75% relative humidity in a closed amber glass bottle. At the start of the test and after storage under each condition, a dissolution test was performed according to the method of Test Example 16.

The results are shown in Fig. 23. It was confirmed that the dissolution rate did not decrease after storage under both conditions.

### (Example 6B-2: Examination 2 of Tablet Comprising Solid Dispersion of Compound Represented by Formula (I))

Uncoated tablets having a different content of the solid dispersion of the compound represented by Formula (I) and copovidone from that of Example 6B-1 were produced, and coated with a photostabilizing agent and a polymer to obtain tablets. The tablets were subjected to a stability test under heated and humidified conditions.

### <Method of Producing Tablets>

An anhydrous crystal of the compound represented by Formula (I) and copovidone were dissolved in acetone, and after confirming complete dissolution, spray drying was performed using a spray dryer under conditions of an outlet temperature of 50°C, a feed rate of about 80 kg/hr, and a spray pressure of 2.5 bar. Thereafter, they were dried in vacuum for about 37 hours in a rotary vacuum drier to obtain a solid dispersion.

The obtained solid dispersion was mixed with D-mannitol (manufactured by ROQUETTE), crystalline cellulose (manufactured by Asahi Kasei Corp.), croscarmellose sodium (manufactured by Dupont), light anhydrous silicic acid (manufactured by Cabot), and half of the sodium stearyl fumarate (manufactured by JRS Pharma) in a mixer for 15 minutes, and sieved using a 1.6-mm screen. Thereafter, dry granulation was carried out using a roller compactor under the conditions of roll pressure of 5 MPa, roll speed of about 5 rpm, and screw speed of about 5 rpm. Thereafter, dry sizing was carried out using a sizing machine at a rotational speed of about 100 rpm. The sized granules and the remaining half of the sodium stearyl fumarate were mixed in a mixer for 3 minutes, and then compressed using a rotary tableting machine at a turret rotation speed of about 20 rpm to produce uncoated tablets.

Thereafter, coating was performed using a coating machine under conditions of an inlet air flow rate of 12 m3/min, an inlet air temperature setting of 60°C, a liquid flow rate of 40 to 80 g/min, a spray pressure of about 0.4 MPa, and a spray air flow rate of about 130 NL/min, thereby obtaining tablets. A formulation is shown below.

**[Table 28]**

| Component (mg) | Example 6 B -2 |
|---|---|
| Solid Dispersion of Compound Represented by formula (I) | 160.0 |
| (Compound Represented by Formula (I) in Solid Dispersion) | (40.0) |
| (Copovidone in Solid Dispersion) | (120.0) |
| D-Mannitol | 148.8 |
| Crystalline Cellulose | 37.2 |
| Croscarmellose Sodium | 40.0 |
| Light Anhydrous Silicic Acid | 2.0 |
| Sodium Stearyl Fumarate | 12.0 |
| Total in Uncoated Tablet (mg) | 400.0 |
| Hypromellose | 11.26 |
| Talc | 4.64 |
| Red Ferric Oxide | 0.06 |
| Yellow Ferric Oxide | 0.06 |
| Total in Coating Layer (mg) | 16.0 |
| Total in Coated Tablet (mg) | 416.0 |

### <Stability Test and Dissolution Test>

The tablets of Example 6B-2 were stored under the following conditions: 2 weeks at 60°C in a closed amber glass bottle; 1 month at 40°C in a closed amber glass bottle; and 1 month at 40°C and 75% relative humidity in a closed amber glass bottle. At the start of the test and after storage under each condition, a dissolution test was performed according to the following method.

### (Dissolution Test Method)

One tablet of each sample was subjected to a dissolution test in accordance with the Dissolution Test described in the Japanese Pharmacopoeia 18th Edition. The second dissolution test medium was used as the test solution, and the test was performed by the paddle method at a paddle rotation speed of 50 rpm.

The results are shown in Fig. 24. It was confirmed that the dissolution rate did not decrease after storage under both conditions.

### (Example 6C: Examination 3 of Tablet Comprising Solid Dispersion of Compound Represented by Formula (I))

Two types of uncoated tablets having different concentration of the solid dispersion comprising an anhydrous crystal of the compound represented by Formula (I) and copovidone from that of Example 6B-1 were produced. Each uncoated tablet was coated with a photostabilizing agent and a polymer to obtain tablets. The tablets were subjected to a stability test under heated and humidified conditions.

### <Method of Producing Tablets>

An anhydrous crystal of the compound represented by Formula (I) and copovidone were dissolved in acetone, and after confirming complete dissolution, spray drying was performed using a spray dryer under conditions of an inlet temperature of 90°C, a liquid feed pump setting of 20%, and a nitrogen flow rate setting of 40.

The obtained solid dispersion, D-mannitol (manufactured by ROQUETTE), crystalline cellulose (manufactured by Asahi Kasei Corp.), croscarmellose sodium (manufactured by Dupont), and half of the sodium stearyl fumarate (manufactured by JRS Pharma) were mixed using a spatula and sieved through a wire mesh. The mixture was then compressed using a simple tablet forming machine (model: HANDTAB-200, manufactured by Ichihashi Seiki Co., Ltd.). Thereafter, the tablets were sized through a 20-mesh sieve, the remaining half of the sodium stearyl fumarate was added and mixed using a spatula, and the resulting granules were compressed using the simple tablet forming machine (model: HANDTAB-200, manufactured by Ichihashi Seiki Co., Ltd.). Thereafter, coating was performed using a coating machine (manufactured by Powrex Corp.)to obtain tablets. A formulation is shown below.

**[Table 29]**

| Component (mg) | Example 6 C- 1 | Example 6 C-2 |
|---|---|---|
| Solid Dispersion of Compound Represented by formula (I) | 60.0 | 90.0 |
| (Compound Represented by Formula (I) in Solid Dispersion) | (30.0) | (30.0) |
| (Copovidone in Solid Dispersion) | (30.0) | (60.0) |
| D-Mannitol | 163.2 | 139.2 |
| Crystalline Cellulose | 40.8 | 34.8 |
| Croscarmellose Sodium | 30.0 | 30.0 |
| Sodium Stearyl Fumarate | 6.0 | 6.0 |
| Total in Uncoated Tablet (mg) | 300.0 | 300.0 |
| Hypromellose | 8.44 | 8.44 |
| Talc | 3.48 | 3.48 |
| Red Ferric Oxide | 0.04 | 0.04 |
| Yellow Ferric Oxide | 0.04 | 0.04 |
| Total in Coating Layer (mg) | 12.0 | 12.0 |
| Total in Coated Tablet (mg) | 312.0 | 312.0 |

### <Stability Test and Dissolution Test>

The tablets of Examples 6C-1 and 6C-2 were stored for 1 week at 60°C in closed amber glass bottles and for 1 week at 40°C and 75% relative humidity in closed amber glass bottles, followed by dissolution test. The dissolution test of Test Example 16 was performed.

The results of Example 6C-1 are shown in Fig. 25, and those of Example 6C-2 are shown in Fig. 26. It was confirmed that the dissolution rate did not decrease after storage for one week in a closed glass bottle at 60°C and after storage for one week in a closed glass bottle at 40°C and 75% relative humidity.

### (Example 6D: Examination 4 of Tablet Comprising Solid Dispersion of Compound Represented by Formula (I))

Uncoated tablets containing a solid dispersion comprising an anhydrous crystal of the compound represented by Formula (I) and copovidone, and tablets obtained by coating the uncoated tablets with a photostabilizing agent and a polymer, were produced. The uncoated tablets and coated tablets were irradiated with light at a total illumination of 1.2 million lux·hr, and the amounts of related substances of the preparations and the appearance thereof were evaluated at the start of the test and after irradiation with light of 1.2 million lux·hr.

### <Method for Producing Uncoated Tablet and Coated Tablet>

An anhydrous crystal of the compound represented by Formula (I) and copovidone were dissolved in acetone, and after confirming complete dissolution, spray drying was performed under conditions of an outlet temperature of 50°C, a feed rate of about 6 kg/hr, and a spray pressure of 1.0 bar. It was then dried in vacuum for about 40 hours in a bench top vacuum drier to obtain a solid dispersion.

The obtained solid dispersion was mixed with D-mannitol (manufactured by ROQUETTE), crystalline cellulose (manufactured by Asahi Kasei Corp.), croscarmellose sodium (manufactured by Dupont), light anhydrous silicic acid (manufactured by Cabot), and half of the sodium stearyl fumarate (manufactured by JRS Pharma) in a mixer for 8 minutes, and sieved through a 30-mesh sieve. Thereafter, dry granulation was carried out using a roller compactor under the conditions of roll pressure of 7 MPa, roll speed of about 4 rpm, and screw speed of about 40 rpm. Thereafter, dry sizing was carried out using a sizing machine at a rotational speed of about 3000 rpm. The sized granules and the remaining half of the sodium stearyl fumarate were mixed in a mixer for 5 minutes, and then compressed using a rotary tableting machine at a turret rotation speed of about 30 rpm to produce uncoated tablets (Example 6D-1).

For Example 6D-2, thereafter, coating was performed using a coating machine under conditions of an inlet air flow rate of 0.80 m3/min, an inlet air temperature setting of 60°C, a liquid flow rate of 2.0 to 2.9 g/min, a spray pressure of about 0.18 MPa, and a spray air flow rate of about 50 NL/min, thereby obtaining tablets. A formulation is shown below.

**[Table 30]**

| Component (mg) | Example 6 D- 1 | Example 6 D- 2 |
|---|---|---|
| Solid Dispersion of Compound Represented by formula (I) | 80.0 | 80.0 |
| (Compound Represented by Formula (I) in Solid Dispersion) | (20.0) | (20.0) |
| (Copovidone in Solid Dispersion) | (60.0) | (60.0) |
| D-Mannitol | 74.4 | 74.4 |
| Crystalline Cellulose | 18.6 | 18.6 |
| Croscarmellose Sodium | 20.0 | 20.0 |
| Light Anhydrous Silicic Acid | 1.0 | 1.0 |
| Sodium Stearyl Fumarate | 6.0 | 6.0 |
| Total in Uncoated Tablet (mg) | 200.0 | 200.0 |
| Hypromellose | - | 5.63 |
| Talc | | 2.32 |
| Red Ferric Oxide | | 0.03 |
| Yellow Ferric Oxide | | 0.03 |
| Total in Coating Layer (mg) | | 8.0 |
| Total in Coated Tablet (mg) | - | 208.0 |

The tablets of Examples 6D-1 and 6D-2 were irradiated with light at a total illumination of 1.2 million lux·hr, and the amounts of related substances of the preparations and the appearance thereof were evaluated at the start of the test and after irradiation with light of 1.2 million lux·hr. For measurement of the amount of related substances, the amount of the substance having a relative retention time of 1.59 and the total amount of related substances were measured by the method of Test Example 17. In addition, the appearance properties were visually evaluated.

### <Results>

The amounts of the related substance having a relative retention time of 1.59 at the start of the test and after light irradiation with a total illumination of 1.2 million lux·hr are shown below.

**[Table 31]**

| Amount of Related Substances With Relative Retention Time of 1.59 (%) | Example 6D- 1 (Uncoated Tablet) | Example 6D-2 (Tablet Having Coating Layer) |
|---|---|---|
| At Start of Test | Not Detected | Not Detected |
| 1.2 Million lux·hr (Light Exposure) | 0.43 | 0.03 |
| 1.2 Million lux·hr (Dark Control) | Not Detected | Not Detected |

The total amount of related substances at the start of the test and after light irradiation with a total illumination of 1.2 million lux·hr is shown below.

**[Table 32]**

| Total Amount of Related Substances (%) | Example 6 D - 1 (Uncoated Tablet) | Example 6 D - 2 (Tablet Having Coating Layer) |
|---|---|---|
| At Start of Test | 0.50 | 0.53 |
| 1.2 Million lux·hr (Light Exposure) | 3.96 | 0.86 |
| 1.2 Million lux·hr (Dark Control) | 0.49 | 0.51 |

After light irradiation with a total illumination of 1.2 million lux·hr, Example 6D-2, as compared with Example 6D-1, showed a marked suppression of both the amount of the related substance having a relative retention time of 1.59 and the total amount of related substances. With respect to appearance after light irradiation with a total illumination of 1.2 million lux·hr, Example 6D-1 exhibited yellow discoloration as compared with before irradiation, whereas Example 6D-2 exhibited no observable change in appearance as compared with before irradiation.

### (Example 6E: Examination 5 of Tablet Containing Solid Dispersion of Compound Represented by Formula (I))

Uncoated tablets having a content of a solid dispersion comprising an anhydrous crystal of the compound represented by Formula (I) and copovidone different from that of Example 6D, as well as tablets obtained by coating the uncoated tablets with a photostabilizing agent and a polymer, were produced. The uncoated tablets and coated tablets were irradiated with light at a total illumination of 1.2 million lux·hr, and the amounts of related substances of the preparations and the appearance thereof were evaluated at the start of the test and after irradiation with light of 1.2 million lux·hr.

### <Method for Producing Uncoated Tablet and Coated Tablet>

An anhydrous crystal of the compound represented by Formula (I) and copovidone were dissolved in acetone, and after confirming complete dissolution, spray drying was performed using a spray dryer under conditions of an outlet temperature of 50°C, a feed rate of about 80 kg/hr, and a spray pressure of 2.5 bar. Thereafter, they were dried in vacuum for about 37 hours in a rotary vacuum drier to obtain a solid dispersion.

The obtained solid dispersion was mixed with D-mannitol (manufactured by ROQUETTE), crystalline cellulose (manufactured by Asahi Kasei Corp.), croscarmellose sodium (manufactured by Dupont), light anhydrous silicic acid (manufactured by Cabot), and half of the sodium stearyl fumarate (manufactured by JRS Pharma) in a mixer for 15 minutes, and sieved using a 1.6-mm screen. Thereafter, dry granulation was carried out using a roller compactor under the conditions of roll pressure of 5 MPa, roll speed of about 5 rpm, and screw speed of about 5 rpm. Thereafter, dry sizing was carried out using a sizing machine at a rotational speed of about 100 rpm. The sized granules and the remaining half of the sodium stearyl fumarate were mixed in a mixer for 3 minutes, and then compressed using a rotary tableting machine at a turret rotation speed of about 20 rpm to produce uncoated tablets (Example 6E-2).

For Example 6E-1, thereafter, coating was performed using a coating machine under conditions of an inlet air flow rate of 12 m³/min, an inlet air temperature setting of 60°C, a liquid flow rate of 40 to 80 g/min, a spray pressure of about 0.4 MPa, and a spray air flow rate of about 130 NL/min, thereby obtaining tablets.

A formulation is shown below.

**[Table 33]**

| Component (mg) | Example 6 E- 1 | Example 6 E- 2 |
|---|---|---|
| Solid Dispersion of Compound Represented by formula (I) (Compound Represented by Formula (I) in Solid Dispersion) (Copovidone in Solid Dispersion) | 160.0 (40.0) (120.0) | 160.0 (40.0) (120.0) |
| D-Mannitol | 148.8 | 148.8 |
| Crystalline Cellulose | 37.2 | 37.2 |
| Croscarmellose Sodium | 40.0 | 40.0 |
| Light Anhydrous Silicic Acid | 2.0 | 2.0 |
| Sodium Stearyl Fumarate | 12.0 | 12.0 |
| | | |
| Total in Uncoated Tablet (mg) | 400.0 | 400.0 |
| Hypromellose | 11.26 | - |
| Talc | 4.64 | |
| Red Ferric Oxide | 0.06 | |
| Yellow Ferric Oxide | 0.06 | |
| Total in Coating Layer (mg) | 16.0 | |
| Total in Coated Tablet (mg) | 416.0 | - |

The tablets of Examples 6E-1 and 6E-2 were irradiated with light at a total illumination of 1.2 million lux·hr, and the amounts of related substances of the preparations and the appearance thereof were evaluated at the start of the test and after irradiation with light of 1.2 million lux·hr. For the amount of related substances, the amount of the substance having a relative retention time of 1.59 and the total amount of related substances were measured by the method of Test Example 17. In addition, the appearance properties were visually evaluated.

### <Results>

The amounts of the related substance having a relative retention time of 1.59 at the start of the test and after light irradiation with a total illumination of 1.2 million lux·hr are shown below.

**[Table 34]**

| Amount of Related Substances With Relative Retention Time of 1.59 (%) | Example 6 E - 1 (Tablet Having Coating Layer) | Example 6 E - 2 (Uncoated Tablet) |
|---|---|---|
| At Start of Test | Not Detected | Not Detected |
| 1.2 Million lux·hr (Light Exposure) | 0.04 | 0.50 |
| 1.2 Million lux·hr (Dark Control) | Not Detected | Not Detected |

The total amount of related substances at the start of the test and after light irradiation with a total illumination of 1.2 million lux·hr is shown below.

**[Table 35]**

| Total Amount of Related Substances (%) | Example 6 B - 1 (Tablet Having Coating Layer) | Example 6 E - 2 (Uncoated Tablet) |
|---|---|---|
| At Start of Test | 0.19 | 0.20 |
| 1.2 Million lux·hr (Light Exposure) | 0.55 | 3.59 |
| 1.2 Million lux·hr (Dark Control) | 0.18 | 0.20 |

Compared with Example 6E-2 and 6E-1 showed a marked suppression of both the amount of the related substance having a relative retention time of 1.59 and the total amount of related substances. With respect to appearance, when the uncoated tablets of Example 6E-2 were irradiated with light at a total irradiation dose of 1.2 million lux·hr, yellow discoloration was observed compared with before irradiation, whereas no change in appearance was observed for the coated tablets of Example 6E-1 when irradiated with light at a total irradiation dose of 1.2 million lux·1hr.

### (Example 7: Capsule Comprising Solution of Compound Represented by Formula (I))

As a study of preparation formulation suitable for confirming absorption, distribution, metabolism, and excretion (ADME test) after oral administration, the capsule described below was produced.

The capsule containing a solution of the compound represented by Formula (I) was produced, and the stability of the capsule was evaluated. The capsule having the formulation shown below was produced, and the stability test was conducted.

**[Table 36]**

| Component (mg) | Example 7 - 1 | Example 7 - 2 | Example 7 - 3 | Example 7 - 4 |
|---|---|---|---|---|
| Anhydrous Crystal of Compound Represented by Formula (I) | 0.5 | 0.5 | 0.5 | 0.5 |
| Macrogol 400 | 95.5 | 92.2 | 96.6 | 94.6 |
| Copovidone | 2.9 | 2.8 | 2.9 | 2.8 |
| Ascorbic Acid | 1.1 | - | - | - |
| Citric Acid | - | 4.5 | - | - |
| *α*-Tocopherol Polyethylene Glycol Succinate | - | - | - | 2.1 |
| Total (mg) | 100.0 | 100.0 | 100.0 | 100.0 |
| Gelatin Capsule | 1 Capsule | 1 Capsule | 1 Capsule | 1 Capsule |

### <Production Method>

Macrogol 400 (manufactured by BASF), which had been stirred using a stirrer, was added with an anhydrous crystal of the compound represented by Formula (I), copovidone (manufactured by ASHLAND), ascorbic acid (manufactured by Kanto Chemical), citric acid (manufactured by FUJIFILM Wako Pure Chemical), and α-tocopherol polyethylene glycol succinate (manufactured by BLD Pharmatech). After confirming that the resulting mixture was clear, the mixture was filled into gelatin capsules (manufactured by Capsugel) to obtain samples for evaluation.

### <Stability Test>

The samples of Examples 7-1 to 7-4 shown in Table 36 were stored in closed amber glass bottles for 6 days at 25°C, and the amounts of related substances in the preparations were evaluated. The related substance test was performed under the conditions of Test Example 17, with an injection volume of 25 µL.

### <Results>

The total amount of related substances present at 0.1% or more at the start of the test and after storage at 25°C for 6 days is shown below. In Examples 7-1 and 7-2, the formation of related substances was suppressed. In addition, in Examples 7-1 and 7-2, no individual related substances present at 0.1% or more were observed from the start of the test through after storage at 25°C for 6 days.

**[Table 37]**

| Total Amount of Related Substances Present at 0.1% or More (%) | Example 7 - 1 | Example 7 - 2 | Example 7 - 3 | Example 7 - 4 |
|---|---|---|---|---|
| At Start of Test (%) | None | None | 0.19 | 0.17 |
| After 6 Days at 25° C (%) | None | None | 4.38 | 3.45 |

### (Example 8: Solution Preparation of Compound Represented by Formula (I))

As a study of preparation formulation suitable for confirming absorption, distribution, metabolism, and excretion (ADME test) after oral administration, the solution preparation described below was produced.

Solution preparations having the formulations shown in Table 38 were produced and stored for 7 days in 5°C or 25°C warmed environments and dissolution tests were performed.

**[Table 38]**

| Component (mg) | Example 8 - 1 |
|---|---|
| Anhydrous Crystal of Compound Represented by Formula (I) | 5.0 |
| Macrogol 400 | 359.7 |
| Copovidone | 10.8 |
| Ascorbic Acid | 4.1 |
| Propylene Glycol | 326.7 |
| Total (mg) | 706,3 |

### <Production Method>

The compound represented by Formula (I) was added to macrogol 400 (manufactured by BASF) while stirring with a stirrer and dissolved therein. Copovidone (manufactured by ASHLAND) and ascorbic acid (manufactured by DSM) were added and dissolved with stirring using a stirrer. After confirming that the resulting solution was clear, propylene glycol (manufactured by BASF) was added, followed by stirring with a stirrer, to obtain a solution preparation.

### <Dissolution Test>

For each solution preparation obtained in the Examples, a dissolution test was performed in accordance with the Dissolution Test described in the Japanese Pharmacopoeia 18th Edition. As the test solution, a second dissolution test medium was used, and the test was performed by the paddle method at a paddle rotation speed of 50 rpm (which was changed to 250 rpm after 120 minutes from the start of the test).

### <Results>

Fig. 27 shows dissolution test results of Example 8-1. The preparation of Example 8-1 also showed good dissolution profile at the start of the test, and there was no decrease in dissolution rate even when stored for 7 days in a 5°C or 25°C warmed environment.

### (Example 9: Preparation Comprising Ethyl Acetate Solvate Crystal of Compound Represented by Formula (I))

Formulation studies of a preparation comprising, as an active ingredient, a ethyl acetate solvate crystal of the compound represented by Formula (I) are shown below.

### (Example 9A: Examination of Uncoated Tablet Comprising Ethyl Acetate Solvate Crystal of Compound Represented by Formula (I))

An uncoated tablet comprising, as an active ingredient, an ethyl acetate solvate crystal of the compound represented by Formula (I) was produced, and stability test and dissolution test were carried out.

### <Method of Producing Uncoated Tablet>

An ethyl acetate solvate crystal of the compound represented by Formula (I), D-mannitol (ROQUETTE), crystalline cellulose (Asahi Kasei Corp.), croscarmellose sodium (Dupont), and half of the magnesium stearate (MALLINCKRODT) were mixed using a spatula and sieved through a wire mesh. The mixture was then compressed using a simple tablet forming machine (model: HANDTAB-200, manufactured by Ichihashi Seiki Co., Ltd.). Thereafter, the tablets were sized through a 20-mesh sieve, the remaining half of the magnesium stearate was added and mixed using a spatula. The resulting tableting granules were compressed using a simple tablet forming machine (model: HANDTAB-200, manufactured by Ichihashi Seiki Co., Ltd.) to obtain uncoated tablets. A formulation is shown below.

**[Table 39]**

| Component (mg) | Example 9 A- 1 |
|---|---|
| Ethyl Acetate Solvate Crystal of Compound Represented by Formula (I) (Compound Represented by Formula (I)) | 35.7 (30.0) |
| D-Mannitol | 51.9 |
| Crystalline Cellulose | 51.9 |
| Croscarmellose Sodium | 7.5 |
| Magnesium Stearate | 3.0 |
| Total in Uncoated Tablet (mg) | 150.0 |

### <Stability Test and Dissolution Test>

The uncoated tablets of Examples 9A-1 were stored for 2 week at 60°C in closed amber glass bottles and for 2 week at 40°C and 75% relative humidity in open amber glass bottles, followed by dissolution test. The dissolution test of Test Example 19 was performed.

### <Dissolution Test>

### Test Example 19: Dissolution Test

For each preparation obtained in the Examples (one tablet in the case of tablets), a dissolution test was performed in accordance with the Dissolution Test described in the Japanese Pharmacopoeia 18th Edition. Fed-state simulated intestinal fluid was used as the test medium, and the test was conducted by the paddle method at a paddle rotation speed of 50 rpm (which was changed to 250 rpm after 120 minutes from the start of the test).

### (Example 9B: Examination 1 of Capsule Comprising Ethyl Acetate Solvate Crystal of Compound Represented by Formula (I))

Capsules containing ethyl acetate solvate crystals of the compound represented by Formula (I) were produced, and a dog pharmacokinetic (PK) study was conducted using the capsules.

### <Method for Producing Capsule>

An ethyl acetate solvate crystal of the compound represented by Formula (I), D-mannitol (ROQUETTE), crystalline cellulose (Asahi Kasei Corp.), croscarmellose sodium (Dupont), and half of the magnesium stearate (MALLINCKRODT) were mixed using a spatula and sieved through a wire mesh. The mixture was then compressed using a simple tablet forming machine (model: HANDTAB-200, manufactured by Ichihashi Seiki Co., Ltd.). Thereafter, the tablets were sized through a 20-mesh sieve, the remaining half of the magnesium stearate was added and mixed using a spatula to obtain granules. The granules were manually filled into gelatin capsules using a spatula to prepare capsules. A formulation is shown below.

**[Table 40]**

| Component (mg) | Example 9 B - 1 |
|---|---|
| Ethyl Acetate Solvate Crystal of Compound Represented by Formula (1) (Compound Represented by Formula (I)) | 28.6 (24.0) |
| Free-base Crystal Form of Compound Represented by Formula (I) | |
| D-Mannitol | 41.5 |
| Crystalline Cellulose | 41.5 |
| Croscarmellose Sodium | 6.0 |
| Magnesium Stearate | 2.4 |
| | |
| Total (mg) | 120.0 |
| Gelatin Capsule | 1 Capsule |

### Test Example 20: Dog PK Study

Male Beagle dogs were fed 30 minutes to 2 hours before dosing, and thereafter one capsule was orally administered. Although

Table 33 shows a capsule preparation containing 120.0 mg of granules of the compound represented by Formula (I), in this study, the mass of granules per capsule was adjusted according to the body weight of each male Beagle dog so that the dose of the compound represented by Formula (I) was 3 mg/kg body weight. After administration of each specimen, blood samples were collected at the designated time points, and the maximum plasma drug concentration (Cmax), the time to reach the maximum plasma drug concentration (Tmax), and the area under the plasma drug concentration-time curve from dosing to 48 hours after dosing (AUC) were calculated using LC/MS/MS.

### (Example 9C: Examination 2 of Capsule Comprising Ethyl Acetate Solvate of Compound Represented by Formula (I))

A capsule preparation having formulation different from that of Example 9B and containing an ethyl acetate solvate of the compound represented by Formula (I) was produced, and the dissolution profile of the capsule were evaluated.

### <Method for Producing Capsule>

An ethyl acetate solvate of the compound represented by Formula (I), copovidone (manufactured by ASHLAND), crystalline cellulose (manufactured by Asahi Kasei Corp.), D-mannitol (manufactured by ROQUETTE), croscarmellose sodium (manufactured by Dupont), and magnesium stearate (manufactured by MALLINCKRODT) were mixed using a spatula and sieved through a wire mesh. The resulting mixture was then manually filled into hypromellose capsules (manufactured by Qualicaps) using a spatula to prepare capsules. A formulation is shown below.

**[Table 41]**

| Component (mg) | Example 9 C - 1 | Example 9 C - 2 | Example 9 C - 3 | Example 9 C - 4 |
|---|---|---|---|---|
| Ethyl Acetate Solvate of Compound Represented by Formula (I) (Compound Represented by Formula (I)) | 58.8 (50.0) | 58.8 (50.0) | 58.8 (50.0) | 58.8 (50.0) |
| Copovidone | | 30 | 30 | 7.5 |
| Crystalline Cellulose | - | 61.2 | 53.7 | |
| D-Mannitol | | | | 36.6 |
| Croscarmellose Sodium | - | - | 7.5 | 7.5 |
| Magnesium Stearate | - | - | - | 3.0 |
| | | | | |
| Total (mg) | 58.8 | 150.0 | 150.0 | 150.0 |
| Hypromellose Capsule | 1 Capsule | 1 Capsule | 1 Capsule | 1 Capsule |

### <Dissolution Test>

The preparation (one capsule) obtained in each Example was subjected to dissolution tests according to the Japanese Pharmacopoeia 18th Edition dissolution test method. Fasted-state simulated intestinal fluid (FaSSIF-V2) was used as test medium and was run by the paddle method at paddle rpm 50 rpm (which was changed to 250 rpm after 120 minutes from the start of the test).

### <Results>

The dissolution test results of Examples 9C-1 through 9C-4 are shown in Fig. 28. Example 9C-1, in which ethyl acetate solvate of a compound represented by Formula (I) was filled in Hypromellose capsule, did not show sufficient dissolution profile, but the dissolution profile were improved by the addition of copovidone 30.0 mg and croscarmellose sodium 7.5 mg (Example 9C-2, Example 9C-3). Also, D-mannitol and magnesium stearate were added, and the content of copovidone was adjusted from 30 mg to 7.5 mg, which showed even better dissolution profile (Example 9C-4).

### (Example 10: Capsule Containing Solution of Ethyl Acetate Solvate of Compound Represented by Formula (I))

As a study of preparation formulation suitable for confirming absorption, distribution, metabolism, and excretion (ADME test) after oral administration, the capsule described below was produced.

The capsule containing a solution of the ethyl acetate solvate of the compound represented by Formula (I) was produced, and the dissolution profile of the capsule were evaluated. The capsule having the formulation shown below was produced, and the dissolution test was conducted.

**[Table 42]**

| Component (mg) | Example 10 -1 | Example 1 0 - 2 |
|---|---|---|
| Ethyl Acetate Solvate of Compound Represented by Formula (I) (Compound Represented by Formula (I)) | 58.8 (50.0) | 58.8 (50.0) |
| Macrogol 400 | 1681.3 | 1666.9 |
| Copovidone | | 150.4 |
| Total (mg) | 1740.1 | 1876.0 |
| Hypromellose Capsule | 2 Capsules | 2 Capsules |

### <Production Method>

An ethyl acetate solvate of the compound represented by Formula (I) and copovidone (manufactured by ASHLAND) were added to macrogol 400 (manufactured by BASF) being stirred with a stirrer. After confirming that the resulting mixture was clear, the mixture was filled into hypromellose capsules (manufactured by Qualicaps) to obtain capsules.

### <Dissolution Test>

For each preparation obtained in the Examples (two capsules), a dissolution test was conducted in accordance with the Dissolution Test described in the Japanese Pharmacopoeia 18th Edition. The second dissolution test medium was used as the test medium, and the test was carried out by the paddle method at a paddle rotation speed of 50 rpm (which was changed to 250 rpm after 120 minutes from the start of the test).

### <Results>

The results of the dissolution tests for Examples 10-1 and 10-2 are shown in Fig. 29. When an ethyl acetate solvate of the compound represented by Formula (I) was dissolved in macrogol 400, good dissolution profile were observed (Example 10-1). Further addition of 150.4 mg of copovidone resulted in even better dissolution profile (Example 10-2).

### (Example 11: Solution Preparation of Ethyl Acetate Solvate of Compound Represented by Formula (I))

As a study of preparation formulation suitable for confirming absorption, distribution, metabolism, and excretion (ADME test) after oral administration, the capsule described below was produced.

Solution preparations having the following formulations were produced and stored for 3 days under 5°C-heating conditions, and dissolution tests were performed.

**[Table 43]**

| Component (mg) | Example 1 1 - 1 | Example 11 - 2 | Example 11 - 3 |
|---|---|---|---|
| Ethyl Acetate Solvate of Compound Represented by Formula (I) (Compound Represented by Formula (1)) | 58.9 (50.1) | 58.9 (50.1) | 58.7 (49.9) |
| Macrogol 400 | 7842.0 | 7837.0 | 7810.9 |
| Copovidone | - | 1107.6 | 1418.6 |
| Water for Injection | - | 4431.3 | 5675.5 |
| Total (mg) | 1900.9 | 13434.8 | 14963.7 |

### <Manufacturing Method>

An ethyl acetate solvate of the compound represented by Formula (I) was added to macrogol 400 (manufactured by BASF) being stirred with a stirrer and dissolved therein. Separately, copovidone (manufactured by ASHLAND) was added to water for injection and dissolved by stirring with a stirrer. The aqueous solution of copovidone (manufactured by ASHLAND) was then added to the macrogol 400 solution of the ethyl acetate solvate of the compound represented by Formula (I), followed by stirring with a stirrer, to obtain a solution preparation.

### <Dissolution Test>

For each solution preparation obtained in the Examples, a dissolution test was performed in accordance with the Dissolution Test described in the Japanese Pharmacopoeia 18th Edition. As the test solution, a second dissolution test medium was used, and the test was performed by the paddle method at a paddle rotation speed of 50 rpm (which was changed to 250 rpm after 120 minutes from the start of the test).

### <Results>

The results of the dissolution tests for Examples 11-1, 11-2, and 11-3 are shown in Fig. 30. When the preparation of Example 11-1 was stored under a temperature-controlled environment at 5°C for 3 days, the dissolution rate decreased. In contrast, in Examples 11-2 and 11-3, in which 1107.6 mg and 1418.6 mg of copovidone were added, respectively, the decrease in dissolution rate was suppressed, and a high dissolution rate was maintained.

### (Example 12: Rat PK Study of Preparation Comprising Compound Represented by Formula (I) and Ethyl Acetate Solvate of Compound Represented by Formula (I))

As a study of preparation formulation suitable for confirming absorption, distribution, metabolism, and excretion (ADME test) after oral administration, the preparation having formulation described below was produced, and the Rat PK Study was performed.

In the Rat PK study, male rats were fed and then administered with the compound represented by Formula (I) at a dose of 0.3 mg/head or 1 mg/kg.

After administration of Examples 12-1 to 12-6 shown below, blood samples were collected at each predetermined sampling time point, and the maximum plasma drug concentration (Cmax) and the area under the plasma drug concentration-time curve from dosing to 24 hours after dosing (AUC) were calculated using LC/MS/MS.

**[Table 44]**

| Component (mg) | Example 12 - 1 |
|---|---|
| Anhydrous Crystal of Compound Represented by Formula (I) | 0.3 |
| Macrogol 400 | 110.7 |
| Copovidone | 3.3 |
| Ascorbic Acid | 1.3 |
| Propylene Glycol | 100.5 |
| Total | 216.1 |

**[Table 45]**

| Component (mg) | Example 12 **-** 2 | Example 12 - 3 | Example 12 - 4 |
|---|---|---|---|
| Ethyl Acetate Solvate of Compound Represented by Formula (I) (Compound Represented by Formula (I)) | 0.35 (0.30) | 0.35 (0.30) | 0.35 (0.30) |
| Macrogol 400 | 10.03 | - | - |
| Copovidone | 0.30 | 0.54 | - |
| Crystalline Cellulose | - | 2.63 | - |
| D-Mannitol | - | 2.63 | - |
| Croscarmellose Sodium | - | 0.54 | - |
| Magnesium Stearate | - | 0.22 | - |
| 1% Aqueous Hydroxypropyl Cellulose Solution | - | - | 10.10 |
| (mg) | 10.68 | 6.91 | 10.35 |
| Gelatin Capsule | 1 Capsule | 1 Capsule | 1 Capsule |

**[Table 46]**

| **Component (mg)** | **Example 12-5** | **Example 12-6** |
|---|---|---|
| **Anhydrous Crystal of Compound Represented by Formula (I)** | - | 0.30 |
| Solid Dispersion of Compound Represented by Formula (I) (Compound Represented by Formula (I) in Solid Dispersion Powder) (Copovidone in Solid Dispersion Powder) | 1.2 (0.3) (0.9) | - |
| 0.5% Aqueous Methyl Cellulose Solution (mL) | 0.6 | 0.6 |

### <Production Method>

### Example 12-1

An anhydrous crystal of the compound represented by Formula (I), copovidone (manufactured by ASHLAND), and ascorbic acid (manufactured by DSM) were added to macrogol 400 (manufactured by BASF) being stirred with a stirrer. After confirming that the resulting mixture was clear, propylene glycol (manufactured by BASF) was added, and the mixture was mixed using a stirrer to obtain a specimen for administration.

### Example 12-2

An ethyl acetate solvate of the compound represented by Formula (I) and copovidone (manufactured by ASHLAND) were added to macrogol 400 (manufactured by BASF) being stirred with a stirrer. After confirming that the resulting mixture was clear, the mixture was filled into gelatin capsules (manufactured by Capsugel) to obtain a test specimen for administration.

### Example 12-3

An ethyl acetate solvate of the compound represented by Formula (I), copovidone (manufactured by ASHLAND), crystalline cellulose (manufactured by Asahi Kasei Corp.), D-mannitol (manufactured by ROQUETTE), croscarmellose sodium (manufactured by Dupont), and magnesium stearate (manufactured by MALLINCKRODT) were mixed using a spatula and passed through a sieve. The resulting mixture was then manually filled into gelatin capsules (manufactured by Capsugel) using a spatula to obtain a test specimen for administration.

### Example 12-4

An ethyl acetate solvate of the compound represented by Formula (I) was suspended in an aqueous solution containing 1% hydroxypropyl cellulose (manufactured by Nippon Soda), and the resulting suspension was filled into gelatin capsules (manufactured by Capsugel) to obtain a test specimen for evaluation.

### Example 12-5

A test specimen for administration was obtained by suspending a solid dispersion of the compound represented by Formula (I) in an aqueous solution containing 0.5% methylcellulose.

### Example 12-6

An anhydrous crystal of the compound represented by Formula (I) was suspended in an aqueous solution containing 0.5% methylcellulose to obtain a test specimen for administration.

### <Results>

The results of the Cmax and AUC of the compound represented by Formula (I) in each Example are shown below. Examples 12-1 to 12-5 exhibited high absorption.

**[Table 47]**

| | Example 12 - 1 | Example 12 - 2 | Example 12 - 3 | Example 12 - 4 | Example 12 - 5 | Example 12 - 6 |
|---|---|---|---|---|---|---|
| Cmax *(ng*/*mL)* | 186 | 218 | 221 | 228 | 182 | 42 |
| AUC (ng·hr/mL) | 1972 | 2171 | 2792 | 2376 | 1967 | 596 |

### Industrial Applicability

The preparation and the crystal according to the present invention has coronavirus 3CL protease inhibitory activity, and it is considered that the compound is useful as a therapeutic agent therapeutic agent a prophylactic agent for a disease or a condition associated with coronavirus 3CL proteases.

## Claims

1. A preparation comprising, as an active ingredient, a compound represented by Formula (I): 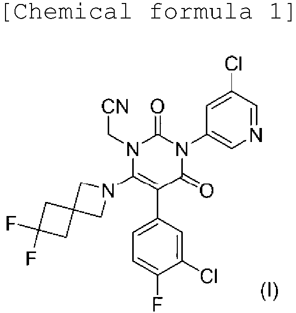 , a pharmaceutically acceptable salt thereof, or a solvate thereof.

2. The preparation according to claim 1, wherein the active ingredient is an amorphous form of the compound represented by Formula (I), a pharmaceutically acceptable salt thereof, or a solvate thereof.

3. The preparation according to claim 2, wherein the active ingredient is an amorphous form of the compound represented by Formula (I).

4. The preparation according to claim 3, wherein the amorphous form of the compound represented by Formula (I) is comprised in a solid dispersion.

5. The preparation according to claim 4, wherein the solid dispersion further comprises a polymer.

6. The preparation according to claim 5, wherein the polymer is one or more selected from vinyl-based polymer, cellulose-based polymer, or acrylic acid-based polymer.

7. The preparation according to claim 6, wherein the polymer is a vinyl-based polymer, and the vinyl-based polymer is one or more selected from the group consisting of copovidone, polyvinyl pyrrolidone, polyvinylpolypyrrolidone, polyvinyl alcohol, copolymer of polyvinyl alcohol, acrylic acid, and methyl methacrylate, poly(vinyl alcohol)-graft-poly(ethylene glycol) copolymer, polyvinyl acetal diethylaminoacetate, a fumaric acid-stearic acid-polyvinyl acetal diethylaminoacetate-hydroxypropyl methylcellulose mixture, and polyvinyl acetal diethylaminoacetate.

8. The preparation according to claim 7, wherein the vinyl-based polymer is copovidone.

9. The preparation according to claim 6, wherein the polymer is a cellulose-based polymer, and the cellulose-based polymer is one or more selected from the group consisting of hypromellose acetate succinate, hypromellose phthalate, hydroxypropyl cellulose, low substituted hydroxypropylcellulose, hypromellose, hydroxyethyl cellulose, hydroxyethyl methylcellulose, hydroxypropyl methylcellulose phthalate, methyl cellulose, methyl hydroxyethylcellulose, carboxymethyl ethylcellulose, ethyl cellulose, crystalline cellulose, microcrystalline cellulose, crystalline cellulose-carmellose sodium, carmellose, carmellose sodium, carmellose calcium, powdered cellulose, and a mixture of fumaric acid, stearic acid, poly(vinyl acetal)diethylaminoacetate, and hydroxypropyl methylcellulose.

10. The preparation according to claim 6, wherein the polymer is an acrylic acid-based polymer, and the acrylic acid-based polymer is one or more selected from the group consisting of a methacrylic acid copolymer L, an aminoalkyl methacrylate copolymer E, a methacrylic acid copolymer LD, a methacrylic acid copolymer S, aminoalkyl methacrylate copolymer RS, ethyl acrylate-methyl methacrylate copolymer, ammonio alkyl methacrylate copolymer, a methyl acrylate-methacrylic acid-methyl methacrylate copolymer, and a 2-methyl-5-vinylpyridine methyl acrylate-methacrylic acid copolymer.

11. The preparation according to claim 1, wherein the active ingredient is a crystal of the compound represented by Formula (I), a pharmaceutically acceptable salt thereof, or a solvate thereof.

12. The preparation according to claim 11, wherein the active ingredient is an anhydrous crystal of the compound represented by Formula (I).

13. The preparation according to any one of claims 1 to 12, further comprising a disintegrant, filler, and/or lubricant.

14. The preparation according to claim 13, wherein the disintegrant is one or more selected from the group consisting of croscarmellose sodium, carmellose, carmellose calcium, carmellose sodium, hydroxypropyl cellulose, low substituted hydroxypropylcellulose, powdered cellulose, partially pregelatinized starch, potato starch, cornstarch, hydroxypropyl starch, sodium carboxymethyl starch, low-substituted sodium carboxymethyl starch, sodium starch glycolate, pregelatinized starch, starch, polyvinyl alcohol, and crospovidone.

15. The preparation according to claim 14, wherein the disintegrant is croscarmellose sodium.

16. The preparation according to claim 14, wherein the disintegrant is crospovidone.

17. The preparation according to any one of claims 13 to 16, wherein the filler is one or more selected from the group consisting of crystalline cellulose, silicified microcrystalline cellulose, lactose, anhydrous lactose, sucrose, glucose, fructose, sucrose, mannitol, sorbitol, erythritol, xylitol, maltodextrin, maltitol, starch, potato starch, corn starch, rice starch, partially pregelatinized starch, pregelatinized starch, porous starch, sodium carboxy starch, hydroxypropyl starch, low-substituted sodium carboxymethyl starch, powdered cellulose, carmellose sodium, carmellose, carmellose calcium, carboxymethyl ethylcellulose, low substituted hydroxypropylcellulose, silicate derivative, phosphate, carbonate, sulfate, magnesium oxide, titanium oxide, calcium lactate, synthetic hydrotalcite, talc, kaolin, dried aluminum hydroxide, magnesium oxide, bentonite, silicon dioxide such as hydrated silica and light anhydrous silicic acid, magnesium aluminometasilicate, synthetic aluminum silicate, calcium silicate, anhydrous dibasic calcium phosphate, calcium monohydrogen phosphate, calcium hydrogen phosphate, sodium hydrogen phosphate, dibasic potassium phosphate, potassium dihydrogen phosphate, calcium dihydrogen phosphate, sodium dihydrogen phosphate, precipitated calcium carbonate, calcium carbonate, magnesium carbonate, and calcium sulfate.

18. The preparation according to any one of claims 13 to 17, wherein the lubricant is one or more selected from the group consisting of sodium stearyl fumarate, magnesium stearate, calcium stearate, stearic acid, stearyl alcohol, polyoxyl stearate 40, talc, light anhydrous silicic acid, hydrated silicon dioxide, magnesium carbonate, precipitated calcium carbonate, dried aluminum hydroxide gel, magnesium aluminometasilicate, magnesium silicate, synthetic aluminum silicate, magnesium oxide, magnesium sulfate, cocoa butter, carnauba wax, glycerine fatty acid ester, hydrogenated oil, bleached beeswax, hydrogenated soybean oil, beeswax, cetanol, sodium laurate, sucrose fatty acid ester, and polyethylene glycol.

19. The preparation according to any one of claims 1 to 18, further comprising a coating layer.

20. The preparation according to claim 19, wherein the coating layer comprises a photostabilizing agent and a polymer.

21. The preparation according to claims 20, wherein the photostabilizing agent in the coating layer is one or more selected from the group consisting of Food Red No. 2, Food Red No. 3, Food Red No. 102, Food Red No. 104, Food Red No. 105, Food Red No. 106, Food Yellow No. 4, Food Yellow No. 5, Food Green No. 3, Food Blue No. 1, Food Blue No. 2, Food Red No. 3 aluminum lake, Food Yellow No. 4 aluminum lake, Food Yellow No. 5 aluminum lake, Food Blue No. 1 aluminum lake, Food Blue No. 2 aluminum lake, carmine, sodium copper chlorophyllin, copper chlorophyll, red iron oxide, black iron oxide, yellow iron oxide, titanium oxide, red ferric oxide, yellow ferric oxide, and talc.

22. The preparation according to claims 21, wherein the photostabilizing agent is red ferric oxide, yellow ferric oxide, and/or talc.

23. The preparation according to any one of claims 20 to 22, wherein the polymer in the coating layer is one or more selected from hypromellose, hydroxypropyl cellulose, carboxymethyl ethylcellulose, hypromellose phthalate, hydroxypropyl methylcellulose acetate succinate, ethyl cellulose, and polyvinyl alcohol.

24. The preparation according to claim 23, wherein the polymer in the coating layer is hypromellose.

25. The preparation according to any one of claims 1 to 24, wherein the preparation is an oral preparation.

26. The preparation according to claim 25, wherein the preparation is a tablet, a granule, a powder, or a capsule.

27. The preparation according to claim 11, wherein the active ingredient is an ethyl acetate solvate crystal of the compound represented by Formula (I).

28. The preparation according to claim 27, wherein the preparation is a capsule.

29. An amorphous form of a compound represented by Formula (I):

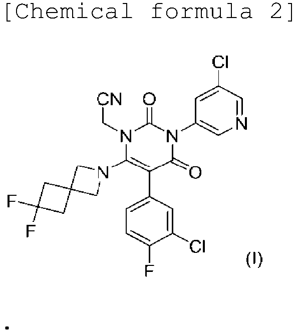

30. An ethyl acetate solvate crystal of a compound represented by Formula (I):
